# EUROPEAN PATENT APPLICATION

(11) **EP 3 789 495 A1**
(43) Date of publication of application: **10.03.2021**
(21) Application number: 19195148.2
(22) Date of filing: 03.09.2019
(51) Int. Cl.: C12P 19/18, C12P 19/26, A23L 33/00, C07H 3/06

(54) **PRODUCTION OF SIALYLATED OLIGOSACCHARIDES IN BACILLUS CELLS**

(71) Applicant: Jennewein Biotechnologie GmbH, 53619 Rheinbreitbach (DE)
(72) Inventor: JENNEWEIN, Stefan, 53605 Bad Honnef (DE); WARTENBERG, Dirk, 55435 Gau-Algesheim (DE); HABERSETZER, Stefanie, 53604 Bad Honnef (DE)

(57) **Abstract**

Disclosed are non-sporulating *Bacillus* cells for the production of a sialylated oligosaccharide wherein said *Bacillus* cell has been genetically engineered to possess a lactose permease, a CMP-NeuNAc biosynthesis pathway and a sialyltransferase, as well as a method for producing a sialylated oligosaccharide using said *Bacillus* cell.

## Description

The present invention relates to the technical field of genetic engineering, in particular to genetic engineering of *Bacillus* cells for the production of sialylated oligosaccharides in said *Bacillus* cells, to the fermentative production of sialylated oligosaccharides using said *Bacillus* cells, and to the use of thus produced sialylated oligosaccharides.

### Background

Human milk provides young infants with all nutrients they need for healthy growth and development. The saccharides that are present in human milk represent its main component, ahead of fats and proteins. In addition to lactose, which serves as an energy source, human milk contains more complex saccharide molecules, i.e. oligosaccharides. Approximately 200 structurally distinct oligosaccharides have been identified in human milk to date. These oligosaccharides are found at signify-cant concentrations only in human milk, and they are collectively known as human milk oligosaccharides (HMOs). Said HMOs are based on the disaccharide lactose (consisting of a glucose (Glc) moiety and a galactose (Gal) moiety) and bear additional monosaccharide residues which are based on *N*-acetyl-glucosamine (GlcNAc), fucose (Fuc), sialic acid/N-acetylneuraminic acid (NeuNAc), and/or galactose (Gal). The concentration and composition of HMOs in human milk varies between individuals and during the lactation period from up to 20 g/L in colostrum to 5-10 g/L in mature milk.

A considerable number of HMOs bear at least one NeuNAc moiety. Among these sialylated human milk oligosaccharides (SHMOs), 3'-sialyllactose, 6'-sialyllactose, sialyllacto-*N*-tetraose a, sialyllacto-*N*-tetraose b, sialyllacto-*N*-tetraose c and disialyllacto-*N*-tetraose are the most prevalent members in human milk.

Sialic acids (Sia) are a family of negatively charged monosaccharides with a nine-carbon backbone. More than 50 forms of these α-keto acids have been found in nature. The most abundant sialic acid appears to be *N*-acetylneuraminic acid (NANA, NeuNAc, Neu5Ac).

Sialic acids are present as terminal monosaccharide moieties of glycans that are present in glycoconjugates (glycoproteins and glycolipids) on the surface of cells of vertebrates and higher invertebrates. Sialic acids are components of the lipopolysaccharides and capsular polysaccharides of pathogenic bacteria including *Escherichia coli* K1, *Haemophilus influenzae, Haemophilus ducreyi, Pateurella multocida, Neisseria gonorrhoeae, Neisseria meningitidis, Campylobacter jejuni* and *Streptococcus agalactiae.*

Sialylated HMOs were observed to support the infant's resistance to enteropathogenic bacteria and viruses. Interestingly, recent studies further demonstrated a protective effect of long-chained SHMOs against necrotizing enterocolitis, which is one of the most common and lethal disease in preterm infants. Also, sialylated oligosaccharides have been shown to neutralize enterotoxins of various pathogenic microbes including *Escherichia coli, Vibrio cholerae* and *Salmonella.* Further, it was found that sialylated oligosaccharides interfere with the colonization of the gut by *Helicobacter pylori* and thereby prevent or inhibit gastric and duodenal ulcers. In addition, SHMOs are believed to support an infant's brain development and its cognitive capabilities.

Due to the known benefits of HMOs, especially of sialylated HMOs, an economically worthwhile process for their production is desired such that these sialylated oligosaccharides or at least some of these sialylated oligosaccharides become available as supplement for infant formulae.

The limited availability of human milk for other purposes than breast feeding an infant and the difficulties to obtain pure fractions of individual human milk oligosaccharides from natural sources led to the development of chemical routes for their synthesis. However, neither chemical synthesis nor biocatalytic *in-vitro* approaches proved to be commercially sustainable. Moreover, chemical synthesis of human milk oligosaccharides involves the use of several noxious chemicals which impose the risk of contaminating the final product.

As an alternative to chemical and biocatalytic *in-vitro* synthesis, fermentative production of HMOs has been developed. To date, recombinant *Escherichia coli* cells are used for microbial production of some HMOs in an industrial scale. However, the genus *Escherichia coli* comprises pathogenic members as well as non-pathogenic members. Notwithstanding that non-pathogenic *E. coli* strains are employed for the microbial production of HMOs, such non-pathogenic *E. coli* are not recognized as safe for the manufacturing of products that are intended for human consumption in a variety of jurisdictions. This hinders regulatory approval of HMOs being manufactured by current biotechnological methods for human consumption in said jurisdictions. Therefore, microbial cells of genera that are recognized as safe for human consumption or recognized as safe when used in the production of compounds for human consumption in such jurisdictions are needed for the manufacturing saccharides that are intended for human consumption, e.g. human milk oligosaccharides, in particular for consumption by infants. The use of production strains recognized as safe reduces the - at least alleged - concerns with respect to the likelihood of the saccharide's risks for human health, and will facilitate their regulatory approval in most jurisdictions.

The problem is solved by using bacterial cells of the genus *Bacillus* for the production of sialylated oligosaccharides, in particular of sialylated HMOs. Bacterial cells of some species of the genus *Bacillus* are already recognized as being safe for human consumption or for the production of compounds/food for human consumption. Hence, provided are *Bacillus* cells of species and/or strains that are generally recognized as safe, for the production of sialylated oligosaccharides, in particular for the production of sialylated human milk oligosaccharides.

Bacteria of the genus *Bacillus* are gram-positive, rod-shaped, endospore-forming microbial cells of either aerobic or facultatively anaerobic species. The genus *Bacillus* belongs to the phylum Firmicutes. The genome of the members of the genus *Bacillus* possesses a bias towards A-T base pairs in its codon usage. *Bacillus* species are almost ubiquitously present in nature. They can for example be found in soil (*B. subtilis*), but also occur in extreme environments such as high pH (*B. alcalophilus*), high temperature (*B*. *thermophilus*), or high salt (*B. halodurans*).

The genus *Bacillus* includes 266 named species which include free living species as well as parasitic pathogenic species. Two *Bacillus* species are considered of being medically significant: *B. anthracis,* which causes anthrax, and *B. cereus,* which causes food poisoning. A third species, *B. thuringiensis,* is an important insect pathogen producing a toxin that can kill insects. Thus, it is used as an insecticide to control insect pests.

Because of their GRAS (Generally Recognized as Safe) status, several *Bacillus* species, e.g. *B. amyloliquefaciens, B. licheniformis and B. subtilis,* are used in the biotechnological production of various proteins and compounds to be used in food and pharmaceutical industry.

*Bacillus amyloliquefaciens* is the source of restriction enzyme BamHI, and also synthesizes the natural antibiotic protein barnase. In addition, *B.amyloliquefaciens* produces plantazolicin, an antibiotic with selective activity against *B.anthracis.* Alpha-amylase from *B. amyloliquefaciens* is often used in starch hydrolysis. *B. amyloliquefaciens* is also a source of subtilisin, which catalyzes the breakdown of proteins.

*Bacillus amyloliquefaciens* is a root-colonizing bacterium that is used to fight some plant root pathogens in agriculture, aquaculture and hydroponics as it acts against bacterial and fungal pathogens, and may prevent infection by competitive exclusion or out-competing the unwanted pathogen.

Its high capacity of secreting alkaline serine protease has made *B. licheniformis* one of the most important bacteria in industrial enzyme production. Subtilisin Carlsberg secreted by *B. licheniformis* is used as a detergent protease, and is sold under the trade name Alcalase®.

*Bacillus subtilis* is a catalase positive bacterium that is found in soil and the gastrointestinal tract of ruminants and humans. *B. subtilis* and substances derived from this endotoxin-free bacterium have been evaluated by different authoritative bodies for their safe and beneficial use in food. In the United States carbohydrase and protease enzymes from *B. subtilis* are recognized as generally safe (GRAS) by the US Food and Safety Administration (FDA). *Bacillus subtilis* has also been granted the "Qualified Presumption of Safety" status by the European Food Safety Authority.

Moreover, nontoxigenic and nonpathogenic strains of *B*. *subtilis* strain are commonly used in food. For example, fermented soy beans in the form of Natto are frequently consumed in Japan and contain as many as 10⁸ viable *B. subtilis* cells per gram. Natto is recognized for its contribution to a healthy gut flora and vitamin K₂ intake. The Natto product and the *B. subtilis var. natto* as its principal component are FOSHU (Foods for Specified Health Use) approved by the Japanese Ministry of Health, Labor and Welfare as effective for preservation of health.

*Bacillus subtilis* is easy to handle, grows rapidly and the culture conditions are simple. Recombinant *B. subtilis* strains are used in the production of polyhydroxy-alkanolates, hyaluronic acid and various enzymes such as amylase and proteases.

Wild-type natural isolates of *B. subtilis* are difficult to work with as compared to laboratory strains that have undergone domestication processes of mutagenesis and selection. These domesticated strains often have improved capabilities to undergo natural competence development (uptake and integration of environmental DNA), improved capabilities of growth and loss of abilities needed "in the wild". In *B. subtilis,* linear DNA as well as multimeric forms of plasmid DNA are actively taken up by natural competent cells.

Under certain physiological conditions, a small subpopulation of *B. subtilis* cells becomes competent. In *B. subtilis,* natural competence is regulated by a complex regulatory network. Key regulators in this network are, amongst others, the master regulator of competence ComK and the transcriptional master regulator of sporulation SpoOA. Transformation efficiency of *B. subtilis* cells and probably the efficacy for integrating DNA into their genome can be improved by genetic engineering. This can be achieved by ectopic integration of an expression cassette, comprising a regulated promoter (e.g. the mannitol-inducible P*_{mtlA}* promoter) and the genes *comK* and *comS,* into the genome of *B. subtilis.* Additionally, this strategy allows transformation of *B*. *subtilis* by natural competence using a complex medium (e.g. LB).

For the production of sialylated oligosaccharides, *Bacillus* cells can be genetically engineered by various methods.

The term "genetically engineered" as used herein refers to the modification of the *Bacillus* cell's genetic make-up using molecular biological methods. The modification of the *Bacillus* cell's genetic make-up may include the transfer of genes within and/or across species boundaries, inserting, deleting, replacing and/or modifying nucleotides, triplets, genes, open reading frames, promoters, enhancers, terminators and other nucleotide sequences mediating and/or controlling gene expression. The modification of the *Bacillus* cell's genetic make-up aims to generate a genetically modified *Bacillus* cell possessing particular, desired properties. Genetically engineered *Bacillus* cells can contain one or more genes that are not present in the native (not genetically engineered) form of the cell. Techniques for introducing exogenous nucleic acid molecules and/or inserting exogenous nucleic acid molecules (recombinant, heterologous) into a *Bacillus* cell's hereditary information for inserting, deleting or altering the nucleotide sequence of a cell's genetic information are known to the skilled artisan. Genetically engineered *Bacillus* cells can contain one or more genes that are present in the native form of the cell, wherein said genes are modified and re-introduced into the *Bacillus* cell by artificial means. The term "genetically engineered" also encompasses *Bacillus* cells that contain a nucleic acid molecule being endogenous to the cell, and that has been modified without removing the nucleic acid molecule from the cell. Such modifications include those obtained by gene replacement, site-specific mutations, and related techniques.

Gene integration and/or (simultaneous) gene inactivation by disruption or deletion can be achieved by homologous recombination. For an efficient homologous recombination, at least 400-500 bp of homology arms are necessary in *B. subtilis.*

Another method for targeted genome engineering is the CRISPR-Cas9 system. This rapid and markerless genome-editing tool can be used for large scale genomic deletions, small and large DNA insertions, gene silencing by introduction of a stop codon as well as introduction of point mutations. No previous genome modifications are required for the scarless genome editing by CRISPR-Cas9.

Random chromosomal integration of genes and insertional mutagenesis can be performed using a modified mariner-derived transposon. This system is not biased towards hotpots in *B. subtilis* while showing high efficiency in random ectopic integration.

Although *Bacillus* species are used for industrial scale production of enzymes, *Bacillus* cells have not been implemented to date for industrial scale production of oligosaccharides, in particular of industrial scale production of sialylated oligosaccharides.

Chinese patent application CN 108 410 787 A discloses recombinant *Bacillus subtilis* cells which synthesize lactyl-N-neotetraose. Said recombinant *B. subtilis* cells possess a lactose permease gene which has been integrated into the cell's genome. In addition, said *Bacillus* cell which bears a plasmid comprising a β-1,3-*N*-glucosamine transferase gene and a β-1,4-galactosyltransferase gene. The *B. subtilis* cells may be cultivated in the presence of exogenous lactose, and synthesizes lactyl-N-neotetraose at titers of up to about 1 g/L which is too little for economically reasonable industrial scale production.

Chinese patent application CN 109 735 479 A discloses recombinant *Bacillus subtilis* cells for synthesizing 2'-fucosyllactose, wherein the expression of a lactose transport enzyme is enhanced, and which cell expresses a fucose kinase, a phosphate guanine transferase and a fucosyltransferase. The yield of 2'-fucosyllactose in the fermentation medium was reported to be between 0.424 g/L and 1.042 g/L.

Although a variety of patent applications mention *Bacillus* as a genus which is deemed to be suitable for the production of neutral oligosaccharides such as lacto-*N-neo*tetraose or 2'-fucosyllactose, use of *Bacillus* for the productions of sialylated oligosaccharides, in particular of sialylated human milk oligosaccharides, has not been implemented yet, presumably because of the significant efforts in metabolic engineering that are required to implement the necessary biosynthetic pathways for HMO production in *Bacillus.* Whereas above-referenced *B. subtilis* for the production of LN*n*T relies on donor substrates that are naturally occurring in *B. subtilis* cells, production of a sialylated oligosaccharide in *Bacillus* requires implementation of a heterologous metabolic pathway in the cell for providing a donor substrate, i.e. CMP-NeuNAc, that is essential for the synthesis of sialylated oligosaccharides.

The object has been achieved by providing a *Bacillus* cell that possesses a lactose permease for import of exogenous lactose into the cell, a CMP-NeuNAc biosynthesis pathway for the intracellular formation of a nucleotide-activated sialic acid, i.e. cytidine monophosphate *N*-acetylneuraminic acid (CMP-NeuNAc), as donor substrate for a sialic acid moiety, and a sialyltransferase for transfer of the sialic acid moiety from CMP-NeuNAc to an acceptor substrate. Cultivation of such *Bacillus* cells in the presence of exogenous lactose allows production of a desired sialylated oligosaccharide.

### Summary

According to a first aspect, provided is a non-sporulating bacterial cell of the genus *Bacillus* for the production of a sialylated oligosaccharide, wherein the *Bacillus* cell possesses a lactose permease, a CMP-NeuNAc biosynthesis pathway and a sialyltransferase.

According to a second aspect, provided is the use of the non-sporulating bacterial cell of the genus *Bacillus* according to the first aspect for the production of a sialylated oligosaccharide.

According to a third aspect, provided is a method for the production of a sialylated oligosaccharide, the method comprising:
- providing a non-sporulating bacterial cell of the genus *Bacillus,* wherein said *Bacillus* cell possesses a lactose permease, a CMP-NeuNAc biosynthesis pathway and a sialyltransferase;
- cultivating the *Bacillus* cell in a culture medium containing lactose and under conditions that are permissive for the *Bacillus* cell to produce the sialylated oligosaccharide, and
- optionally retrieving the sialylated oligosaccharide from the culture medium and/or the *Bacillus* cell.

According to a fourth aspect, provided are sialylated oligosaccharides that were produced by a *Bacillus* cell which possesses a lactose permease, a CMP-NeuNAc biosynthesis pathway and a sialyltransferase.

According to a fifth aspect, provided is the use of a sialylated oligosaccharide that was produced by a *Bacillus* cell which possesses a lactose permease, a CMP-NeuNAc biosynthesis pathway and a sialyltransferase for the manufacturing of a nutritional composition.

According to a sixth aspect, provided are nutritional compositions containing at least one sialylated oligosaccharide that had been produced by a *Bacillus* cell which possesses a lactose permease, a CMP-NeuNAc biosynthesis pathway and a sialyltransferase.

### Description of the figures

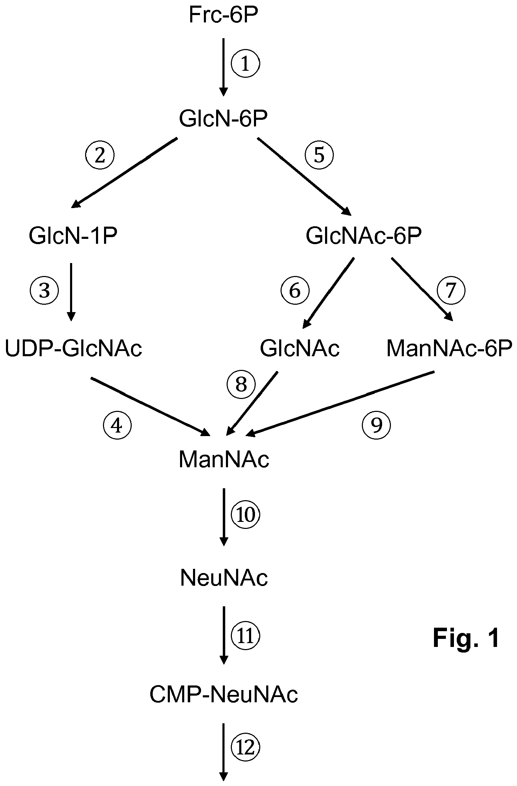

Fig. 1 illustrates different embodiments of the CMP-NeuNAc biosynthesis pathway that is implemented in a *Bacillus* cell for the production of a sialylated oligosaccharide.

### Detailed description

According to the first aspect, provided is a non-sporulating bacterial cell of the genus *Bacillus* for the production of a sialylated oligosaccharide. For being able to produce a sialylated oligosaccharide, the *Bacillus* cell has to provide a donor substrate comprising a sialic acid moiety, and an acceptor substrate being a disaccharide or an oligosaccharide to a sialyltransferase such that the sialyltransferase can transfer the sialic acid moiety from the donor substrate to said acceptor substrate thereby generating the sialylated oligosaccharide.

It is to be understood that the sialylated oligosaccharide said *Bacillus* cell is intended to produce is the desired sialylated oligosaccharide, whereas other sialylated oligosaccharides that may be generated due to the promiscuity of the sialyltransferase during production of the desired sialylated oligosaccharide in the *Bacillus* cell are considered as undesired sialylated oligosaccharides or by-products.

The *Bacillus* cell for producing a sialylated oligosaccharide is a non-sporulating *Bacillus* cell. Wild-type *Bacillus* cells are capable of forming spores. Sporulation, i.e. the process of forming spores, in bacteria is considered to be a reaction of the bacterial cell which initiates a developmental program leading to the formation of daughter cells of distinct morphology and fate. Sporulation of *Bacillus* was studied as a basic model for cell differentiation. During sporulation, a rod-shaped *Bacillus* cell divides asymmetrically, thereby resulting in two genetically identical cells with different morphology and fates.

However, in industrial production it is not desired if the bacterial production strain sporulates during fermentative production of the desired product. Therefore, it is preferred to use *Bacillus* cells for the production of sialylated oligosaccharides that are not capable of forming spores. Such *Bacillus* cells are called "non-sporulating". In an embodiment, the non-sporulating *Bacillus* cell for the production of a sialylated oligosaccharide originated from one of the *B. subtilis* strains listed in Table 1.

**Table 1: Non-limiting list of Bacillus subtilis strains.**

| **Strain** | **Manufacturer/Reference** | **Product** | **Application** |
|---|---|---|---|
| *Bacillus subtilis* 168 | BGSCID 1A1 | - | reference strain |
| *Bacillus subtilis* 168 *ΔspoIIGA::erm* | BGSCID BKE15310 | - | reference strain |
| *Bacillus subtilis* 168 *ΔsigE::erm* | BGSCID BKE15320 | - | reference strain |
| *Bacillus subtilis* 168 *ΔsigG::erm* | BGSCID BKE15330 | - | reference strain |
| *Bacillus subtilis* 168 *ΔsigF::erm* | BGSCID BKE23450 | - | reference strain |
| *Bacillus subtilis* 3NA | BGSCID 1S1 | - | reference strain |
| *Bacillus subtilis* W23 | BGSCID 2A9 | - | reference strain |
| *Bacillus subtilis* PY79 | BGSCID 1A747 | - | reference strain |
| *Bacillus subtilis* | Garden of Life® | Primal Defense® | Human use |
| *Bacillus subtilis* | Roux-Ocefa Laboratorios | Totalflora | Human use |
| *Bacillus subtilis* | Ist. Bioch. Italiano | Lactipan Plus | Human use |
| *Bacillus subtilis* 2335 | Biopharma | Biosporin® | Human use |
| LifeinU™ *Bacillus subtilis* CU1 | Lesaffre Human Care | Super Smart® | Human use |
| *Bacillus subtilis* PXN/21 | Probiotics International Ltd | Bio-Kult® | Human use |
| *Bacillus subtilis* HU58 | Microbiome LABS | HU58™ | Human use |
| *Bacillus subtilis* R0179 | Hanmi Pharmaceutical Co. Ltd. | Medilac® | Human use |
| *Bacillus subtilis* DE111 | Deerland Enzymes & Probiotics | NutriCommit | Human use |
| *Bacillus subtilis* DSM21097 | MorinagaGarden B.V. | Morinaga Plus⁺ | Human use |
| *Bacillus subtilis* IDCC1101 | Ildong Pharmaceutical | Biobaby® | Human use |
| *Bacillus subtilis* KATMIRA1933 | JSL Foods | YoguFarm | Human use |
| *Bacillus subtilis* spp. Natto | *various* | Natto | Human use |
| *Bacillus subtilis* SC-8 | *various* | Cheonggukjang | Human use |
| *Bacillus subtilis* CH201/DSM5750 | Christian Hansen | BioPlus2B® | Veterinary Use |
| *Bacillus subtilis* PB6 | Kenim® | CLOSTAT® | Veterinary Use |

In some embodiments, the *Bacillus* cell has been genetically engineered to become non-sporulating, for example by deletion or functional inactivation of SpoOA. SpoOA is a DNA-binding protein which influences, directly or indirectly, the expression of more than 500 genes during the early stage of sporulation. Suitable functional inactivation of SpoOA includes deletion of the phosphorylation site at which SpoOF and Spo0B phosphotransferases phosphorylate SpoOA. Deletion or functional inactivation of the SpoOF and Spo0B phosphotransferase phosphorylation site leads to a functional inactivation of SpoOA and thereby impairs the *Bacillus* cell's ability to sporulate. Alternatively, the gene or nucleotide sequence encoding SpoOA, or part of the gene or nucleotide sequence encoding SpoOA may be deleted from the *Bacillus* cell's genome.

In an additional and/or alternative embodiment, the *Bacillus* cell has been genetically engineered by deletion or functional inactivation of the gene(s) encoding sigma factor SigE (*sigE*) and/or sigma factor SigF (*sigF*). Sigma factors SigE and SigF are transcription factors that are involved in the expression of genes which encode proteins that are involved in the early phase of sporulation.

Deletion or functional inactivation of SpoOA, SigE and/or SigF impairs the sporulation ability of a *Bacillus* cell to sporulate. Such non-sporulating *Bacillus* cell may be used as a progenitor for generating a *Bacillus* cell that is able to produce a sialylated oligosaccharide.

Wild-type *Bacillus* cells neither synthesize lactose intracellularly nor do they internalize exogenous lactose. However, lactose is the acceptor substrate for a sialic acid moiety by a lactose-accepting sialyltransferase in the formation of some sialylated oligosaccharides such as 3'-sialyllactose (3-SL) or 6'-sialyllactose (6-SL). Hence, to be able to produce such sialylated oligosaccharides, the *Bacillus* cell has to have the capability of providing lactose to the lactose-accepting sialyltransferase, either by generating lactose intracellularly and/or by internalizing exogenous lactose. Moreover, lactose is typically the disaccharide moiety most if - not all - sialylated HMOs bear. Hence, internalization of lactose is also required for the biosynthesis of other sialylated HMOs than trisaccharides, i.e. when the acceptor substrate for the NeuNAc moiety is an oligosaccharide rather than the disaccharide lactose.

In an embodiment, the *Bacillus* cell for producing a sialylated oligosaccharide possesses a lactose permease for internalization of exogenous lactose. Hence, the *Bacillus* cell is able to internalize exogenous lactose. Alternatively, the *Bacillus* cell may be genetically engineered to synthesize lactose intracellularly from glucose and galactose such that an exogenous supply of lactose will not be necessary for the production of a sialylated oligosaccharide.

The term "exogenous" with respect to lactose as used herein refers to lactose that does not originate from the *Bacillus* cell for the production of the sialylated oligosaccharide, i.e. being intracellularly synthesized by said *Bacillus* cell, but which originates from outside of said *Bacillus* cell and which is added to the culture medium in which the *Bacillus* cell for producing the sialylated oligosaccharide is grown to produce the sialylated oligosaccharide.

In some embodiments, the *Bacillus* cell has been genetically engineered to be able to internalize exogenous lactose, i.e. it has been genetically engineered to possess a lactose permease. Thus, the *Bacillus* cell for producing a sialylated oligosaccharide possesses a heterologous lactose permease. A suitable heterologous lactose permease is *E. coli* LacY or a functional variant thereof.

The term "heterologous" as used herein with respect to proteins, polypeptides, enzymes and transporters, but also with respect to nucleic acid molecules, nucleotide sequences and/or genes, refers to a molecule that is non-native to the species of the *Bacillus* cell which contains said molecule. The term "non-native" indicates that said molecule is not present in the naturally occurring or wild-type progenitor *Bacillus* cell, i.e. in the *Bacillus* cell of the same species that is most commonly occurring in nature. Thus, a "heterologous sequence" or a "heterologous nucleic acid" or "heterologous polypeptide", as used herein, is one that originates from a source foreign to the *Bacillus* cell (e.g. from a different species), or, if from the same source, is modified from its original form. The heterologous sequence may be stably introduced, e.g. by transfection, transformation, conjugation or transduction, into the genome of the host microbial host cell, thus representing a genetically modified host cell. Techniques may be applied which will depend on the host cell the sequence is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989). Accordingly, a "heterologous polypeptide" is a polypeptide that does not naturally occur in the cell, and a "heterologous sialyltransferase" is a sialyltransferase that does not naturally occur in the microbial cell.

The term "functional variant" as used herein with respect to enzymes and/or transport molecules refers to proteins or polypeptides possessing the same activity (enzymatic, catalytic or translocating) as the referenced enzyme or transporter, but which has a different amino acid sequence than the referenced enzyme or transporter molecule. Thus, a typical variant of a protein/polypeptide differs in amino acid sequence from the reference protein/polypeptide. A variant and the reference protein/polypeptide may differ in amino acid sequence by one or more substitutions, additions, and/or deletions in any combination. Hence, the term "functional variant" comprises truncated versions of the referenced protein/polypeptide which possesses the same activity as the reference protein/polypeptide. A substituted or inserted amino acid residue may or may not be one encoded by the genetic code. A variant of a protein/ polypeptide may be naturally occurring such as an allelic variant, or it may be a variant that is not known to occur naturally. Non-naturally occurring variants of proteins/polypeptides may be made by mutagenesis techniques, by direct synthesis, and by other recombinant methods known to the persons skilled in the art. Within the scope of the present disclosure, also proteins and interspecies homologs are comprised by the term "variant" that have an amino acid sequence that has greater than about 60% amino acid sequence identity, preferably 65%, 70%, 75%, 80%, 85%, 90%, more preferably 91%, 92%, 93%, 94%, 95%, 96%, 97%, 98% or 99% or greater amino acid sequence identity, preferably over a region of at least about 25, 50, 100, 200, 500, 1000, or more amino acids, to the referenced polypeptide.

The term "same activity" as used herein refers to an enzymatic, catalytic or transporting activity of a protein/polypeptide in qualitative manner only. Hence, "functional variant" also comprises variants which possess an increased or decreased activity as compared to the activity of the referenced protein/polypeptide.

In various embodiments, the *Bacillus* cell has been genetically engineered to contain and express a nucleotide sequence which encodes a lactose permease, preferably a nucleotide sequence that encodes the *E. coli* lactose permease LacY (UniProtKB - P02920) or a functional variant thereof.

The *E. coli* lactose permease LacY is encoded by the protein coding region (i.e. the open reading frame) of the *E. coli lac*Y gene (GenBank acc. no: NP_414877.1).

Hence, in some embodiments, the *Bacillus* cell has been genetically engineered to contain and express a nucleotide sequence encoding the protein-coding region of *E. coli lacY.*

In some embodiments, the codon usage of the nucleotide sequence encoding the heterologous lactose permease is adapted to the codon usage of *Bacillus.* The codon usage of *B. subtilis* for example is peculiar in that the overall GC-content is below about 45 %, the GC-content of the first letter of the codons above about 51 %, the CG-content of the second letter of the codons below about 36.1 %, and the CG-content of the third letter of the codons below about 46 %.

For expression of the lactose permease, the *Bacillus* cell contains a recombinant lactose permease gene, wherein the nucleotide sequence which encodes the lactose permease is operably linked to expression control sequences which mediate expression of the nucleotide sequence encoding the lactose permease.

The term "operably linked" as used herein shall mean a functional linkage between a nucleotide sequence which encodes a polypeptide or protein (typically referred to as "protein coding region", "open reading frame", and sometimes even as "gene") and a second nucleotide sequence (the expression control sequence such as a promoter, signal sequence, or array of transcription factor binding sites) which effects transcription and/or translation of the nucleotide sequence which encodes the polypeptide or protein. Accordingly, the term "promoter" designates desoxyribonucleic acid (DNA) sequences which usually "precede" an open reading frame in a DNA polymer and provides a site for initiation of the transcription into mRNA. "Regulator" DNA sequences, also usually "upstream" of (i.e., preceding) an open reading frame in a given DNA polymer, bind proteins that determine the frequency (or rate) of transcriptional initiation. Collectively referred to as "promoter/regulator" or "control" DNA sequence, these sequences which precede a selected open reading frame (or series of open reading frames) in a functional DNA polymer cooperate to determine whether the transcription (and eventual expression) of an open reading frame will occur. DNA sequences which "follow" a gene in a DNA polymer and provide a signal for termination of the transcription into mRNA are referred to as transcription "terminator" sequences.

The recombinant lactose permease gene may be present as being integrated into the *Bacillus* chromosome, or present as an episomal version on an additional plasmid within the *Bacillus* cell.

Expression of the heterologous lactose permease gene in the *Bacillus* cell enables the resulting *Bacillus* cell to internalize exogenously supplied lactose from the culture medium. The internalized lactose can then serve as an acceptor substrate for a sialic acid moiety to be transferred by a lactose-accepting sialyltransferase (see herein below), for example in the formation of 3'-SL or 6'-SL.

For the production of a sialylated oligosaccharide, the *Bacillus* has to be able to provide a donor substrate for transferring a NeuNAc moiety to an acceptor substrate. An exemplary donor substrate for a NeuNAc moiety is CMP-NeuNAc. Hence, the *Bacillus* cell has to be able to intracellularly produce CMP-NeuNAc for the production of sialylated oligosaccharides. For intracellular biosynthesis of CMP-NeuNAc, the *Bacillus* cell possesses a CMP-NeuNAc biosynthesis pathway (Fig. 1). Thus, the *Bacillus* cell has been genetically engineered to possess a CMP-NeuNAc biosynthesis pathway.

The CMP-NeuNAc biosynthesis pathway comprises a NeuNAc salvage pathway or a sialic acid biosynthesis pathway for intracellular *de novo* biosynthesis of NeuNAc. Hence, the *Bacillus* cell possesses a CMP-NeuNAc biosynthesis pathway comprising a NeuNAc salvage pathway and/or a sialic acid biosynthesis pathway.

The NeuNAc salvage pathway comprise internalization of exogenous sialic acid by the *Bacillus* cell, and conversion of the internalized sialic acid to CMP-NeuNAc. For internalization of exogenous NeuNAc, the *Bacillus* cell possesses a sialic acid transporter. In some embodiments, the genetically engineered *Bacillus* cell has been genetically engineered to possess a sialic acid transport for internalization of exogenous NeuNAc. A suitable sialic acid transporter is *E. coli* NanT (UniProtKB P41036). *E. coli* NanT catalyzes the proton-dependent symport of sialic acid. NanT can transport NeuNAc as well as the related sialic acids *N*-glycolylneuraminic acid (NeuNGc) and 3-keto-3-deoxy-D-glycero-D-galactonononic acid (KDN). In an embodiment, the genetically engineered *Bacillus* cell possesses *E. coli* NanT or a functional variant thereof.

In some embodiments, the *Bacillus* cell has been genetically engineered to contain and express a nucleotide sequence which encodes a sialic acid transporter for internalization of exogenous NeuNAc, preferably a nucleotide sequence that encodes *E. coli* NanT or a functional variant thereof.

The *E. coli* NanT is encoded by the protein -coding region of the *E. coli nanT gene.* Hence, in some embodiments, the *Bacillus* cell has been genetically engineered to contain and express a nucleotide sequence that encodes the *E. coli* sialic acid transporter NanT or a functional variant thereof.

For expression of the sialic acid transporter, the Bacillus cell contains a recombinant sialic acid transporter gene, wherein the nucleotide sequence that encodes the sialic acid transporter is operably linked to expression control sequences which mediate expression of the nucleotide sequence encoding the sialic acid transporter.

The recombinant sialic acid transporter gene may be present as being integrated into the Bacillus chromosome, or present as an episomal version on an additional plasmid in the *Bacillus* cell.

In some embodiment, the codon usage of the nucleotide sequence encoding the sialic acid transporter is adapted to the codon usage of *Bacillus.*

In various embodiments, the CMP-NeuNAc biosynthesis pathway comprises a sialic acid biosynthesis pathway. Thus, the *Bacillus* cell may comprise a sialic acid biosynthesis pathway for the intracellular biosynthesis of *N*-acetylneuraminic acid. The sialic acid biosynthesis pathway comprises the enzymatic activities of a glutamine:fructose-6-phosphate aminotransferase (Fig. 1: ①) and an *N*-acetylneuraminic acid synthase (= sialic acid synthase) (Fig.1: ⑩). Hence, the *Bacillus* cell for the production of a sialylated oligosaccharide possesses a glutamine:fructose-6-phosphate aminotransferase and an *N*-acetylneuraminic acid synthase.

The enzyme glutamine:fructose-6-phosphate aminotransferase (EC 2.6.1.16) catalyzes the conversion of fructose-6-phosphate (Frc-6P) to glucosamine-6-phosphate (GlcN-6P) using glutamine (Fig. 1: ①). This enzymatic reaction is typically considered to be the first step in the hexosamine biosynthesis pathway. Alternative names of the glutamine:fructose-6-phosphate aminotransferase are D-fructose-6-phosphate aminotransferase, GFAT, glucosamine-6-phosphate synthase, hexosephosphate aminotransferase, and L-glutamine-D-fructose-6-phosphate aminotransferase.

In an additional and/or alternative embodiment, the genetically engineered *Bacillus* cell possesses a glutamine:fructose-6-phosphate aminotransferase, preferably a heterologous a glutamine:fructose-6-phosphate aminotransferase. An example of a suitable heterologous glutamine:fructose-6-phosphate aminotransferase is derived from *E. coli.* The *E. coli* glutamine:fructose-6-phosphate aminotransferase (UniProtKB - P17169) is designated GlmS. Hence, the *Bacillus* cell possesses an *E. coli* GlmS or a functional variant of the *E. coli* GlmS. Preferably, the functional variant of the *E. coli* GlmS is a version which shows significantly reduced sensitivity to glucosamine-6-phosphate inhibition as compared to the wild-type enzyme. An example of a functional variant of the *E. coli* GlmS which shows significantly reduced sensitivity to glucosamine-6-phosphate inhibition. Examples of functional variants of the *E. coli* GlmS which shows significantly reduced sensitivity to glucosamine-6-phosphate inhibition are GlmS*54 and GlmS* as described in international application No. PCT/EP2019/063669 (incorporated herein by reference).

In an additional and/or alternative embodiment, the genetically engineered *Bacillus* cell contains a nucleic acid molecule comprising a nucleotide sequence which encodes a glutamine:fructose-6-phosphate aminotransferase, preferably the *E. coli* glutamine:fructose-6-phosphate aminotransferase GlmS, or a nucleotide sequence encoding a functional variant of the *E. coli* GlmS which shows significantly reduced sensitivity to glucosamine-6-phosphate inhibition as compared to the wild-type enzyme.

For expression of the glutamine:fructose-6-phosphate aminotransferase, the *Bacillus* cell contains a recombinant glutamine:fructose-6-phosphate aminotransferase gene, wherein the nucleotide sequence which encodes the glutamine:fructose-6-phosphate aminotransferase is operably linked to expression control sequences which mediate expression of the glutamine:fructose-6-phosphate aminotransferase open reading frame.

In an additional and/or alternative embodiment, the codon usage of the nucleotide sequence encoding the heterologous glutamine:fructose-6-phosphate aminotransferase is adapted to the codon usage of *Bacillus.*

The recombinant glutamine:fructose-6-phosphate aminotransferase gene may be integrated into the *Bacillus* chromosome, or present as an episomal version on a plasmid within the *Bacillus* cell.

In an additional and/or alternative embodiment, the genetically engineered *Bacillus* cell comprises a sialic acid synthase. The sialic acid synthase catalyzes the condensation of *N*-acetylmannosamine (ManNAc) and phosphoenolpyruvate (PEP) to *N*-acetylneuraminic acid (NeuNAc) (Fig. 1: ⑩). The enzymatic formation of NeuNAc is the last step of the sialic acid biosynthesis pathway.

In an additional and/or alternative embodiment, the genetically engineered *Bacillus* cell comprises a sialic acid synthase or a functional variant thereof, preferably a heterologous sialic acid synthase. Examples of sialic acid synthases are known from a variety of bacterial species such as *Campylobacter jejuni, Streptococcus agalactiae, Butyrivibrio proteoclasticus, Methanobrevibacter ruminatium, Acetobacterium woodii, Desulfobacula toluolica, Escherichia coli, Prevotella nigescens, Halorhabdus tiamatea, Desulfotignum phosphitoxidans, or Candidatus Scalindua* sp., *Idomarina loihiensis, Fusobacterium nucleatum* or *Neisseria meningitidis.* An example of a suitable sialic acid synthase is the *N*-acetylneuraminic acid synthase NeuB of *C. jejuni* as encoded by the *C. jejuni neu*B gene.

Thus, in an additional and/or alternative embodiment, the genetically engineered *Bacillus* cell contains a nucleic acid molecule comprising and expressing a nucleotide sequence which encodes a *N*-acetylneuraminic acid synthase. Hence, the nucleotide sequence which encodes a *N*-acetylneuraminic acid synthase is operably linked to at least one nucleic acid expression control sequence effecting transcripttion and/or translation of said nucleotide sequence which encodes a *N*-acetylneuraminic acid synthase in the genetically engineered *Bacillus* cell to provide intracellular *N*-acetylneuraminic acid synthase activity.

In an additional and/or alternative embodiment, the codon usage of the nucleotide sequence encoding the heterologous *N*-acetylneuraminic acid synthase is adapted to the codon usage of *Bacillus.*

The recombinant *N*-acetylneuraminic acid synthase gene may be integrated into the *Bacillus* chromosome, or present as an episomal version on a plasmid within the *Bacillus* cell.

In an embodiment, the sialic acid biosynthesis pathway comprises uridine diphosphate-N-acetylglucosamine (UDP-GlcNAc) as an intermediate compound. The *Bacillus* cell comprising a sialic acid biosynthesis pathway which comprises UDP-GlcNAc as intermediate compound further possesses a phosphoglucosamine mutase (Fig. 1: ②), a glucosamine-1-phosphate *N*-acetyltransferase (Fig. 1: ③), an *N*-acetylglucosamine-1-phosphate uridyltransferase (Fig. 1 ④), and a UDP-N-acetylglucosamine 2-epimerase (Fig. 1: ④).

The phosphoglucosamine mutase converts glucosamine-6-phosphate (GlcN-6P) to glucosamine-1-phosphate (GlcN-1P).

In an additional and/or alternative embodiment, the *Bacillus* cell has been genetically engineered to possess a phosphoglucosamine mutase. A suitable phosphoglucosamine mutase is the *E. coli* phosphoglucosamine mutase GlmM or a functional variant thereof. Said *E. coli* GlmM is encoded by the *E. coli glmM* gene.

In an additional and/or alternative embodiment, the *Bacillus* cell has been genetically engineered to contain and express a nucleotide sequence which encodes a phosphoglucosamine mutase, preferably a nucleotide sequence that encodes the *E. coli* GlmM or a functional variant thereof.

The *E. coli* phosphoglucosamine mutase GlmM is encoded by the protein coding region of the *E. coli glmM* gene. Hence, the *Bacillus* cell has been genetically engineered to contain and express a nucleotide sequence that encodes the *E. coli* GlmM.

Thus, in an additional and/or alternative embodiment, the genetically engineered *Bacillus* cell contains a nucleic acid molecule comprising and expressing a nucleotide sequence which encodes a phosphoglucosamine mutase. Hence, the nucleotide sequence which encodes a phosphoglucosamine mutase is operably linked to at least one nucleic acid expression control sequence effecting transcription and/or translation of said nucleotide sequence which encodes a phosphoglucosamine mutase in the genetically engineered *Bacillus* cell to provide intracellular phosphoglucosamine mutase activity.

In an additional and/or alternative embodiment, the codon usage of the nucleotide sequence encoding the heterologous phosphoglucosamine mutase is adapted to the codon usage of *Bacillus.*

The recombinant phosphoglucosamine mutase gene may be integrated into the *Bacillus* chromosome, or present as an episomal version on a plasmid within the *Bacillus* cell.

The glucosamine-1-phosphate N-acetyltransferase catalyzes the transfer of an acetyl group from acetyl coenzyme A to glucosamine-1-phosphate (GlcN-1-P) to produce *N*-acetylglucosamine-1-phosphate (GlcNAc-1-P).

In an additional and/or alternative embodiment, the *Bacillus* cell has been genetically engineered to possess a glucosamine-1-phosphate *N*-acetyltransferase.

In an additional and/or alternative embodiment, the *Bacillus* cell has been genetically engineered to contain and express a nucleotide sequence which encodes a glucosamine-1-phosphate *N*-acetyltransferase.

Thus, in an additional and/or alternative embodiment, the genetically engineered *Bacillus* cell contains a nucleic acid molecule comprising and expressing a nucleotide sequence which encodes a glucosamine-1-phosphate *N*-acetyltransferase. Hence, the nucleotide sequence which encodes a glucosamine-1-phosphate *N*-acetyltransferase is operably linked to at least one nucleic acid expression control sequence effecting transcription and/or translation of said nucleotide sequence which encodes a glucosamine-1-phosphate *N*-acetyltransferase in the genetically engineered *Bacillus* cell to provide intracellular glucosamine-1-phosphate N-acety-Itransferase activity.

In an additional and/or alternative embodiment, the codon usage of the nucleotide sequence encoding the heterologous glucosamine-1-phosphate *N*-acetyltransferase is adapted to the codon usage of *Bacillus.*

The recombinant glucosamine-1-phosphate *N*-acetyltransferase gene may be integrated into the *Bacillus* chromosome, or present as an episomal version on a plasmid within the *Bacillus* cell.

N-acetylglucosamine-1-phosphate uridyltransferase converts *N*-acetylglucosamine-1-phosphate (GlcNAc-1-P) into UDP-GlcNAc by the transfer of uridine 5-monophosphate (from uridine 5-triphosphate).

In an additional and/or alternative embodiment, the *Bacillus* cell has been genetically engineered to possess a *N*-acetylglucosamine-1-phosphate uridyltransferase.

In an additional and/or alternative embodiment, the *Bacillus* cell has been genetically engineered to contain and express a nucleotide sequence which encodes a *N*-acetylglucosamine-1-phosphate uridyltransferase.

Thus, in an additional and/or alternative embodiment, the genetically engineered *Bacillus* cell contains a nucleic acid molecule comprising and expressing a nucleotide sequence which encodes a *N*-acetylglucosamine-1-phosphate uridyltransferase. Hence, the nucleotide sequence which encodes a *N*-acetylglucosamine-1-phosphate uridyltransferase is operably linked to at least one nucleic acid expression control sequence effecting transcription and/or translation of said nucleotide sequence which encodes a *N*-acetylglucosamine-1-phosphate uridyltransferase in the genetically engineered *Bacillus* cell to provide intracellular *N*-acetylglucosamine-1-phosphate uridyltransferase activity.

In an additional and/or alternative embodiment, the codon usage of the nucleotide sequence encoding the heterologous *N*-acetylglucosamine-1-phosphate uridyltransferase is adapted to the codon usage of *Bacillus.*

The recombinant *N*-acetylglucosamine-1-phosphate uridyltransferase gene may be integrated into the *Bacillus* chromosome, or present as an episomal version on a plasmid within the *Bacillus* cell.

In an additional and/or alternative embodiment, the glucosamine-1-phosphate *N*-acetyltransferase activity and the N-acetylglucosamine-1-phosphate uridyltransferase activity are provided by a bifunctional enzyme. An example of such bifunctional enzyme is *E. coli* GlmU. *E. coli* GlmU catalyzes the last two sequential reactions which convert glucosamine-1-phosphate to UDP-GlcNAc (Fig. 1: ③). The C-terminal domain catalyzes the transfer of acetyl group from acetyl coenzyme A to glucosamine-1-phosphate (GlcN-1-P) to produce *N*-acetylglucosamine-1-phosphate (GlcNAc-1-P), which is converted into UDP-GlcNAc by the transfer of uridine 5-monophosphate (from uridine 5-triphosphate), a reaction catalyzed by the N-terminal domain.

Such bifunctional enzyme is also known from *Bacillus subtilis* and *Haemophilus influenzae.*

In an additional and/or alternative embodiment, the *Bacillus* cell has been genetically engineered to possess a heterologous bifunctional glucosamine-1-phosphate N-acetyltransferase / N-acetylglucosamine-1-phosphate uridyltransferase. A suitable example is *E. coli* GlmU or a functional variant thereof (including the *H. influenzae* GlmU). Said *E. coli* GlmU is encoded by the *E. coli glmU* gene.

In an additional and/or alternative embodiment, the *Bacillus* cell has been genetically engineered to contain and express or overexpress a nucleotide sequence which encodes said bifunctional enzyme, preferably a nucleotide sequence that encodes the *E. coli* GlmU or a functional variant thereof.

Thus, in an additional and/or alternative embodiment, the genetically engineered *Bacillus* cell contains a nucleic acid molecule comprising and expressing a nucleotide sequence which encodes the bifunctional glucosamine-1-phosphate *N*-acetyltransferase / *N*-acetylglucosamine-1-phosphate uridyltransferase. Hence, the nucleotide sequence which encodes a bifunctional glucosamine-1-phosphate *N*-acetyltransferase / *N*-acetylglucosamine-1-phosphate uridyltransferase is operably linked to at least one nucleic acid expression control sequence effecting transcription and/or translation of said nucleotide sequence which encodes a bifunctional glucosamine-1-phosphate *N*-acetyltransferase / *N*-acetylglucosamine-1-phosphate uridyltransferase in the genetically engineered *Bacillus* cell to provide intracellular glucosamine-1-phosphate *N*-acetyltransferase activity and *N*-acetylglucosamine-1-phosphate uridyltransferase activity.

In an additional and/or alternative embodiment, the codon usage of the nucleotide sequence encoding the heterologous bifunctional glucosamine-1-phosphate *N*-acetyltransferase / *N*-acetylglucosamine-1-phosphate uridyltransferase is adapted to the codon usage of *Bacillus.*

The recombinant bifunctional glucosamine-1-phosphate *N*-acetyltransferase / *N-*acetylglucosamine-1-phosphate uridyltransferase gene may be integrated into the *Bacillus* chromosome, or present as an episomal version on a plasmid within the *Bacillus* cell.

In an additional and/or alternative embodiment, the sialic acid biosynthesis pathway comprises the conversion of UDP-GlcNAc to *N*-acetylmannosamine (ManNAc). This conversion can be catalyzed by an UDP-*N*-acetylglucosamine 2-epimerase which does not just convert UDP-*N*-acetylglucosamine to UDP-*N*-acetylmannosamine, but releases UDP concomitantly (Fig. 1: ④).

Hence, the *Bacillus* cell comprises an UDP-*N*-acetylglucosamine 2-epimerase epimerase which releases UDP concomitantly.

A suitable UDP-*N*-acetylglucosamine 2-epimerase epimerase with concomitant release of UDP is the *Campylobacter jejuni* UDP-*N*-acetylglucosamine 2-epimerase NeuC, encoded by the *C. jejuni neuC* gene.

In an additional and/or alternative embodiment, the *Bacillus* cell has been genetically engineered to possess an UDP-*N*-acetylglucosamine 2-epimerase with concomitant release of UDP.

In an additional and/or alternative embodiment, the *Bacillus* cell has been genetically engineered to contain and express a nucleotide sequence which encodes a UDP-*N*-acetylglucosamine 2-epimerase which releases UDP concomitantly, preferably to contain and express a nucleotide sequence that encodes the *C. jejuni* NeuC or a functional variant thereof.

The *C. jejuni* UDP-*N*-acetylglucosamine 2-epimerase NeuC is encoded by the protein coding region of the *C. jejuni neuC* gene. Hence, the *Bacillus* cell has been genetically engineered to contain and express a nucleotide sequence that encodes the *C. jejuni* NeuC.

Thus, in an additional and/or alternative embodiment, the genetically engineered *Bacillus* cell contains a nucleic acid molecule comprising and expressing a nucleotide sequence which encodes the UDP-*N*-acetylglucosamine 2-epimerase with concomitant release of UDP during its enzymatic reaction to release ManNAc. Hence, the nucleotide sequence which encodes a UDP-N-acetylglucosamine 2-epimerase which releases UDP concomitantly is operably linked to at least one nucleic acid expression control sequence effecting transcription and/or translation of said nucleotide sequence which encodes said UDP-*N*-acetylglucosamine 2-epimerase in the genetically engineered *Bacillus* cell to provide intracellular UDP-*N*-acetylglucosamine 2-epimerase activity.

In an additional and/or alternative embodiment, the codon usage of the nucleotide sequence encoding the heterologous UDP-*N*-acetylglucosamine 2-epimerase with concomitant release of UDP is adapted to the codon usage of *Bacillus.*

The recombinant UDP-*N*-acetylglucosamine 2-epimerase gene may be integrated into the *Bacillus* chromosome, or present as an episomal version on a plasmid within the *Bacillus* cell.

In an alternative embodiment, the sialic acid biosynthesis pathway utilizes N-acetylglucosamine-6-phosphate (GlcNAc-6-P) as an intermediate, but not utilize UDP-GlcNAc. The genetically engineered *Bacillus* cell comprises a sialic acid biosynthesis pathway for the intracellular biosynthesis of *N*-acetylneuraminic acid which uses GlcNAc-6-P as intermediate comprises a glucosamine-6-phosphate *N*-acetyltransferase (Fig. 1: ⑤). A sialic acid biosynthesis pathway using a glucosamine-6-phosphate *N*-acetyltransferase for the intracellular biosynthesis of *N-*acetylneuraminic acid does not utilize UDP-GlcNAc for the biosynthesis of sialic acid.

The sialic acid biosynthesis pathway using GlcNAc-6P comprises the enzymatic activities of a glutamine:fructose-6-phosphate aminotransferase (Fig. 1: ①) and an *N*-acetylneuraminic acid synthase (Fig. 1: ⑩). Said sialic acid biosynthesis pathway further comprises a) the enzymatic activities of a glucosamine-6-phosphate *N*-acetyltransferase (Fig. 1: ⑤), an *N*-acetylglucosamine-6-phosphate phosphatase (Fig. 1: ⑥) and an *N*-acetylglucosamine 2-epimerase (Fig. 1: ⑧) or b) the enzymatic activities of a glucosamine-6-phosphate *N*-acetyltransferase (Fig. 1: ⑤), an *N*-acetylglucosamine-6-phosphate epimerase (Fig. 1: ⑦) and an N-acetylmannosamine-6-phosphate phosphatase (Fig. 1: ⑨). Therefore, it is not necessary that the genetically engineered *Bacillus* cell comprises the enzymatic activities of a phosphoglucosamine mutase, an *N*-acetylglucosamine-1-phosphate uridyltransferase and an UDP *N*-acetylglucosamine 2-epimerase with concomitant release of UDP for intracellular sialic acid biosynthesis. Thus, in an additional and/or alternative embodiment, the genetically engineered *Bacillus* cell being capable of synthesizing sialic acid does not comprise one or more enzymatic activities selected from the group consisting of the enzymatic activities of a phosphoglucosamine mutase, an *N*-acetylglucosamine-1-phosphate uridyltransferase and an UDP *N*-acetylglucosamine 2-epimerase with concomitant release of UDP.

In an additional and/or alternative embodiment, the genetically engineered *Bacillus* cell possesses glucosamine-6-phosphate *N*-acetyltransferase activity. Said glucosamine-6-phosphate *N*-acetyltransferase activity converts GlcN-6P to N-acetylglucosamine-6-phosphate (GlcNAc-6P). An example of a glucosamine-6-phosphate *N*-acetyltransferase is the *Saccharomyces cerevisiae* Gna1 (UniProtKB - P43577).

In an additional and/or alternative embodiment, the genetically engineered *Bacillus* cell contains a glucosamine-6-phosphate *N*-acetyltransferase, preferably a heterologous glucosamine-6-phosphate *N*-acetyltransferase, more preferably Gna1 from *S. cerevisiae* or a functional variant thereof.

Thus, in an additional and/or alternative embodiment, the genetically engineered *Bacillus* cell contains a nucleic acid molecule comprising and expressing a nucleotide sequence encoding a glucosamine-6-phosphate *N*-acetyltransferase, preferably *S. cerevisiae* Gna1, wherein said nucleotide sequence is operably linked to at least one nucleic acid expression control sequence effecting transcription and/or translation of said nucleotide sequence in the genetically engineered *Bacillus* cell to provide intracellular glucosamine-6-phosphate *N*-acetyltransferase activity.

In an additional and/or alternative embodiment, the codon usage of the nucleotide sequence encoding the heterologous glucosamine-6-phosphate *N*-acetyltransferase is adapted to the codon usage of *Bacillus.*

The recombinant glucosamine-6-phosphate *N*-acetyltransferase gene may be integrated into the *Bacillus* chromosome, or present as an episomal version on a plasmid within the *Bacillus* cell.

In an additional and/or alternative embodiment, the genetically engineered *Bacillus* cell possesses a *N*-acetylglucosamine-6-phosphate phosphatase activity. Said *N*-acetylglucosamine-6-phosphate phosphatase activity converts GlcNAc-6P to *N*-acetylglucosamine (GlcNAc). Examples of an *N*-acetylglucosamine-6-phosphate phosphatase are sugar phosphatases of the HAD-like superfamily which catalyze the conversion of GlcNAc6P to GlcNAc. The HAD-like superfamily of enzymes is named after the bacterial enzyme haloacid dehydrogenase and includes phosphatases. A suitable phosphatase of the HAD-like superfamily catalyzing the conversion of GlcNAc6P to GlcNAc may be selected from the group consisting of fructose-1-phosphate phosphatase (YqaB, UniProtKB - P77475) and alpha-D-glucose 1-phosphate phosphatase (YihX, UniProtKB - P0A8Y3). The *E. coli YqaB* and *E. coli* YihX enzymes are considered to also act on GlcNAc6P (Lee, S.-W. and Oh, M.-K. (2015) Metabolic Engineering 28: 143-150).

In an additional and/or alternative embodiment, the sugar phosphatase of the HAD-like superfamily catalyzing the conversion of GlcNAc-6P to GlcNAc is a heterologous enzyme in the genetically engineered *Bacillus* cell. In an additional and/or alternative embodiment, the sugar phosphatase of the HAD-like superfamily catalyzing the conversion of GlcNAc6P to GlcNAc is selected from the group consisting of *E. coli* YqaB, *E. coli* YihX, and functional variants thereof.

In an additional and/or alternative embodiment, the genetically engineered *Bacillus* cell contains a nucleic acid molecule which comprises and expresses a nucleotide sequence encoding a sugar phosphatase of the HAD-like superfamily catalyzing the conversion of GlcNAc6P to GlcNAc. In an additional and/or alternative embodiment, the nucleotide sequence encoding the sugar phosphatase of the HAD-like superfamily catalyzing the conversion of GlcNAc6P to GlcNAc is a heterologous nucleotide sequence. In an additional and/or alternative embodiment, the nucleotide sequence encoding the sugar phosphatase of the HAD-like superfamily catalyzing the conversion of GlcNAc6P to GlcNAc encodes the *E. coli* YqaB or *E. coli* YihX or a functional variant of one of these two enzymes.

In an additional and/or alternative embodiment, the codon usage of the nucleotide sequence encoding the heterologous *N*-acetylglucosamine-6-phosphate phosphatase is adapted to the codon usage of *Bacillus.*

The recombinant *N*-acetylglucosamine-6-phosphate phosphatase gene may be integrated into the *Bacillus* chromosome, or present as an episomal version on a plasmid within the *Bacillus* cell.

In an additional and/or alternative embodiment, the genetically engineered *Bacillus* cell possesses *N*-acetylglucosamine 2-epimerase activity. *N*-acetylglucosamine 2-epimerase (EC 5.1.3.8) is an enzyme that catalyzes the conversion of *N*-acetylglucosamine (GlcNAc) to *N*-acetylmannosamine (ManNAc). The enzyme is a racemase acting on carbohydrates and their derivatives. The systematic name of this enzyme class is *N*-acyl-D-glucosamine 2-epimerase. This enzyme participates in amino-sugar metabolism and nucleotide-sugar metabolism, preferably a heterologous *N*-acetylglucosamine 2-epimerase.

In an additional and/or alternative embodiment, the genetically engineered *Bacillus* cell comprises an *N*-acetylglucosamine 2-epimerase, preferably a heterologous *N*-acetylglucosamine 2-epimerase. Examples of *N*-acetylglucosamine 2-epimerases were described from *Anabena variabilis, Acaryochloris* sp., *Nostoc* sp., *Nostoc punctiforme, Bacteroides ovatus* or *Synechocystis* sp. An example of a suitable *N*-acetylglucosamine 2-epimerase is the *N*-acetylglucosamine 2-epimerase of B. *ovatus* ATCC 8483 (UniProtKB - A7LVG6) as encoded by gene BACOVA_01816. Another example is the *N*-acetylglucosamine 2-epimerase of *Synechocystis* sp. (strain PCC 6803) (UniProtKB - P74124) which is also known as renin-binding protein and is encoded by the *slr1975* gene.

In an additional and/or alternative embodiment, the genetically engineered *Bacillus* cell contains a nucleic acid molecule comprising a nucleotide sequence which encodes an *N*-acetylglucosamine 2-epimerase, preferably the *N*-acetylglucosamine 2-epimerase of *B. ovatus* ATCC 8483 or *Synechocystis* sp. (strain PCC 6803) or a functional variant thereof.

Hence, the nucleotide sequence which encodes the *N*-acetylglucosamine 2-epimerase is operably linked to at least one nucleic acid expression control sequence effecting transcription and/or translation of said nucleotide sequence which encodes a *N*-acetylglucosamine 2-epimerase in the genetically engineered *Bacillus* cell to provide intracellular *N*-acetylglucosamine 2-epimerase activity.

In an additional and/or alternative embodiment, the codon usage of the nucleotide sequence encoding the heterologous *N*-acetylglucosamine 2-epimerase is adapted to the codon usage of *Bacillus.*

The recombinant *N*-acetylglucosamine 2-epimerase gene may be integrated into the *Bacillus* chromosome, or present as an episomal version on a plasmid within the *Bacillus* cell.

In an additional and/or alternative embodiment, the genetically engineered *Bacillus* cell possesses *N*-acetylglucosamine-6-phosphate epimerase activity and *N*-acetylmannosamine-6-phosphate phosphatase activity. *N*-acetylglucosamine-6-phosphatase epimerase converts *N*-acetylglucosamine-6-phosphate (GlcNAc-6P) to *N*-acetylmannosamine-6-phosphate (ManNAc-6P), whereas *N*-acetylmannosamine-6-phosphate phosphatase dephosphorylates ManNAc-6P to give *N*-acetylmannosamine (ManNAc). Possessing *N*-acetylglucosamine-6-phosphate epimerase activity and *N*-acetylmannosamine-6-phosphate phosphatase activity provides an additional or alternative way for providing ManNAc for Neu5Ac production.

In an additional and/or alternative embodiment, the genetically engineered *Bacillus* cell contains an *N*-acetylglucosamine-6-phosphate epimerase. An example of a suitable *N*-acetylglucosamine-6-phosphate epimerase is *E. coli* NanE (UniprotKB P0A761) as encoded by the *E. coli nan*E gene.

Thus, in an additional and/or alternative embodiment, the genetically engineered *Bacillus* cell contains a nucleic acid molecule comprising and expressing a nucleotide sequence encoding an *N*-acetylglucosamine-6-phosphate epimerase, preferably a nucleotide sequence encoding *E. coli* NanE or a functional variant thereof.

Thus, in an additional and/or alternative embodiment, the genetically engineered *Bacillus* cell contains a nucleic acid molecule comprising and expressing a nucleotide sequence encoding the *N*-acetylglucosamine-6-phosphate epimerase, wherein said nucleotide sequence is operably linked to at least one nucleic acid expression control sequence effecting transcription and/or translation of said nucleotide sequence in the genetically engineered microbial cell to provide intracellular *N*-acetylglucosamine-6-phosphate epimerase activity.

In an additional and/or alternative embodiment, the codon usage of the nucleotide sequence encoding the heterologous *N*-acetylglucosamine-6-phosphate epimerase is adapted to the codon usage of *Bacillus.*

In an additional and/or alternative embodiment, the genetically engineered *Bacillus* cell contains an *N*-acetylmannosamine-6-phosphate phosphatase which converts ManNAc-6P to ManNAc.

Thus, in additional and/or alternative embodiment, the genetically engineered *Bacillus* cell contains a nucleic acid molecule comprising and expressing a nucleotide sequence encoding an *N*-acetylmannosamine-6-phosphate phosphatase. Hence, the nucleotide sequence which encodes the *N*-acetylmannosamine-6-phosphate phosphatase is operably linked to at least one nucleic acid expression control sequence effecting transcription and/or translation of said nucleotide sequence which encodes a *N*-acetylmannosamine-6-phosphate phosphatase in the genetically engineered *Bacillus* cell to provide intracellular *N*-acetylmannosamine-6-phosphate phosphatase activity.

In an additional and/or alternative embodiment, the codon usage of the nucleotide sequence encoding the heterologous *N*-acetylmannosamine-6-phosphate phosphatase is adapted to the codon usage of *Bacillus.*

The recombinant *N*-acetylmannosamine-6-phosphate phosphatase gene may be integrated into the *Bacillus* chromosome, or present as an episomal version on a plasmid within the *Bacillus* cell.

The genetically engineered *Bacillus* cell possesses cytidine 5'-monophospho-(CMP)-*N*-acetylneuraminic acid synthetase (Fig. 1: ⑪) activity for transferring cytidine 5'-monophosphate onto *N*-acetylneuraminic acid to generate a CMP-activated *N*-acetylneuraminic acid (CMP-NeuNAc). Several 5'-monophospho-(CMP)-sialic acid synthetases are known in the art and have been described, e.g. 5'-monophospho- (CMP)-sialic acid synthetases from *E. coli, Neisseria meningitidis, Campylobacter jejuni, Streptococcus sp.,* etc.

In an additional and/or alternative embodiment, the genetically engineered *Bacillus* cell contains a cytidine 5'-monophospho- (CMP)-*N*-acetylneuraminic acid synthetase, preferably a heterologous cytidine 5'-monophospho- (CMP)-*N*-acetylneuraminic acid synthetase, more preferably the *N*-acetylneuraminate cytidyltransferase NeuA from *C. jejuni.* The *C. jejuni* NeuA is encoded by the *C. jejuni neuA* gene.

Thus, in an additional and/or alternative embodiment, the genetically engineered *Bacillus* cell contains a nucleic acid molecule comprising and expressing a nucleotide sequence encoding a cytidine 5'-monophospho- (CMP)-*N*-acetylneuraminic acid synthetase, wherein said nucleotide sequence is operably linked to at least one nucleic acid expression control sequence effecting transcription and/or translation of said nucleotide sequence in the genetically engineered microbial cell to provide *N*-acetylneuraminate cytidyltransferase activity.

In an additional and/or alternative embodiment, the codon usage of the nucleotide sequence encoding the heterologous cytidine 5'-monophospho- (CMP)-*N*-acetylneuraminic acid synthetase is adapted to the codon usage of *Bacillus.*

The recombinant cytidine 5'-monophospho- (CMP)-*N*-acetylneuraminic acid synthetase gene may be integrated into the *Bacillus* chromosome, or present as an episomal version on a plasmid within the *Bacillus* cell.

The genetically engineered *Bacillus* cell possesses a sialyltransferase, preferably a heterologous sialyltransferase, and more preferably a sialyltransferase activity selected from the group consisting of α-2,3-sialyltransferase activity, α-2,6-sialyltransferase activity and/or α-2,8-sialyltransferase activity. The sialyltransferase activity is capable of transferring the *N*-acetylneuraminic acid moiety from the CMP-NeuNAc to an acceptor molecule, wherein said acceptor molecule is a saccharide molecule, to provide a sialylated saccharide.

In an additional and/or alternative embodiment, the genetically engineered microbial cell contains at least one sialyltransferase, preferably at least one heterologous sialyltransferase, wherein said sialyltransferase is capable of possessing an α-2,3-sialyltransferase activity and/or an α-2,6-sialyltransferase activity and/or an α-2,8-sialyltransferase activity for transferring the NeuNAc moiety from CMP-NeuNAc as donor substrate to the acceptor saccharide. Exemplary sialyltransferases and their genes are identified in Table 2.

The term "sialyltransferase" as used herein refers to polypeptides being capable of possessing sialyltransferase activity. "Sialyltransferase activity" refers to the transfer of a sialic acid residue, preferably of an *N*-acetylneuraminic acid (Neu5Ac) residue, from a donor substrate to an acceptor molecule. The term "sialyltransferase" comprises functional fragments of the sialyltransferases described herein, functional variants of the sialyltransferases described herein, and functional fragments of the functional variants. "Functional" in this regard means that the fragments and/or variants are capable of possessing sialyltransferase activity. Functional fragments of a sialyltransferase encompass truncated versions of a sialyltransferase as encoded by it naturally occurring gene, which truncated version is capable of possessing sialyltransferase activity. Examples of truncated versions are sialyltransferases which do not comprise a so-called leader sequence which typically directs the polypeptide to a specific subcellular localization. Typically, such leader sequences are removed from the polypeptide during its subcellular transport, and are also absent in the naturally occurring mature sialyltransferase.

The heterologous sialyltransferase is capable of transferring a sialic acid residue from a donor substrate to an acceptor molecule. The term "capable of" with respect to the heterologous sialyltransferase refers to the sialyltransferase activity of the heterologous sialyltransferase and the provision that suitable reaction conditions are required for the heterologous sialyltransferase to possess its enzymatic activity. In the absence of suitable reaction conditions, the heterologous sialyltransferase does not possess its enzymatic activity, but retains its enzymatic activity and possesses its enzymatic activity when suitable reaction conditions are restored. Suitable reaction conditions include the presence of a suitable donor substrate, the presence of suitable acceptor molecules, the presence of essential cofactors such as - for example - monovalent or divalent ions, a pH value in an appropriate range, a suitable temperature and the like. It is not necessary that the optimum values for each and every factor effecting the enzymatic reaction of the heterologous sialyltransferase is met, but the reaction conditions have to be such that the heterologous sialyltransferase performs its enzymatic activity. Accordingly, the term "capable of" excludes any conditions upon which the enzymatic activity of the heterologous sialyltransferase has been irreversibly impaired and also excluded exposure of the heterologous sialyltransferase to any such condition. Instead, "capable of" means that the sialyltransferase is enzymatically active, i.e. possesses its sialyltransferase activity, if permissive reactions conditions (where all requirements being necessary for the sialyltransferase to perform its enzymatic activity) are provided to the sialyltransferase.

Sialyltransferases can be distinguished on the type of sugar linkage they form. As used herein, the terms "α-2,3-sialyltransferase" and "α-2,3-sialyltransferase activity" refer to polypeptides and their enzymatic activity which add a sialic acid residue with an α-2,3 linkage to galactose, *N*-acetylgalactosamine or a galactose or N-acetylgalactosamine residue of the acceptor molecule. Likewise, the terms "α-2,6-sialyltransferase" and "α-2,6-sialyltransferase activity" refer to polypeptides and their enzymatic activity which add a sialic acid residue with an α-2,6 linkage to galactose, *N*-acetylgalactosamine or a galactose or *N*-acetylgalactosamine residue of the acceptor molecule. Likewise, the terms "α-2,8-sialyltransferase" and "α-2,8-sialyltransferase activity" refer to polypeptides and their enzymatic activity which add a sialic acid residue with an α-2,8 linkage to galactose, *N*-acetylgalactosamine or a galactose or *N*-acetylgalactosamine residue of the acceptor molecule.

In addition, the genetically engineered *Bacillus* cell has been genetically engineered to contain and express the nucleotide sequence encoding the heterologous sialyltransferase. To this end, the nucleotide sequence encoding the heterologous sialyltransferase is operably linked to at least one expression control sequence effecting transcription and/or translation of said nucleotide sequence encoding the heterologous sialyltransferase in the genetically engineered *Bacillus* cell to provide intracellular sialyltransferase activity.

In another embodiment, the heterologous sialyltransferase is capable of possessing α-2,6-sialyltransferase activity.

In an additional and/or alternative embodiment, the genetically engineered *Bacillus* cell contains and expresses a nucleic acid molecule which comprises at least one nucleotide sequence encoding said heterologous sialyltransferase being capable of possessing α-2,6-sialyltransferase activity.

In an additional and/or alternative embodiment, the heterologous sialyltransferase is capable of possessing α-2,8-sialyltransferase activity. An example of a heterologous sialyltransferase is capable of possessing α-2,8-sialyltransferase activity is the sialyltransferase Cstll of *Campylobacter jejuni* OH4384.

The sialyltransferase is capable of transferring a sialic acid residue, e.g. a *N-*acetylneuraminic acid (Neu5Ac) residue, from a donor substrate, e.g. CMP-Neu5Ac, to an acceptor molecule. The acceptor molecule is a saccharide molecule, preferably a saccharide molecule set forth in Table 3.

In an additional and/or alternative embodiment, the genetically engineered microbial cell contains a nucleic acid molecule which comprises and expresses at least one nucleotide sequence encoding said heterologous sialyltransferase being capable of possessing α-2,3-sialyltransferase activity.

**Table 2: List of sialyltransferase-encoding nucleotide sequences. The sialyltransferase-encoding nucleotide sequences were either cloned as full length constructs (FL) or without a predicted signal peptide (Δ) as compared to their wild-type protein coding regions. The number behind the Δ indicates the N-terminal amino acids deleted from the corresponding sequence.**

| Origin of the sialyltransferase gene | accession number of the sialyltransferase gene | Cloned as full length (FL) gene or without signal peptide (Δ) |
|---|---|---|
| *Neisseria meningitidis* | U60660 | FL |
| *Campylobacter jejuni* strain OH4384 | AF130466 | FL |
| *Campylobacter jejuni* strain OH4384 | AX934425 | FL |
| *Helicobacter acinonychis* | NC_008229 | FL |
| *Helicobacter acinonychis* | NC_008229 | FL |
| *Photobacterium* sp. JT-ISH-224 | BAF92026 | Δ17 |
| *Pasteurella dagmatis* strain DSM 22969 | AFY98851 | FL |
| *Photobacterium* sp. JT-ISH-224 | BAF92025 | Δ20 |
| *Vibrio* sp. JT-FAJ-16 | BAF91160 | Δ22 |
| *Pasteurella multocida* PM70 | AAK02272 | Δ25 |
| *Photobacterium damselae* JT0160 | BAA25316 | FL |
| *Streptococcus agalactiae* | AB050723 | FL |
| *Haemophilus-somnus-*2336 | ACA31578 | FL |
| *Haemophilus ducreyi* 35000HP | AF101047 | FL |
| *Haemophilus ducreyi* 35000HP | AAP95068 | FL |
| *Photobacterium phosphoreum* JT-ISH-467 | BAF63530 | Δ20 |
| *Photobacterium leiognathi* JT-SHIZ-119 | AB500947 | Δ15 |
| *Photobacterium leiognathi* JT-SHIZ-145 | BAF91416 | Δ15 |
| *Campylobacter coli* | YP_008473374 | FL |
| *Vibrio harveyi* | WP_017817635 | Δ24 |
| *Streptococcus entericus* | WP_018369230 | FL |
| *Avibacterium paragallinarum* | WP_021724759 | FL |
| *Haemophilus parahaemolyticus* HK385 | EIJ71207 | FL |
| *Alistipes* sp. CAG:268 | CDC95697 | Δ17 |
| *Alistipes* sp. AL-1 | WP_032134786 | FL |
| *Pasteurella multocida* PM70 | NC_002663 | FL |
| *Campylobacter jejuni* strain 81-176 | AAL09368 | FL |
| *Alistipes shahii* WAL 8301 | YP_007816735 | Δ21 |
| *Actinobacillus suis* ATCC 33415 | AIJ32009 | FL |
| *Actinobacillus capsulatus* DSM 19761 | WP_018652686 | FL |
| *Bibersteinia trehalosi USDA-ARS-USMARC-189* | AHG84654 | FL |
| *Photobacterium damselae subsp. damselae* CIP 102761 | EEZ40509 | FL |
| *Haemophilus somnus* 2336 | ACA31170 | FL |
| *Streptococcus suis* | AGL48117 | FL |
| *Streptococcus suis* | EEF64118 | FL |
| *Photobacterium phosphoreum* | CDN97322 | |
| *Alistipes timonensis* | WP_026020701 | |
| *Haaemophilus parasuis* | KEZ22922 | |
| *Acinetobacter pittii* | EOQ74854 | |
| *Photobacterium leiognathi* | BAI49484 | |
| *Porphyroonas sp.* | KGO0080 | |
| *Photobacterium damsalae* | WP_005298232 | |
| *Pasteurella multocida* | EGP04633 | |
| *Actinobacillus ureae* | WP_005624339 | |
| *Actinobacillus equuli* | WP_039196193 | |
| *Acinetobacter calcoaceticus* | WP_017391462 | |
| *Acinetobacter baumannii* | WP_032039596 | |
| *Acinetobacter oleivorans* | WP_042898369 | |
| *Acinetobacter baumannii* | ELX04805 | |
| *Acinetobacter gyllenbergii* | WP_032865812 | |
| *Shigella boydii* | ACD37071 | |
| *Kingella kingae* | WP_026036248 | |
| *Alysiella crassa* | WP_034294366 | |
| *Alistipes sp.* | EFR56292 | |
| *Streptococcus suis* | WP_024397787 | |
| *Fusobacterium mortiferum* | EEO36694 | |
| *Porphyromonas catoniae* | WP_005467220 | |

In an additional and/or alternative embodiment, the acceptor molecule is a monosaccharide, preferably a monosaccharide selected from the group consisting of *N*-acetylglucosamine, galactose and *N*-acetylgalactosamine.

In an additional and/or alternative embodiment, the acceptor molecule is a disaccharide, preferably a disaccharide selected from the group consisting lactose, lactulose, *N*-acetyllactosamine, lacto-*N*-biose, lactulose and melibiose.

In an additional and/or alternative embodiment, the acceptor molecule is a trisaccharide, preferably a trisaccharide selected from the group consisting of raffinose, lacto-*N*-triose II, 2'-fucosyllactose, 3-fucosyllactose, 3'-sialyllactose, 6'-sialyllactose, 3'-sialyl-*N*-acetyllactosamine, 6'-sialyl-*N*-acetyllactosamine, 3'-galactosyllactose and 6'-galactosyllactose.

**Table 3: List of saccharides that may be used as acceptor substrate for the production of a sialylated saccharide. The sialylated oligosaccharide itself may also be used as acceptor substrate for the production of a further sialylated oligosaccharide.**

| Name | Abbreviation | Structure |
|---|---|---|
| *N*-acetylglucosamine | GlcNAc | GlcNAc |
| Galactose | Gal | Gal |
| *N-*acetylgalactosamine | GalNAc | GalNAc |
| Lactose | Lac | Gal(β1,4)Glc |
| *N*-acetyllactosamine | LacNAc | Gal(β1,4)GlcNAc |
| Lacto-*N*-biose | LNB | Gal(β1,3)GlcNAc |
| Lactulose | LacU | Gal(β1,4)Frc |
| Melibiose | Mel | Gal(α1,6)Glc |
| Raffinose | Raf | Gal(α1,6)Glc(α1,2)Frc |
| 2'-Fucosyllactose | 2'-FL | Fuc(α1,2)Gal(β1,4)Glc |
| 3-Fucosyllactose | 3-FL | Gal(β1,4)[Fuc(α1,3)]Glc |
| 2',3-Difucosyllactose | DFL | Fuc(α1,2)Gal(β1,4)[Fuc(α1,3)]Glc |
| 6'-Galactosyllactose | 6'-GL | Gal(β1,6)Gal(β1,4)Glc |
| 3'-Galactosvllactose | 3'-GL | Gal(β1,3)Gal(β1,4)Glc |
| Lacto-*N*-triose II | LNT II | GlcNAc(β1,3)Gal(β1,4)Glc |
| Lacto-*N*-tetraose | LNT | Gal(β1,3)GlcNAc(β1,3)Gal(β1,4)Glc |
| Lacto-*N-neo*tetraose | LN*n*T | Gal(β1,4)GlcNAc(β1,3)Gal(β1,4)Glc |
| Lacto-*N-*fucopentaose I | LNFP I | Fuc(α1,2)Gal(β,3)GlcNAc(β1,3)Gal(β1,4)Glc |
| Lacto-*N-neo*fucopentaose I | LNnFP I | Fuc(α1,2)Gal(β1,4)GlcNAc(β1,3)Gal(β1,4)Glc |
| Lacto-*N-*fucopentaose II | LNFP II | Gal(β1,3)[Fuc(α1,4)]GlcNAc(β1,3)Gal(β1,4)Glc |
| Lacto-*N-*fucopentaose III | LNFP III | Gal(β1,4)[Fuc(α1,3)]GlcNAc(β1,3)Gal(β1,4)Glc |
| Lacto-*N-*fucopentaose V | LNFP V | Gal(β1,3)GlcNAc(β1,3)Gal(β1,4)[Fuc(α1,3)]Glc |
| Lacto-*N-neo*fucopentaose V | LNnFP V | Gal(β1,4)GlcNAc(β1,3)Gal(β1,4)[Fuc(α1,3)]Glc |
| Lacto-*N-*difucohexaose I | LNDH I | Fuc(α1,2)Gal(β1,3)[Fuc(α1,4)]GlcNAc(β1,3)Gal(β1,4)Glc |
| Lacto-*N-*difucohexaose II | LND | Gal(β1,3)[Fuc(α1,4)]GlcNAc(β1,3)Gal(β1,4)[Fuc(α1,3)]Glc |
| Lacto-*N-neo*difucohexaose I | LNnDFH I | Gal(β1,4)[Fuc(α1,3)]GlcNAc(β1,3)Gal(β1,4)[Fuc(α1,3)]Glc |
| Lacto-*N*-hexaose | LNH | Gal(β1,4)GlcNAc(β1,6)[Gal(β1,3)GlcNAc(β1,3)]Gal(β1,4)Glc |
| Lacto-*N-neo*hexaose | LNnH | Gal(β1,4)GlcNAc(β1,6)[Gal(β1,4)GlcNAc(β1,3)]Gal(β1,4)Glc |
| *para*-Lacto-*N-*hexaose | paraLNT | Gal(β1,3)GlcNAc(β1,3)Gal(β1,4)GlcNAc(β1,3)Gal(β1,4)Glc |
| *para*-Lacto-*N-neo*hexaose | paraLNnH | Gal(β1,4)GlcNAc(β1,3)Gal(β1,4)GlcNAc(β1,3)Gal(β1,4)Glc |
| 3'-Sialyllactose | 3'-SL | Neu5Ac(α2,3)Gal(β1,4)Glc |
| 6'-Sialyllactose | 6'-SL | Neu5Ac(α2,6)Gal(β1,4)Glc |
| Sialyllacto-*N-*tetraose a | LSTa | Neu5Ac(α2,3)Gal(β1,3)GlcNAc(β1,3)Gal(β1,4)Glc |
| Sialyllacto-*N-*tetraose b | LSTb | Gal(β1,3)[Neu5Ac(α2,6)]GlcNAc(β1,3)(Gal(β1,4)Glc |
| Sialyllacto-*N-*tetraose c | LSTc | Neu5Ac(α2,6)Gal(β1,4)GlcNAc(β1,3)Gal(β1,4)Glc |
| Fucosyllacto-*N-*sialylpentaose a | F-LST-a | Neu5Ac(α2,3)Gal(β1,3)[Fuc(α1,4)]GlcNAc(β1,3)Gal(β1,4)Glc |
| Fucosyllacto-*N* sialylpentaose b | F-LST-b | Fuc(α1,2)Gal(β1,3)[Neu5Ac(α2,6)]GlcNAc(β1,3)Gal(β1,4)Glc |
| Fucosyllacto-*N-*sialylpentaose c | F-LST-c | Neu5Ac(α2,3)Gal(β1,3)GlcNAc(β1,3)Gal(β1,4)[Fuc(α1,3)]Glc |
| Disialyllacto-*N-*tetraose | DS-LNT | Neu5Ac(α2,3)Gal(β1,3)[Neu5Ac(α2,6)]GlcNAc(β1,3)Gal(β1,4)Glc |
| 3-Fucosyl-3'-sialyllactose | 3F-3'-SL | Neu5Ac(α2,3)Gal(β1,4)[Fuc(α1,3)]Glc |
| 3-Fucosyl-6'-sialyllactose | 3F-6'-SL | Neu5Ac(α2,6)Gal(β1,4)[Fuc(α1,3)]Glc |
| 3'-sialyl-*N-*acetyllactosamine | 3'-SLN | Neu5Ac(α2,3)Gal(β1,4)GlcNAc |
| 6'-sialyl-*N-*acetyllactosamine | 6'-SLN | Neu5Ac(α2,6)Gal(β1,4)GlcNAc |

In an additional and/or alternative embodiment, the acceptor molecule is a tetrasaccharide, preferably a tetrasaccharide selected from the group consisting of lacto-*N*-tetraose, lacto-*N*-*neo*tetraose, 2'3-difucosyllactose, 3-fucosyl-3'-sialyllactose and 3-fucosyl-6'-sialyllactose.

In an additional and/or alternative embodiment, the acceptor molecule is a pentasaccharide, preferably a pentasaccharide selected from the group consisting of sialyllacto-*N*-tetraose a, sialyllacto-*N*-tetraose b, sialyllacto-*N*-tetraose c, lacto-*N-*fucopentaose I, lacto-*N*-fucopentaose II, lacto-*N*-fucopentaose III, lacto-*N-*fucopentaose V, lacto-*N*-neofucopentaose I and lacto-*N*-neofucopentaose V.

It is to be understood that a *Bacillus* cell already carrying one or more genes encoding said enzymes, and expressing said genes in a manner sufficient to produce NeuNAc, CMP-NeuNAc and/or the sialylated saccharide does not need to be genetically engineered to complete sialic acid biosynthesis and to transfer a sialic acid moiety to a saccharide acceptor, but may nevertheless be genetically engineered to alter the expression level of one or more of said genes to increase the intracellular level of said one or more gene products such as - for example the quantity of glutamine:fructose-6-phosphate aminotransferase, glucosamine-6-phosphate *N*-acetyltransferase, *N*-acetylglucosamine-6-phosphate phosphatase, *N*-acetylglucosamine 2-epimerase and/or *N*-acetylneuraminic acid synthase, thus increasing the rate of Neu5Ac biosynthesis and, as a consequence, of the sialylated saccharide, in the genetically engineered cell.

In some embodiments, the *Bacillus* cell is for use in the production of 3'-SL and has been genetically engineered to possess a lactose permease, a CMP-NeuNAc biosynthesis pathway that uses GlcN-1P as intermediate, and a α-2,3-sialyltransferase.

In various embodiments, the *Bacillus* cell is for use in the production of 6'-SL and has been genetically engineered to possess a lactose permease, a CMP-NeuNAc biosynthesis pathway that uses GlcN-1P as intermediate, and a α-2,6- sialyltransferase.

In an additional and/or alternative embodiment, the genetically engineered *Bacillus* cell synthesizes more PEP than the wildtype of the cell. In an additional and/or alternative embodiment, the genetically engineered microbial cell has been genetically engineered to possess an enhanced PEP biosynthesis pathway. Preferably, the genetically engineered microbial cell has been genetically engineered to possess an increased phosphoenolpyruvate synthase activity, for example in that the *pps*A gene encoding phosphoenolpyruvate synthase gene is overexpressed and/or in that the non-naturally-occurring microorganisms contains at least one additional copy of a nucleotide sequence allowing the expression of a phosphoenolpyruvate synthase or a functional variant thereof. Overexpression of ppsA enhances intracellular PEP synthesis such that more PEP is available for the production of sialic acid. For example, a suitable phosphoenolpyruvate synthase is PpsA of *E. coli.*

In an additional and/or alternative embodiment, the genetically engineered *Bacillus* cell contains a nucleic acid molecule comprising a nucleotide sequence encoding E. *coli* PpsA or a functional variant thereof. Said nucleotide sequence encoding *E. coli* PpsA or a functional variant thereof has a sequence identity of at least 80%, at least 85%, at least 90%, at least 95%, at least 98% or at least 99% to the *E. coli pps*A gene.

In an additional and/or alternative embodiment, the genetically engineered *Bacillus* cell is further modified to be capable to transfer said sole carbon source into the cell via a mechanism, that is not consuming PEP.

In an additional and/or alternative embodiment, the *Bacillus* cell for producing a sialylated oligosaccharide lacks any β-galactosidase activity or possesses reduced β-galactosidase activity as compared to a wild type *Bacillus* cell of the same species.

The intracellular biosynthesis of sialylated oligosaccharides requires import of lactose as acceptor substrate for the lactose-accepting sialyltransferase. Any intracellular enzyme activity that hydrolyzes internalized lactose would affect the efficacy of sialyllactose formation as the pool of intracellular lactose would be diminished. Therefore, it would be advantageous if the *Bacillus* cell for producing a sialylated oligosaccharide would lack or at least possess - as compared to the wild-type *Bacillus* cell - reduced beta-galactosidase activity.

In an additional and/or alternative embodiment, the *Bacillus* cell has been genetically engineered to abolish or at least reduce the cell's β-galactosidase activity.

In an additional and/or alternative embodiment, the *Bacillus* cell has been genetically engineered by deletion or functional inactivation of the ganA gene. In another embodiment, the *Bacillus* cell has been genetically engineered to reduce the expression level of the *ganA* gene - as compared to the wild-type *Bacillus* cell.

The *Bacillus ganA* gene is also called *yvfN* or *lac*A*.* It is a gene of the GanR regulon which contains genes encoding enzymes involved in the utilization of galactan. The ganA gene encodes a beta-galactosidase that is involved in the galactan utilization of *Bacillus.*

Deletion or functional inactivation of the ganA gene abolishes the GanA-mediated β-galactosidase activity in the *Bacillus* cell, whereas reduction of the *gan*A expression lowers the amount of GanA in the *Bacillus* cell and hence the β-galactosidase activity which could interfere with the biosynthesis of a sialylated oligosaccharide.

In an additional and/or alternative embodiment, the *Bacillus* cell has been genetically engineered by deletion or functional inactivation of the *yesZ* gene. The *Bacillus yesZ* gene encodes the beta-galactosidase YesZ which plays a role in the degradation of rhamnogalacturonan being derived from plant cell walls. The expression of *Bacillus yesZ* gene is induced by rhamnogalacturonan I. In another embodiment, the *Bacillus* cell has been genetically engineered to reduce the expression level of the *yesZ* gene - as compared to the wild-type *Bacillus* cell.

Deletion or functional inactivation of the *yesZ* gene abolishes the YesZ-mediated β-galactosidase activity in the *Bacillus* cell, whereas reduction of the *yesZ* expression lowers the amount of YesZ in the *Bacillus* cell and hence the β-galactosidase activity which could interfere with the biosynthesis of a sialylated oligosaccharide.

When *B. subtilis* enters the post-exponential growth phase, they (start to) produce large quantities of extracellular proteases. Foreign proteins are often protease sensitive. Therefore, an exoprotease-free strain is desirable to increase the stability of heterologous proteins and to allow accumulation of high levels of foreign proteins. The genome of *Bacillus* encodes for at least eight extracellular proteases, namely *nprE, aprE, epr, bpr, mpr, nprB, vpr* and *wprA.* Thus, in an additional and/or alternative embodiment, the *Bacillus* cell has been genetically engineered in that at least one gene encoding for an extracellular protease has been deleted or functionally inactivated, preferably at least one of the genes selected from the group consisting of *nprE, aprE, epr, bpr, mpr, nprB, vpr* and *wprA.* Preferably, two, three, four, five six, seven or eight of the genes selected from the group consisting of *nprE, aprE, epr, bpr, mpr, nprB, vpr* and *wprA* have been deleted or functionally inactivated.

*B. subtilis* synthesizes pulcherriminic acid when growing in media containing a carbohydrate such as glucose or lactose. Excreted pulcherriminic acid forms the red pigment pulcherrimin, the salt of pulcherriminic acid (a ferric chelate), when iron is present in the growth medium. The formation of this undesired by-product during fermentation processes can be avoided/abolished by deletion or disruption of the genes *yvmC* and/or *cypX.* The gene *yvmC* encodes a cyclodipeptide synthase and the gene *cypX* encodes a cytochrome P450 cyclo-I-leucyl-I-leucyl dipeptide oxidase.

Thus, in an additional and/or alternative embodiment, the *Bacillus* cell has been genetically engineered in that at least one of the genes *yvmC* and *cypX* has been deleted or functionally inactivated.

The rhizobacterium *B. subtilis* possesses genes for the synthesis of more than 20 antibiotics. Among them are peptide antibiotics like *Bacillus subtilis* lantibiotics and lantibiotic-like peptides (subtilin, ericin S, mersacidin, sublancin 168, subtilosin A) and non-ribosomally synthesized (peptide) antibiotics (surfactin, iturin, bacillomycin, mycosubtilin, corynebactin/ bacillibactin, fengycin plipastatin, mycobacillin, TL-119, bacilysin, bacilysocin, amicoumacin, 3,3'-neotrehalosadiamine, difficidin, rhizocticin).

For producing a sialylated oligosaccharide, it is preferred to use a *Bacillus* cell that does not produce an antibiotic. Thus, in an additional and/or alternative embodiment, the *Bacillus* cell does not synthesize one or more of the antibiotics selected from the group consisting of lantibiotics and lantibiotic-like peptides such as subtilin, ericin S, mersacidin, sublancin 168, subtilosin A; non-ribosomally synthesized (peptide) antibiotics such as surfactin, iturin, bacillomycin, mycosubtilin, corynebactin/ bacillibactin, fengycin plipastatin, mycobacillin, TL-119, bacilysin, bacilysocin, amicoumacin, 3,3'-neotrehalosadiamine, difficidin and rhizocticin. The *Bacillus* cell may have been genetically engineered to impair or abolish their ability to synthesize one or more of said antibiotics.

The genetically engineered *Bacillus* cell that can produce sialylated oligosaccharides may - optionally - include additional features, and may be genetically engineered to possess these additional features. These additional features are considered to improve the productivity of the non-naturally-occurring microorganism leading to higher sialylated oligosaccharide yields.

The *Bacillus* cell of the invention is able to produce a sialylated oligosaccharide when cultivated in the presence of lactose in a medium and under conditions that are permissive for the *Bacillus* cell to produce the sialylated oligosaccharide. Thus, provided is also the use of a genetically engineered *Bacillus* cell as described herein before for the production of a sialylated oligosaccharide.

In an aspect, provided is a method for the production of a sialylated oligosaccharide. The method comprises (i) providing a *Bacillus* cell as describe herein before, (ii) cultivating said *Bacillus* cell in a culture medium or fermentation broth and under conditions that are permissive for the production of the sialylated oligosaccharide. The method may further include retrieving the sialylated oligosaccharide from the culture medium/fermentation broth and/or from the *Bacillus* cell.

The fermentation broth contains at least one carbon source for the *Bacillus* cell's metabolism. In some embodiments, the at least one carbon source is selected from the group consisting of glucose, fructose, sucrose, glycerol and combinations thereof. The carbon source can be internalized by the *Bacillus* cell and may be sued by the *Bacillus* cell's metabolism to generate biomass and/or energy in form of energy rich triphosphates.

In some embodiments, the fermentation broth contains lactose, in particular if the *Bacillus* cell is not able to synthesize lactose by its own. The lactose may be supplied exogenously to the fermentation broth. Lactose may serve as acceptor substrate for a NeuNAc moiety in the production of some sialylated oligosaccharides, in particular in the production of sialyllactoses.

In various embodiments, the fermentation broth contains sialic acid, in particular if a *Bacillus* cell is used for the production of a sialylated oligosaccharide which does not possess a sialic acid biosynthesis pathway, but a sialic acid salvage pathway.

In some embodiments, the production of a sialylated oligosaccharide does not require addition of *N*-acetylglucosamine, *N*-acetylmannosamine and/or *N*-acetylneuraminic acid to the fermentation broth and/or cultivating the genetically engineered microbial cell in the presence of *N*-acetylglucosamine, *N*-acetylmannosamine and/or N-acetylneuraminic acid for the intracellular biosynthesis of the sialylated oligosaccharide, because a *Bacillus* cell is used for the production of the sialylated oligosaccharide which possesses a *de novo* sialic acid biosynthesis pathway for intracellular biosynthesis of NeuNAC.

In the method, the at least one genetically engineered *Bacillus* cell is cultivated in a fermentation broth and under conditions which are permissive for the production of the saccharide comprising at least one *N*-acetylneuraminic acid moiety.

While the process employs a carbon source in the fermentation broth for the genetically engineered *Bacillus* cell, it is not necessary to add glucosamine and/or *N*-acetylneuraminic acid and/or *N*-acetylglucosamine and/or *N*-acetylmannosamine to the fermentation broth when the *Bacillus* cell is able to intracellularly produce *N*-acetylneuraminic acid. Thus, in various embodiments for the production of a sialylated oligosaccharide, the genetically engineered *Bacillus* cell is cultivated in the absence of and/or without addition of one or more selected from the group consisting of glucosamine, *N*-acetylglucosamine, *N*-acetylmannosamine and *N*-acetylneuraminic acid. The genetically engineered *Bacillus* cell may be cultivated in the absence and/or without addition of galactose, as far as galactose is not supplied as an acceptor substrate for the sialyltransferase reaction.

In additional and/or alternatives embodiments, the genetically engineered *Bacillus* cell is cultivated in the presence of one or more monosaccharides (e.g. galactose), disaccharides (e.g. lactose), trisaccharides (e.g. lacto-*N*-triose II), tetrasaccharides (e.g. lacto-*N*-tetraose) and/or pentasaccharides (e.g. sialyllacto-*N*-tetraose a).

According to an additional and/or alternative embodiment, the genetically engineered *Bacillus* cell is cultivated in the presence of at least one acceptor substrate selected from the group consisting of galactose, *N*-acetylgalactosamine, *N*-acetylglucosamine, lactose, lactulose, *N*-acetyllactosamine, lacto-*N*-biose, lacto-*N*-triose, 2'-fucosyllactose, 3-fucosyllactose, 3'-sialyllactose, 6'-sialyllactose, 3'-sialyl-*N*-acetyllactosamine, 6'-sialyl-*N*-acetyllactosamine, 3'-galactosyllactose, 6'-galactosyllactose, lacto-*N*-triose II, lacto-*N*-tetraose, lacto-*N*-*neo*tetraose, 2'3-difucosyllactose, 3-fucosyl-3'-sialyllactose and 3-fucosyl-6'-sialyllactose. These substrates are imported into the cell and used as acceptor molecules in the cell.

The method comprises the optional step of recovering the sialylated oligosaccharide that has been produced by the genetically engineered *Bacillus* cell during its cultivation and proliferation in the fermentation broth. The sialylated oligosaccharide can be recovered from the fermentation broth after the genetically engineered *Bacillus* cells have been removed, for example by centrifugation or filtration, and/or can be recovered from the cells, for example in that the cells are harvested from the fermentation broth by centrifugation and are subjected to a cell lysis step. Subsequently, the sialylated oligosaccharides can be further purified from the fermentation broth and/or cell lysates by suitable techniques known to the skilled artisan. Suitable techniques include microfiltration, ultrafiltration, diafiltration, simulated moving bed type chromatography, electrodialysis, reverse osmosis, gel filtration, anion exchange chromatography, cation exchange chromatography, and the like.

The method and the genetically engineered microbial cell that is employed in the method are used for the production of a sialylated oligosaccharide. The term "sialylated oligosaccharide" refers to an oligosaccharide molecule comprising at least one *N*-acetylneuraminic acid moiety.

In an additional and/or alternative embodiment, the sialylated saccharide is an oligosaccharide. The term "oligosaccharide" as used herein refers to polymers of monosaccharide residues, wherein said polymers comprise at least two monosaccharide residues, but no more than 10 monosaccharide residues, preferably no more than 7 monosaccharide residues. The oligosaccharides are either a linear chain of monosaccharides or are branched. In addition, the monosaccharide residues of the oligosaccharides may feature a number of chemical modifications. Accordingly, the oligosaccharides may comprise one or more non-saccharide moieties. The term "sialylated oligosaccharide" as used herein refers to oligosaccharides comprising one or more *N*-acetylneuraminic acid moieties.

According to additional and/or alternative embodiment, the sialylated oligosaccharide is selected from the group consisting of 3'-sialyllactose, 6'-sialyllactose, sialyllacto-*N*-tetraose a, sialyllacto-*N*-tetraose b, sialyllacto-*N*-tetraose c, fucosyl-sialyllacto-*N*-tetraose a, fucosyl-sialyllacto-*N*-tetraose b, fucosyl-sialyllacto-*N*-tetraose c, disialyllacto-*N*-tetraose, fucosyldisialyllacto-*N*-tetraose I, fucosyldisialyllacto-*N*-tetraose II, 3'-sialylgalactose, 6'-sialylgalactose, 3'-sialyl-*N*-acetyllactosamine and 6'-sialyl-*N*-acetyllactosamine.

In another aspect of the invention, the use of a genetically engineered *Bacillus* cell as described herein before for the production of a sialylated oligosaccharide in a whole cell fermentation process is provided, i.e. the sialylated oligosaccharide is synthesized by and in the genetically engineered *Bacillus* cell.

In another aspect of the invention, a sialylated oligosaccharide is provided that has been produced by the method and/or by using the genetically engineered *Bacillus* cell as described herein before.

In another aspect of the invention, provided is the use of a sialylated oligosaccharide that has been produced by a method as described herein before and/or by using the genetically engineered *Bacillus* cell as described herein before for the manufacture of a nutritional composition.

Thus, provided is a nutritional composition containing at least one sialylated oligosaccharide which has been produced by the method and/or by the genetically engineered *Bacillus* cell as described herein before.

In an additional and/or alternative embodiment, the sialylated oligosaccharide is selected from the group consisting of 3'-sialyllactose, 6'-sialyllactose, sialyllacto*-N-*tetraose a, sialyllacto-*N*-tetraose b, sialyllacto-*N*-tetraose c, fucosyl-sialyllacto-*N-*tetraose a, fucosyl-sialyllacto-*N*-tetraose b, fucosyl-sialyllacto-*N*-tetraose c, disialyllacto-*N*-tetraose, fucosyldisialyllacto-*N*-tetraose I, fucosyldisialyllacto-*N*-tetraose II, 3'-sialylgalactose, 6'-sialylgalactose, 3'-sialyl-*N*-acetyllactosamine and 6'-sialyl-N-acetyllactosamine.

In some embodiments, the nutritional composition further contains at least one neutral HMO, preferably at least one neutral HMO that has been produced by using a genetically engineered *Bacillus* cell. The at least one neutral HMO is selected from the group consisting of 2'-fucosyllyctose, 3-fucosyllactose, 2',3-difucosyllactose, lacto-*N*-triose II, lacto-*N*-tetraose, lacto-*N*-*neo*tetraose, lacto-*N*-fucopentaose I, lacto-*N*-neofucopentaose I, lacto-*N*-fucopentaose II, lacto-*N*-fucopentaose III, lacto-*N*-fucopentaose V, lacto-*N*-neofucopentaose V, lacto-*N*-difucohexaose I, lacto-*N-*difucohexaose II, lacto-*N*-*neo*difucohexaose I, lacto-*N*-hexaose, lacto-*N*-*neo*hexaose, *para*-lacto-*N*-hexaose, and *para*-lacto-*N*-*neo*hexaose.

In an additional embodiment, the nutritional composition is selected from the group consisting of medicinal compositions, pharmaceutical compositions, neutraceutical formulations, infant formula and dietary supplements.

The nutritional composition may be present in liquid form or in solid form including, but not limited to, powders, granules, flakes and pellets.

The present invention will be described with respect to particular embodiments, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding. The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

### Examples

### Example 1: Transformation of Bacillus subtilis

*Bacillus subtilis* can be genetically manipulated by various techniques. For transformation of *B. subtilis,* competent cells were prepared by a modified protocol of the two-step method (Anagnostopoulos, C. and Spizizen, J. (1961) J Bacteriol 81 (5): 741-746). An overnight culture was inoculated in MG1 medium and shaken at 37 °C. MG1 medium is Spizizen's minimal medium, which is supplemented with 0.5 % glucose, 5 mM MgSO₄ and 0.02 % casamino acids (optionally additionally supplemented with biotin and/or L-tryptophan). At the next morning, this culture was diluted 1:20 in fresh MG1 medium and incubated at 37 °C for approximately 6 h. 1 ml of the culture was diluted in 8 ml MG2 medium which differs from MG1 medium in the concentration of casamino acids (0.01 % instead of 0.02 %). In a shortened protocol the overnight culture is directly diluted in MG2 medium. After incubation for another 90 min, a 1-ml portion of the culture was mixed with 1-3 µg multimeric plasmid DNA or linear DNA and incubated at 37 °C for 30-60 min with shaking. Multimeric plasmid DNA was obtained either by using *E. coli* strain NM538 for propagation of plasmid DNA or by linearization of the plasmid by digestion with a single-cutter restriction enzyme, which cleaves within the backbone, followed by re-ligation with T4 DNA ligase.

Afterwards, cells were spread on 2x YT agar plates containing the appropriate antibiotic. Antibiotics were added in the following concentrations: 5 µg·mL⁻¹ erythromycin, 5 µg·mL⁻¹ chloramphenicol, 10 µg·mL⁻¹ kanamycin,100 µg·mL⁻¹ spectinomycin.

Alternatively, for protoplast transformation (Romero, D. et al. (2006) Journal of Microbiological Methods 66:556-559), cells were grown in 20 ml of Penassay broth (PAB) at 37 °C until the onset of the stationary phase of growth (OD₆₀₀=1.7-2). Cells were then pelleted and resuspended in 10 ml of SMPP medium (0.3% bovine serum albumin, 5% 2 M sucrose, 25% 4x PAB, 50% 2x SMM), composition of 2× SMM being 1 M sucrose, 0.04 M maleic acid disodium salt hydrate and 0.04 M MgCl₂ (pH 6.5). After the addition of lysozyme (10 mg ml⁻¹) and mutanolysin (75 U ml⁻¹) the mixture was incubated at 37 °C with shaking to generate protoplasts. Protoplast formation was checked microscopically. Protoplasts were then carefully harvested by centrifugation at 5200 xg and 4 °C for 5 min, washed twice with ice cold washing electrotransformation buffer (1× SMM) and finally suspended in this solution. Plasmid DNA (1-3 µg) was added to 120 µl of protoplast suspension and the mixture was kept on ice for at least 5 min. The transformation mixture was transferred to a 0.2-cm cuvette and a single electroporation pulse was applied at 25 µF, 400 Ω and 0.7 kV. Immediately after the electroporation shock 1 ml of recovering medium (equal volumes of 4x PAB and 2x SMM, prepared freshly before use) was added to the cuvette. The transformation reaction was then transferred to a 2 ml tube and incubated at 37 °C with shaking for 12 h. For regeneration, the cell suspension was spread on DM3 agar plates (Chang, S. and Cohen, S. (1979) MGG 168(1):111-115) and incubated at 37 °C for 48 h. DM3 regeneration medium contained the following sterile solutions per liter: 200 ml 4% agar, 100 ml 5% casamino acids, 50 ml 10% yeast extract, 100 ml 3.5% K₂HPO₄ and 1.5% KH₂PO₄, 25 ml 20% glucose, 20 ml 1 M MgCl₂, 500 ml 0.5 M sorbitol and 5 ml filter sterilized 2% bovine serum albumin (added to the mixture when the temperature is below 55 °C) and was supplemented with the appropriate antibiotic.

Electroporation of *B. subtilis* was done according to a modified protocol from Zhang et al. (2011) provided by MoBiTec GmbH (Zhang,G., Bao,P., Zhang,Y., Deng,A., Chen,N. and Wen,T. (2011) Anal. Biochem., 409:130-137). A 2x YT overnight culture was diluted 100-fold with fresh 2x YT medium and the culture was grown to an OD₆₀₀ of 0.2 at 37 °C on a rotary shaker. Then the culture was supplemented with 1% DL-threonine, 2% glycine, 0.1% tryptophan and 0.03% Tween 80. After cultivation for another 60 min, the cell suspension was cooled on ice for 20 min, centrifuged at 5000 x g for 10 min at 4 °C and washed twice with electroporation buffer (0.5 M trehalose, 0.5 M sorbitol, 0.5 M mannitol, 0.5 mM MgCl₂, 0.5 mM K₂HPO₄, 0.5 mM KH₂PO₄, pH 7.4, filter-sterilized and stored frozen). Finally, cells were resuspended in electroporation buffer at 1/100 of the original culture volume and 100 µl of cell suspension was mixed with DNA. The transformation mixture was transferred to a 0.1-cm cuvette and electroporation was performed at 1.8 kV with a single pulse delivered with a MicroPulser™ device (Bio-Rad). Immediately after pulse delivery, 1 ml 2x YT broth containing 0.5 M sorbitol and 0.38 M mannitol was added to the cuvette. The transformation suspension was transferred to a 2 ml tube and incubated at 37 °C for 3 h on a rotary shaker. The cells were spread on a selective 2x YT agar plate and incubated at 37 °C overnight.

Using an alternative electroporation protocol (Xue, G. P., J. S. Johnson, and B. P. Dalrymple: 1999; Journal of Microbiological Methods 34:183-191), 5 ml of LB medium containing 0.5 M glucitol was inoculated with *B. subtilis* and incubated overnight at 37 °C. Subsequently, the overnight culture was diluted (1:16) by 75 ml of LB containing 0.5 M glucitol and incubated until an OD₆₀₀ of 0.85 - 0.95 was obtained. The cells were then pelleted by centrifugation for 10 min at 4 °C at 5.000xg and washed four times by ice-cooled electroporation buffer (10 % glycerol, 0.5 M glucitol, 0.5 M mannitol). Finally, the cells were resuspended in 1-2 ml electroporation buffer. Electroporation was performed using 60 µl of competent cells with DNA in a cooled electroporation cuvette (1-mm electrode gap). The cell-DNA mixture was subjected to a single electrical pulse at 25 µF, 200 Ω and 21 kV/cm. Finally, 1 ml recovery broth (LB containing 0.5 M glucitol and 0.38 mannitol) was added to the electropermeabilized cells and the bacterial culture was incubated for 3 h at 37 °C followed by plating on LB agar supplemented by an antibiotic.

Two different rich media were used, namely Luria Broth (LB) and 2x YT.

Luria Broth (LB) medium consisted of 1% tryptone, 0.5% yeast extract and 0.5% NaCl (pH 7.2).

2x YT medium consisted of 1.6% tryptone, 1% yeast extract and 0.5% NaCl (pH 7.5).

To prepare rich-medium agar plates 15 g L⁻¹ agar were added.

For shaking flask experiments, Spizizen's minimal medium (Spizizen, J. 1958 Proc. Natl. Acad. Sci. U. S. A. 44(10):1072-1078) was used.

Spizizen's minimal medium contains the following salts: 2 g/L (NH₄)₂SO₄, 14 g/L K₂HPO₄, 6 g/L KH₂PO₄, 1 g/L Na₃ citrate × 2·H₂O and 0.2 g/L MgSO₄ × 7·H₂O.

The preculture medium consisted of Spizizen's minimal salts supplemented with 2 % D-glucose, 0.05 % casamino acids and MgSO₄ to a final concentration of 2 mM (optionally additionally supplemented with biotin and/or L-tryptophan).

The main culture medium consisted of Spizizen's minimal salts supplemented with 2 % D-glucose, 0.05 % casamino acids, MgSO₄ to a final concentration of 2 mM and 0.5 mL·L⁻¹ 1000x trace element solution (optionally additionally supplemented with biotin and/or L-tryptophan).

Trace element solution (1000x) consisted of 100.6 g/L C₆H₉NO₆, 56.4 g/L ammonium ferric citrate, 9.8 g/L MnCl₂ × 4·H₂O, 1.6 g/L CoCl₂ × 6·H₂O, 1 g/L CuCl₂ × 2·H₂O, 1.9 g/L H₃BO₃, 9 g/L ZnSO4 × 7·H₂O, 1.1 g/L Na₂MoO₄ × 2·H₂O, 1.5 g/L Na₂SeO₃, 1.5 g/L NiSO₄ × 6·H₂O.

If necessary, the appropriate antibiotic(s) were added to the medium to make it selective.

*B. subtilis* strains were initially grown on rich-media agar plates to obtain single colonies. These plates were grown for 1 day at 30-37°C. For shaking flask experiments, a 20-ml preculture was inoculated with a single colony and grown over night at 30-37 °C on a rotary shaker. Subsequent 20-ml main cultures were inoculated with this preculture to a starting OD₆₀₀ of about 0.1 and incubated at 30-37 °C on a rotary shaker. If induction was required, a 40-60 ml main culture was splitted into 20-ml proportions at the timepoint of induction. The culture volume did not exceed 20 % of the shaking flask capacity.

### Example 2: Construction of a Bacillus subtilis production strain for 3'-sialyllactose

For the synthesis of 3'-sialyllactose from the metabolic intermediate UDP-N-acetylglucosamine using the *neuCBA* pathway, a *B. subtilis* expression plasmid (SEQ ID NO: 1) was constructed. Firstly, the genes *neuA, neuB, neuC* as well as a α-2,3-sialyltransferase were codon-optimized for expression in *B. subtilis* and prepared synthetically by GenScript Corp. The *Campylobacter jejuni* gene *neuA* (UniProtKB accession number: Q93MP7) encodes a CMP-Neu5Ac synthetase. The *Campylobacter jejuni* gene *neuB* (UniProtKB accession number: Q93MP9) encodes a sialic acid synthase. The *Campylobacter jejuni* gene *neuC* (UniProtKB accession number: Q93MP8) encodes a *N*-acetylglucosamine-6-phosphate 2-epimerase. The *Pasteurella multocida* gene *siaT* (UniProtKB accession number: Q9CLP3) encodes an α-2,3-sialyltransferase. The open reading frame of the *E. coli gene lacY* (Gen Bank accession number: NP_414877.1), encoding a lactose permease, was amplified by PCR from chromosomal DNA. Afterwards, the expression cassette <*P_{grac100}-neuBCA-siaT-lacY-terminator>* was constructed, that comprises all necessary genes under the control of the inducible promoter P*_{grac100}.* For this, the *B*. *subtilis* expression vector pHT253 (MoBiTec GmbH, Göttingen, Germany) was used as backbone. Each gene within the expression cassette was linked to a *B. subtilis* RBS sequence. Additionally, a suitable *B. subtilis* terminator sequence from the iGem part repository (sequence ID: BBa_B0015) was placed downstream of the expression cassette. The resulting plasmid <pHT253-P*_{grac100}-neuBCA*-2,3*siaT-lacY-terminator>* (SEQ ID NO: 1) was used to transform a sporulating *B. subtilis* strain (table 1) by exploiting its natural competence.

Gene expression was confirmed by targeted proteomics and/or by real-time PCR.

Transformants were cultivated under conditions that are permissive for *B. subtilis* to produce 3'-sialyllactose in the presence of exogenous lactose.

### Example 3: Construction of a Bacillus subtilis production strain for 3'-sialyllactose

For the synthesis of 3'-sialyllactose from the metabolic intermediate UDP-*N-*acetylglucosamine using the *neuCBA* pathway, a constitutive B. *subtilis* expression plasmid (SEQ ID NO: 2) was constructed. Firstly, the genes *neuA, neuB, neuC* and *siaT* were codon-optimized for expression in *B. subtilis* and prepared synthetically by GenScript Corp. The *Campylobacter jejuni* gene *neuA* (UniProtKB accession number: Q93MP7) encodes a CMP-Neu5Ac synthetase. The *Campylobacter jejuni* gene *neuB* (UniProtKB accession number: Q93MP9) encodes a sialic acid synthase.

The *Campylobacter jejuni* gene *neuC* (UniProtKB accession number: Q93MP8) encodes a *N*-acetylglucosamine-6-phosphate 2-epimerase. The *Pasteurella multocida* gene *siaT* (UniProtKB accession number: Q9CLP3) encodes an α-2,3-sialyltransferase. The open reading frame of the *E. coli gene lacY (Gen* Bank accession number: NP_414877.1), encoding a lactose permease, was amplified by PCR from chromosomal DNA. Afterwards, the expression cassette *<P43-neuBCA-P_{lepA}-siaT-lacY-terminator>* was constructed, that comprises all necessary genes operably linked to the two strong constitutive *B. subtilis* promoters P*_{lepA}* and/or P43. The *B. subtilis* expression vector pHT253 (MoBiTec GmbH, Göttingen, Germany) was used as plasmid backbone. Each gene within the expression cassette was linked to a *B. subtilis* RBS sequence. Additionally, a suitable *B. subtilis* terminator sequence from the iGem part repository (sequence ID: BBa_B0015) was placed downstream of the expression cassette.

The resulting plasmid <pHT253-P43-*neuBCA-P_{lepA}-siaT-*/*acY-terminator>* (SEQ ID NO: 2) was used to transform spore-forming and non-sporulating B. *subtilis* strains (Table 1) by exploiting their natural competence.

Gene expression was confirmed by targeted proteomics and/or by real-time PCR.

Transformants were cultivated under conditions that are permissive for *B. subtilis* to produce 3'-sialyllactose in the presence of exogenous lactose.

### Example 4: Construction of a Bacillus subtilis production strain for 6'-sialyllactose

For production of 6'-sialyllactose in *B. subtilis* the α-2,6-sialyltransferase from *Photobacterium leiognathi* was used. The open reading frame of the gene *siaT* (UniProtKB accession number: D0VYB7) was codon-optimized for expression in *B. subtilis* and prepared synthetically by GenScript Corp.

The expression plasmid <pHT253-P*_{grac100}-neuBCA*-2,6*siaT-lacY-terminator>* (SEQ ID NO: 3) was constructed as described in example 2. The resulting plasmid (SEQ ID NO: 3) was used to transform spore-forming and non-sporulating *B. subtilis* strains (Table 1) by exploiting their natural competence. Gene expression was confirmed by targeted proteomics and/or by real-time PCR. Transformants were cultivated under conditions that are permissive for *B. subtilis* to produce 6'-sialyllactose in the presence of exogenous lactose.

### Example 5: Construction of a Bacillus subtilis strain for production of 3'-sialyllactose

Metabolic engineering of *B. subtilis* (Table 1) was achieved by integration of the heterologous genes *Campylobacter jejuni neuA, E. coli nanT* and *Haemophilus parahaemolyticus siaT* and simultaneous deletion of the endogenous gene *ganA* by homologous recombination. The *B. subtilis* gene *ganA* (*yvfN, lacA)*, which is positioned within the galactan operon, encodes a beta-galactosidase.

For the production of 3'-sialyllactose from exogenous sialic acid/N-acetylneuraminic acid and lactose, the open reading frames of *neuA, nanT* and *siaT* were operably linked to the *B. subtilis* constitutive promoter P43 (iGem part repository: sequence ID: BBa_K143013) as an operon. For this purpose, firstly, the genes *neuA, nanT* and *siaT* were codon-optimized for expression in *B. subtilis* and prepared syntheticcally by GenScript Corp. The *Campylobacter jejuni* gene *neuA* (UniProtKB accession number: Q93MP7) encodes a CMP-Neu5Ac synthetase, the *E. coli* gene *nanT* (GenBank accession number: NP_417691.4) encodes a sialic acid transporter and the *H. parahaemolyticus* gene *siaT* (UniProtKB accession number: I3DHL4) encodes an alpha-*N*-acetylneuraminyl-2,3-beta-galactosyl-1,3-*N*-acetylgalactosaminide 6-alpha-sialyltransferase (alpha-2,3-sialyltransferase).

Each gene within the expression cassette was linked to a *B. subtilis* RBS sequence. Additionally, a suitable *B. subtilis* terminator sequence from the iGem part repository (sequence ID: BBa_B0015) was placed downstream of the expression cassette.

The cloning vector pBR322 (New England Biolabs GmbH, Frankfurt, Germany) was used as plasmid backbone. The complete integration cassette was assembled to create the suicide plasmid <pBR322 flank *ganA* up-P43*-siaT-neuA-nanT-terminator*erm-flank *ganA* down> (SEQ ID NO: 4). Afterwards, *B. subtilis* was transformed with this plasmid by its natural competence. Cells were spread on 2x YT agar plates containing the appropriate antibiotic (5 µg mL⁻¹ erythromycin). Integration of the expression cassette <P43*-siaT-neuA-nanT-terminator>* into the *ganA* locus of the *B. subtilis* genome, yielding strain A, was verified by colony PCR. Gene expression was confirmed by targeted proteomics and/or by real-time PCR.

For import of exogenous lactose into the *Bacillus* cell, the *E*. *coli* gene *lacY* was integrated into the endogenous *amyE* (*amyA*) locus of the *B. subtilis* genome (encoding for an alpha-amylase). For this purpose, the open reading frame of the *E. coli lacY* gene (Gen Bank accession number: NP_414877.1), encoding a lactose permease, was amplified by PCR from chromosomal DNA. The integration cassette <flank *amyE* up*-lox71-aad9-lox66*-P43*-lacY*-flank *amyE* down> (SEQ ID NO: 5) was constructed and cloned into pBR322 (New England Biolabs GmbH, Frankfurt, Germany). Strain A was transformed with the resulting suicide plasmid by natural competence. Integration of the expression cassette <P43-*lacY*> into the *amyE* locus of strain A, yielding the final strain B, was verified by colony PCR.

Gene expression was confirmed by targeted proteomics and/or by real-time PCR. Strain B was cultivated under conditions that are permissive for *B. subtilis* to produce 3'-sialyllactose in the presence of exogenous lactose and sialic acid/ *N*-acetylneuraminic acid.

### Example 6: Production of sialyllactose using metabolically engineered Bacillus subtilis strains

A preculture was inoculated with a metabolically engineered *Bacillus subtilis* strain suitable for the biosynthesis of sialyllactose (as described in examples 2-5).

The preculture was incubated at 30-37 °C over night and then diluted to a starting OD₆₀₀ of about 0.1 in fresh main culture medium. When the main culture reached an OD₆₀₀ of approximately 0.5, 2 mM lactose was added to the growth medium. When the inducible promoter P*_{grac100}* was used for gene expression, induction was carried out with lactose (2 mM) or with both lactose (2 mM) and IPTG (1 mM). For the salvage biosynthesis of CMP-*N*-acetylneuraminic acid, additionally, 2 mM sialic acid/N-acetylneuraminic acid were added. Cultivation was discontinued after about 24 h / 48 h after induction and intracellular and extracellular sialyllactose was analyzed by thin layer chromatography and/or HPLC and/or mass spectrometry (as described in WO 2017/042382 A or WO 2019/008133 A). Biosynthesis of substantial amounts of sialyllactose (3'-sialyllactose/6'-sialyllactose) was detectable.

## Claims

1. A non-sporulating *Bacillus* cell for the production of a sialylated oligosaccharide, wherein said *Bacillus* cell has been genetically engineered to possess a lactose permease, a CMP-NeuNAc biosynthesis pathway and a sialyltransferase.

2. The *Bacillus* cell according to claim 1, wherein the sporulation capability of the *Bacillus* cell has been impaired by deletion or functional inactivation of one or more of the genes encoding SpoOA, sigma E and sigma F.

3. The *Bacillus* cell according to claim 1 or 2, wherein the lactose permease is *E*. *coli* LacY or a functional variant thereof.

4. The *Bacillus* cell according to any one of claims 1 to 3, wherein the CMP-NeuNAc biosynthesis pathway uses GlcN-1P or GlcNAc-6P as intermediate.

5. The *Bacillus* cell according to any one of claims 1 to 4, wherein the CMP-NeuNAc biosynthesis pathway uses a sialic acid salvage pathway.

6. The *Bacillus* cell according to any one of claims 1 to 5, wherein the sialyltransferase is a lactose-accepting sialyltransferase, preferably a sialyltransferase selected from the group consisting of α-2,3-sialyl-transferases, α-2,6-sialyltransferases and α-2,8-sialyltransferases.

7. The *Bacillus* cell according to any one of claims 1 to 6, wherein said *Bacillus* cell lacks any β-galactosidase activity or possesses reduced β-galactosidase activity as compared to a wild-type progenitor *Bacillus* cell of the same species.

8. The *Bacillus* cell according to claim 7, wherein the *Bacillus* cell has been genetically engineered by deletion or functional inactivation of at least one of the genes selected from the group consisting of *yes*Z and *gan*A*.*

9. The *Bacillus* cell according to any one of claims 1 to 8, wherein said *Bacillus* cell is a *Bacillus subtilis* cell.

10. The *Bacillus* cell according to any one of claims 1 to 9, for use in the production of 3'-sialylactose, wherein the CMP-NeuNAc biosynthesis pathway uses GlcN-1P as intermediate, and wherein the sialyltransferase is a α-2,3-sialyltransferase.

11. The *Bacillus* cell according to any one of claims 1 to 9, for use in the production of 6'-sialylactose, wherein the CMP-NeuNAc biosynthesis pathway uses GlcN-1P as intermediate, and wherein the sialyltransferase is a α-2,6-sialyltransferase.

12. Use of a *Bacillus* cell according to any one of claims 1 to 11 for the production of a sialylated oligosaccharide.

13. A method for the production of a sialylated oligosaccharide, the method comprising
- providing a non-sporulating *Bacillus* cell as defined in any one of claims 1 to 11;
- cultivating said *Bacillus* cell in a fermentation broth and under conditions that are permissive for the production of a sialylated oligosaccharide; and optionally
- retrieving the sialylated oligosaccharide from the medium and/or the *Bacillus* cell.

14. The method according to claim 13, wherein the fermentation broth contains lactose.

15. A sialylated oligosaccharide produced by a method according to claim 13 or 14.

16. Use of a sialylated oligosaccharide according to claim 15 for the manufacture of a nutritional composition, preferably of an infant formula.

17. A nutritional composition containing a sialylated oligosaccharide according to claim 15.


SEQUENCE LISTING

<110>  Jennewein Biotechnologie GmbH

<120>  Production of sialylated oligosaccharides in Bacillus cells

<130>  P 1906 EP

<160>  5

<170>  PatentIn version 3.5

<210>  1
<211>  13121
<212>  DNA
<213>  Artificial Sequence

<220>
<223>  Expression plasmid

<400>  1
ttaagttatt ggtatgactg gttttaagcg caaaaaaagt tgctttttcg tacctattaa      60

tgtatcgttt tagaaaaccg actgtaaaaa gtacagtcgg cattatctca tattataaaa     120

gccagtcatt aggcctatct gacaattcct gaatagagtt cataaacaat cctgcatgat     180

aaccatcaca aacagaatga tgtacctgta aagatagcgg taaatatatt gaattacctt     240

tattaatgaa ttttcctgct gtaataatgg gtagaaggta attactatta ttattgatat     300

ttaagttaaa cccagtaaat gaagtccatg gaataataga aagagaaaaa gcattttcag     360

gtataggtgt tttgggaaac aatttccccg aaccattata tttctctaca tcagaaaggt     420

ataaatcata aaactctttg aagtcattct ttacaggagt ccaaatacca gagaatgttt     480

tagatacacc atcaaaaatt gtataaagtg gctctaactt atcccaataa cctaactctc     540

cgtcgctatt gtaaccagtt ctaaaagctg tatttgagtt tatcaccctt gtcactaaga     600

aaataaatgc agggtaaaat ttatatcctt cttgttttat gtttcggtat aaaacactaa     660

tatcaatttc tgtggttata ctaaaagtcg tttgttggtt caaataatga ttaaatatct     720

cttttctctt ccaattgtct aaatcaattt tattaaagtt catttgatat gcctcctaaa     780

tttttatcta aagtgaattt aggaggctta cttgtctgct ttcttcatta gaatcaatcc     840

ttttttaaaa gtcaatatta ctgtaacata aatatatatt ttaaaaatat cccactttat     900

ccaattttcg tttgttgaac taatgggtgc tttagttgaa gaataaagac cacattaaaa     960

aatgtggtct tttgtgtttt tttaaaggat ttgagcgtag cgaaaaatcc ttttctttct    1020

tatcttgata ataagggtaa ctattgccga tcgtccattc cgacagcatc gccagtcact    1080

atggcgtgct gctagcgcca ttcgccattc aggctgcgca actgttggga agggcgatcg    1140

gtgcgggcct cttcgctatt acgccagctg gcgaaagggg gatgtgctgc aaggcgatta    1200

agttgggtaa cgccagggtt ttcccagtca cgacgttgta aaacgacggc cagtgaattc    1260

gagctcaggc cttaactcac attaattgcg ttgcgctcac tgcccgcttt ccagtcggga 1320

aacctgtcgt gccagctgca ttaatgaatc ggccaacgcg cggggagagg cggtttgcgt 1380

attgggcgcc agggtggttt ttcttttcac cagtgagacg ggcaacagct gattgccctt 1440

caccgcctgg ccctgagaga gttgcagcaa gcggtccacg ctggtttgcc ccagcaggcg 1500

aaaatcctgt ttgatggtgg ttgacggcgg gatataacat gagctgtctt cggtatcgtc 1560

gtatcccact accgagatat ccgcaccaac gcgcagcccg gactcggtaa tggcgcgcat 1620

tgcgcccagc gccatctgat cgttggcaac cagcatcgca gtgggaacga tgccctcatt 1680

cagcatttgc atggtttgtt gaaaaccgga catggcactc cagtcgcctt cccgttccgc 1740

tatcggctga atttgattgc gagtgagata tttatgccag ccagccagac gcagacgcgc 1800

cgagacagaa cttaatgggc ccgctaacag cgcgatttgc tggtgaccca atgcgaccag 1860

atgctccacg cccagtcgcg taccgtcttc atgggagaaa ataatactgt tgatgggtgt 1920

ctggtcagag acatcaagaa ataacgccgg aacattagtg caggcagctt ccacagcaat 1980

ggcatcctgg tcatccagcg gatagttaat gatcagccca ctgacgcgtt gcgcgagaag 2040

attgtgcacc gccgctttac aggcttcgac gccgcttcgt tctaccatcg acaccaccac 2100

gctggcaccc agttgatcgg cgcgagattt aatcgccgcg acaatttgcg acggcgcgtg 2160

cagggccaga ctggaggtgg caacgccaat cagcaacgac tgtttgcccg ccagttgttg 2220

tgccacgcgg ttgggaatgt aattcagctc cgccatcgcc gcttccactt tttcccgcgt 2280

tttcgcagaa acgtggctgg cctggttcac cacgcgggaa acggtctgat aagagacacc 2340

ggcatactct gcgacatcgt ataacgttac tggtttcatc aaaatcgtct ccctccgttt 2400

gaatatttga ttgatcgtaa ccagatgaag cactctttcc actatcccta cagtgttatg 2460

gcttgaacaa tcacgaaaca ataattggta cgtacgatct ttcagccgac tcaaacatca 2520

aatcttacaa atgtagtctt tgaaagtatt acatatgtaa gatttaaatg caaccgtttt 2580

ttcggaagga aatgatgacc tcgtttccac cggaattagc ttggtaccaa aggaggtaag 2640

gatcactaga aaatttttta aaaaatctct tgacattgga agggagatat gttattataa 2700

gaattgcgga attgtgagcg gataacaatt cccatataga ttaactaata aggaggacaa 2760

acatgaaaga aatcaaaatc caaaacatca tcatcagcga agaaaaagcg ccgctggttg 2820

tgccggaaat cggcattaat cataacggat ctttagaact ggctaaaatc atggttgatg 2880

cagcgttttc agctggagcc aaaatcatca aacatcaaac acatatcgtc gaagatgaaa 2940

tgagcaaagc tgcaaagaaa gttatcccgg gcaacgctaa aatctctatc tacgaaatca 3000

tgcagaaatg cgctttagat tacaaagatg aacttgccct gaaagaatac acagaaaaac 3060

tgggacttgt gtatcttagc acaccgtttt caagagcagg cgcgaatcgc ttagaagata 3120

tgggagtctc tgcatttaaa atcggctcag gagaatgtaa taactatccg ctgatcaaac 3180

atattgcagc gtttaaaaaa ccgatgattg tgtctacagg catgaactca atcgaaagca 3240

ttaaaccgac agtgaaaatc ctgcttgata acgaaatccc gtttgtcctg atgcatacaa 3300

caaacctgta tccgacaccg cataatcttg ttagattaaa cgccatgctg gaactgaaga 3360

aagaatttag ctgcatggtg ggactttctg atcatacaac agataatctg gcatgccttg 3420

gcgcggtcgt tcttggagcg tgtgtcttag aaagacattt tacagattca atgcatcgca 3480

gcggaccgga tattgtttgt tctatggata caaaagcctt aaaagaactg atcattcaat 3540

cagaacagat ggcaatcatt cgcggcaata acgaatcaaa gaaagcagcc aaacaagaac 3600

aggttacaat cgattttgct tttgcctctg tggtctcaat caaagatatt aagaaaggcg 3660

aagttctgtc aatggataat atttgggtga aaagaccggg acttggcgga atcagcgcag 3720

cggaatttga aaatattctg ggcaagaaag cactgcgcga tattgaaaac gatgcgcagt 3780

taagctatga agattttgct taagctctta aggaggattt tagaatgaag aaaattctgt 3840

ttatcacagg ctcaagagcg gattactcta aaatcaaatc acttatgtac cgcgtccaaa 3900

attcaagcga atttgaactt tacatctttg ctacaggcat gcatctgagc aaaaactttg 3960

gatacacagt taaagaatta tataaaaatg gctttaaaaa catctacgaa tttatcaact 4020

acgataaata ttatcagaca gataaagccc tggcaacaac aattgatgga ttttcaagat 4080

atgcgaacga attaaaaccg gatctgattg ttgtgcatgg cgatcgcatc gaaccgcttg 4140

cagcggctat tgtcggagcc cttaacaaca tcctggttgc acatatcgaa ggcggagaaa 4200

ttagcggaac aatcgatgat tctttaagac atgcgatttc aaaacttgct catatccatc 4260

tggtgaacga tgaatttgca aaaagacgcc ttatgcaatt aggcgaagat gaaaaatcaa 4320

tctttatcat cggatctccg gatctggaac tgcttaacga taacaaaatc tcacttagcg 4380

aagccaaaaa atactacgat attaactacg aaaactatgc attactgatg tttcatccgg 4440

tcacaacaga aatcacatct atcaaaaacc aagccgataa cctggtgaaa gcacttatcc 4500

agtcaaacaa aaactacatc gtcatctatc cgaataacga tctgggcttt gaactgatcc 4560

ttcagagcta tgaagaattt aaaaacaacc cgagatttaa actgtttccg tctctgcgct 4620

ttgaatactt tatcacactt ctgaaaaacg ccgattttat tatcggaaac tcttcatgca 4680

tcctgaaaga agcgttatac ctgaaaacag ctggcattct ggttggatca agacaaaatg 4740

gccgcttagg aaatgaaaac acactgaaag tgaatgcgaa cagcgatgaa atccttaaag 4800

ctatcaacac aatccataaa aaacaggatt tattttctgc taaactggaa attcttgata 4860

gctctaaact gtttttcgaa tatcttcaat caggcgattt ctttaaactt agcacacaga 4920

aagtttttaa agatattaaa taaaaaggag gaactactat gtcacttgca attatcccgg 4980

cgagaggcgg aagcaaaggc atcaaaaaca aaaacctggt tctgcttaac aacaaaccgc 5040

ttatctacta cacaatcaaa gcagcgctga atgctaaatc tatttcaaaa gttgtggtct 5100

caagcgatag cgatgaaatc cttaactacg ccaaatctca aaacgtggat attctgaaaa 5160

gaccgatctc tcttgcacag gatgatacaa catcagataa agtcttactg catgcgctga 5220

aattttacaa agattacgaa gatgttgtgt ttttacaacc gacatctccg ctgcgcacaa 5280

acattcatat caacgaagca tttaacctgt acaaaaattc aaacgctaat gccctgatta 5340

gcgtctctga atgcgataac aaaatcctta aagcatttgt ttgcaacgat tgtggcgatt 5400

tagccggaat ttgtaatgat gaatatccgt ttatgccgcg ccagaaactg ccgaaaacat 5460

atatgagcaa cggagcgatc tacatcctta aaatcaaaga atttctgaac aacccgtcat 5520

ttctgcaaag caaaacaaaa catttcttta tggatgaatc atcatcactg gatattgatt 5580

gcctggaaga tttaaagaaa gttgaacaaa tttggaaaaa ataaagatta actaataagg 5640

aggacaaaca tggataaatt tgctgaacat gaaattccga aagctgttat cgtggccggc 5700

aacggagaat ctctttcaca aatcgattac agactgcttc cgaaaaacta tgatgtcttt 5760

agatgcaacc agttttactt tgaagaacgc tattttcttg gcaacaaaat caaagcagtt 5820

ttctttacac cgggagtgtt tttagaacaa tactacacac tgtaccatct taaacgcaac 5880

aacgaatact ttgtcgataa cgttatcctg tcatcattta accatccgac agttgatctg 5940

gaaaaatcac aaaaaatcca ggctctgttt attgatgtga tcaacggcta cgaaaaatac 6000

ctgtctaaac tgacagcctt tgatgtttat ctgagataca aagaacttta cgaaaaccaa 6060

cgcattacat caggagtgta tatgtgcgcc gtcgcaatcg cgatgggcta tacagatatt 6120

taccttacag gaatcgattt ttatcaggca agcgaagaaa actacgcgtt tgataacaaa 6180

aaaccgaaca tcatcagatt actgccggat tttcgcaaag aaaaaacatt attttcatat 6240

catagcaaag atattgatct tgaagcatta tcttttctgc aacagcatta ccatgttaac 6300

ttttacagca tttctccgat gtctccgctg tcaaaacatt ttccgatccc gacagtggaa 6360

gatgattgtg aaacaacatt tgtcgcgccg ctgaaagaaa actacatcaa cgatattctt 6420

ttaccgccgc attttgtcta tgaaaaactt ggcgttgata aacttgcagc ggctttagaa 6480

catcatcatc atcatcatta aagtgatagc ggtaccatta taggtaagag aggaatgtac 6540

acatgtacta tttaaaaaac acaaactttt ggatgttcgg tttattcttt ttcttttact 6600

tttttatcat gggagcctac ttcccgtttt tcccgatttg gctacatgac atcaaccata 6660

tcagcaaaag tgatacgggt attatttttg ccgctatttc tctgttctcg ctattattcc 6720

aaccgctgtt tggtctgctt tctgacaaac tcgggctgcg caaatacctg ctgtggatta 6780

ttaccggcat gttagtgatg tttgcgccgt tctttatttt tatcttcggg ccactgttac 6840

aatacaacat tttagtagga tcgattgttg gtggtattta tctaggcttt tgttttaacg 6900

ccggtgcgcc agcagtagag gcatttattg agaaagtcag ccgtcgcagt aatttcgaat 6960

ttggtcgcgc gcggatgttt ggctgtgttg gctgggcgct gtgtgcctcg attgtcggca 7020

tcatgttcac catcaataat cagtttgttt tctggctggg ctctggctgt gcactcatcc 7080

tcgccgtttt actctttttc gccaaaacgg atgcgccctc ttctgccacg gttgccaatg 7140

cggtaggtgc caaccattcg gcatttagcc ttaagctggc actggaactg ttcagacagc 7200

caaaactgtg gtttttgtca ctgtatgtta ttggcgtttc ctgcacctac gatgtttttg 7260

accaacagtt tgctaatttc tttacttcgt tctttgctac cggtgaacag ggtacgcggg 7320

tatttggcta cgtaacgaca atgggcgaat tacttaacgc ctcgattatg ttctttgcgc 7380

cactgatcat taatcgcatc ggtgggaaaa acgccctgct gctggctggc actattatgt 7440

ctgtacgtat tattggctca tcgttcgcca cctcagcgct ggaagtggtt attctgaaaa 7500

cgctgcatat gtttgaagta ccgttcctgc tggtgggctg ctttaaatat attaccagcc 7560

agtttgaagt gcgtttttca gcgacgattt atctggtctg tttctgcttc tttaagcaac 7620

tggcgatgat ttttatgtct gtactggcgg gcaatatgta tgaaagcatc ggtttccagg 7680

gcgcttatct ggtgctgggt ctggtggcgc tgggcttcac cttaatttcc gtgttcacgc 7740

ttagcggccc cggcccgctt tccctgctgc gtcgtcaggt gaatgaagtc gcttaaggat 7800

ccatgtctag agtcgacgtc cccggggcag cccgcctaat gagcgggctt ttttcacgtc 7860

ccaggcatca aataaaacga aaggctcagt cgaaagactg ggcctttcgt tttatctgtt 7920

gtttgtcggt gaacgctctc tactagagtc acactggctc accttcgggt gggcctttct 7980

gcgtttatac ccggggcagc ccgcctaatg agcgggcttt tttcacgtca cgcgtccatg 8040

gagatctttg tctgcaactg aaaagtttat accttacctg gaacaaatgg ttgaaacata 8100

cgaggctaat atcggcttat taggaatagt ccctgtacta ataaaatcag gtggatcagt 8160

tgatcagtat attttggacg aagctcggaa agaatttgga gatgacttgc ttaattccac 8220

aattaaatta agggaaagaa taaagcgatt tgatgttcaa ggaatcacgg aagaagatac 8280

tcatgataaa gaagctctaa aactattcaa taaccttaca atggaattga tcgaaagggt 8340

ggaaggttaa tggtacgaaa attaggggat ctacctagaa agccacaagg cgataggtca 8400

agcttaaaga acccttacat ggatcttaca gattctgaaa gtaaagaaac aacagaggtt 8460

aaacaaacag aaccaaaaag aaaaaaagca ttgttgaaaa caatgaaagt tgatgtttca 8520

atccataata agattaaatc gctgcacgaa attctggcag catccgaagg gaattcatat 8580

tacttagagg atactattga gagagctatt gataagatgg ttgagacatt acctgagagc 8640

caaaaaactt tttatgaata tgaattaaaa aaaagaacca acaaaggctg agacagactc 8700

caaacgagtc tgttttttta aaaaaaatat taggagcatt gaatatatat tagagaatta 8760

agaaagacat gggaataaaa atattttaaa tccagtaaaa atatgataag attatttcag 8820

aatatgaaga actctgtttg tttttgatga aaaaacaaac aaaaaaaatc cacctaacgg 8880

aatctcaatt taactaacag cggccaaact gagaagttaa atttgagaag gggaaaaggc 8940

ggatttatac ttgtatttaa ctatctccat tttaacattt tattaaaccc catacaagtg 9000

aaaatcctct tttacactgt tcctttaggt gatcgcggag ggacattatg agtgaagtaa 9060

acctaaaagg aaatacagat gaattagtgt attatcgaca gcaaaccact ggaaataaaa 9120

tcgccaggaa gagaatcaaa aaagggaaag aagaagttta ttatgttgct gaaacggaag 9180

agaagatatg gacagaagag caaataaaaa actttctttt agacaaattt ggtacgcata 9240

taccttacat agaaggtcat tatacaatct taaataatta cttctttgat tttgggggct 9300

attttttagg tgctgaagga attgcgctct atgctcacct aactcgttat gcatacggca 9360

gcaaagactt ttgctttcct agtctacaaa caatcgctaa aaaaatggac aagactcctg 9420

ttacagttag aggctacttg aaactgcttg aaaggtacgg ttttatttgg aaggtaaacg 9480

tccgtaataa aaccaaggat aacacagagg aatccccgat ttttaagatt agacgtaagg 9540

ttcctttgct ttcagaagaa cttttaaatg gaaaccctaa tattgaaatt ccagatgacg 9600

aggaagcaca tgtaaagaag gctttaaaaa aggaaaaaga gggtcttcca aaggttttga 9660

aaaaagagca cgatgaattt gttaaaaaaa tgatggatga gtcagaaaca attaatattc 9720

cagaggcctt acaatatgac acaatgtatg aagatatact cagtaaagga gaaattcgaa 9780

aagaaatcaa aaaacaaata cctaatccta caacatcttt tgagagtata tcaatgacaa 9840

ctgaagagga aaaagtcgac agtactttaa aaagcgaaat gcaaaatcgt gtctctaagc 9900

cttcttttga tacctggttt aaaaacacta agatcaaaat tgaaaataaa aattgtttat 9960

tacttgtacc gagtgaattt gcatttgaat ggattaagaa aagatattta gaaacaatta 10020

aaacagtcct tgaagaagct ggatatgttt tcgaaaaaat cgaactaaga aaagtgcaat 10080

aaactgctga agtatttcag cagttttttt tatttagaaa tagtgaaaaa aatataatca 10140

gggaggtatc aatatttaat gagtactgat ttaaatttat ttagactgga attaataatt 10200

aacacgtaga ctaattaaaa tttaatgagg gataaagagg atacaaaaat attaatttca 10260

atccctatta aattttaaca agggggggat taaaatttaa ttagaggttt atccacaaga 10320

aaagacccta ataaaatttt tactagggtt ataacactga ttaatttctt aatgggggag 10380

ggattaaaat ttaatgacaa agaaaacaat cttttaagaa aagcttttaa aagataataa 10440

taaaaagagc tttgcgatta agcaaaactc tttacttttt cattgacatt atcaaattca 10500

tcgatttcaa attgttgttg tatcataaag ttaattctgt tttgcacaac cttttcagga 10560

atataaaaca catctgaggc ttgttttata aactcagggt cgctaaagtc aatgtaacgt 10620

agcatatgat atggtatagc ttccacccaa gttagccttt ctgcttcttc tgaatgtttt 10680

tcatatactt ccatgggtat ctctaaatga ttttcctcat gtagcaaggt atgagcaaaa 10740

agtttatgga attgatagtt cctctctttt tcttcaactt ttttatctaa aacaaacact 10800

ttaacatctg agtcaatgta agcataagat gtttttccag tcataatttc aatcccaaat 10860

cttttagaca gaaattctgg acgtaaatct tttggtgaaa gaattttttt atgtagcaat 10920

atatccgata cagcaccttc taaaagcgtt ggtgaatagg gcattttacc tatctcctct 10980

cattttgtgg aataaaaata gtcatattcg tccatctacc tatcctatta tcgaacagtt 11040

gaacttttta atcaaggatc agtccttttt ttcattattc ttaaactgtg ctcttaactt 11100

taacaactcg atttgttttt ccagatctcg agggtaacta gcctcgccga tcccgcaaga 11160

ggcccggcag tcaggtggca cttttcgggg aaatgtgcgc ggaaccccta tttgtttatt 11220

tttctaaata cattcaaata tgtatccgct catgagacaa taaccctgat aaatgcttca 11280

ataatattga aaaaggaaga gtatgagtat tcaacatttc cgtgtcgccc ttattccctt 11340

ttttgcggca ttttgccttc ctgtttttgc tcacccagaa acgctggtga aagtaaaaga 11400

tgctgaagat cagttgggtg cacgagtggg ttacatcgaa ctggatctca acagcggtaa 11460

gatccttgag agttttcgcc ccgaagaacg ttttccaatg atgagcactt ttaaagttct 11520

gctatgtggc gcggtattat cccgtattga cgccgggcaa gagcaactcg gtcgccgcat 11580

acactattct cagaatgact tggttgagta ctcaccagtc acagaaaagc atcttacgga 11640

tggcatgaca gtaagagaat tatgcagtgc tgccataacc atgagtgata acactgcggc 11700

caacttactt ctgacaacga tcggaggacc gaaggagcta accgcttttt tgcacaacat 11760

gggggatcat gtaactcgcc ttgatcgttg ggaaccggag ctgaatgaag ccataccaaa 11820

cgacgagcgt gacaccacga tgcctgtagc aatggcaaca acgttgcgca aactattaac 11880

tggcgaacta cttactctag cttcccggca acaattaata gactggatgg aggcggataa 11940

agttgcagga ccacttctgc gctcggccct tccggctggc tggtttattg ctgataaatc 12000

tggagccggt gagcgtgggt ctcgcggtat cattgcagca ctggggccag atggtaagcc 12060

ctcccgtatc gtagttatct acacgacggg gagtcaggca actatggatg aacgaaatag 12120

acagatcgct gagataggtg cctcactgat taagcattgg taactgtcag accaagttta 12180

ctcatatata ctttagattg atttaaaact tcatttttaa tttaaaagga tctaggtgaa 12240

gatccttttt gataatctca tgaccaaaat cccttaacgt gagttttcgt tccactgagc 12300

gtcagacccc gtagaaaaga tcaaaggatc ttcttgagat cctttttttc tgcgcgtaat 12360

ctgctgcttg caaacaaaaa aaccaccgct accagcggtg gtttgtttgc cggatcaaga 12420

gctaccaact ctttttccga aggtaactgg cttcagcaga gcgcagatac caaatactgt 12480

ccttctagtg tagccgtagt taggccacca cttcaagaac tctgtagcac cgcctacata 12540

cctcgctctg ctaatcctgt taccagtggc tgctgccagt ggcgataagt cgtgtcttac 12600

cgggttggac tcaagacgat agttaccgga taaggcgcag cggtcgggct gaacgggggg 12660

ttcgtgcaca cagcccagct tggagcgaac gacctacacc gaactgagat acctacagcg 12720

tgagctatga gaaagcgcca cgcttcccga agggagaaag gcggacaggt atccggtaag 12780

cggcagggtc ggaacaggag agcgcacgag ggagcttcca gggggaaacg cctggtatct 12840

ttatagtcct gtcgggtttc gccacctctg acttgagcgt cgatttttgt gatgctcgtc 12900

aggggggcgg agcctatgga aaaacgccag caacgcggcc tttttacggt tcctggcctt 12960

ttgctggcct tttgctcaca tgttctttcc tgcgttatcc cctgattctg tggataaccg 13020

tattaccgcc tttgagtgag ctgataccgc tcgccgcagc cgaacgaccg agcgcagcga 13080

gtcagtgagc gaggaagcgg aagagcgccc aatacgcatg c 13121

<210> 2
<211> 11125
<212> DNA
<213> Artificial Sequence

<220>
<223> Plasmid

<400> 2
ttaagttatt ggtatgactg gttttaagcg caaaaaaagt tgctttttcg tacctattaa 60

tgtatcgttt tagaaaaccg actgtaaaaa gtacagtcgg cattatctca tattataaaa 120

gccagtcatt aggcctatct gacaattcct gaatagagtt cataaacaat cctgcatgat 180

aaccatcaca aacagaatga tgtacctgta aagatagcgg taaatatatt gaattacctt 240

tattaatgaa ttttcctgct gtaataatgg gtagaaggta attactatta ttattgatat 300

ttaagttaaa cccagtaaat gaagtccatg gaataataga aagagaaaaa gcattttcag 360

gtataggtgt tttgggaaac aatttccccg aaccattata tttctctaca tcagaaaggt 420

ataaatcata aaactctttg aagtcattct ttacaggagt ccaaatacca gagaatgttt 480

tagatacacc atcaaaaatt gtataaagtg gctctaactt atcccaataa cctaactctc 540

cgtcgctatt gtaaccagtt ctaaaagctg tatttgagtt tatcaccctt gtcactaaga 600

aaataaatgc agggtaaaat ttatatcctt cttgttttat gtttcggtat aaaacactaa 660

tatcaatttc tgtggttata ctaaaagtcg tttgttggtt caaataatga ttaaatatct 720

cttttctctt ccaattgtct aaatcaattt tattaaagtt catttgatat gcctcctaaa 780

tttttatcta aagtgaattt aggaggctta cttgtctgct ttcttcatta gaatcaatcc 840

ttttttaaaa gtcaatatta ctgtaacata aatatatatt ttaaaaatat cccactttat 900

ccaattttcg tttgttgaac taatgggtgc tttagttgaa gaataaagac cacattaaaa 960

aatgtggtct tttgtgtttt tttaaaggat ttgagcgtag cgaaaaatcc ttttctttct 1020

tatcttgata ataagggtaa ctattgccga tcgtccattc cgacagcatc gccagtcact 1080

atggcgtgct gctagcattt tacattttta gaaatgggcg tgaaaaaaag cgcgcgatta 1140

tgtaaaatat aaagattaac taataaggag gacaaacatg aaagaaatca aaatccaaaa 1200

catcatcatc agcgaagaaa aagcgccgct ggttgtgccg gaaatcggca ttaatcataa 1260

cggatcttta gaactggcta aaatcatggt tgatgcagcg ttttcagctg gagccaaaat 1320

catcaaacat caaacacata tcgtcgaaga tgaaatgagc aaagctgcaa agaaagttat 1380

cccgggcaac gctaaaatct ctatctacga aatcatgcag aaatgcgctt tagattacaa 1440

agatgaactt gccctgaaag aatacacaga aaaactggga cttgtgtatc ttagcacacc 1500

gttttcaaga gcaggcgcga atcgcttaga agatatggga gtctctgcat ttaaaatcgg 1560

ctcaggagaa tgtaataact atccgctgat caaacatatt gcagcgttta aaaaaccgat 1620

gattgtgtct acaggcatga actcaatcga aagcattaaa ccgacagtga aaatcctgct 1680

tgataacgaa atcccgtttg tcctgatgca tacaacaaac ctgtatccga caccgcataa 1740

tcttgttaga ttaaacgcca tgctggaact gaagaaagaa tttagctgca tggtgggact 1800

ttctgatcat acaacagata atctggcatg ccttggcgcg gtcgttcttg gagcgtgtgt 1860

cttagaaaga cattttacag attcaatgca tcgcagcgga ccggatattg tttgttctat 1920

ggatacaaaa gccttaaaag aactgatcat tcaatcagaa cagatggcaa tcattcgcgg 1980

caataacgaa tcaaagaaag cagccaaaca agaacaggtt acaatcgatt ttgcttttgc 2040

ctctgtggtc tcaatcaaag atattaagaa aggcgaagtt ctgtcaatgg ataatatttg 2100

ggtgaaaaga ccgggacttg gcggaatcag cgcagcggaa tttgaaaata ttctgggcaa 2160

gaaagcactg cgcgatattg aaaacgatgc gcagttaagc tatgaagatt ttgcttaagc 2220

tcttaaggag gattttagaa tgaagaaaat tctgtttatc acaggctcaa gagcggatta 2280

ctctaaaatc aaatcactta tgtaccgcgt ccaaaattca agcgaatttg aactttacat 2340

ctttgctaca ggcatgcatc tgagcaaaaa ctttggatac acagttaaag aattatataa 2400

aaatggcttt aaaaacatct acgaatttat caactacgat aaatattatc agacagataa 2460

agccctggca acaacaattg atggattttc aagatatgcg aacgaattaa aaccggatct 2520

gattgttgtg catggcgatc gcatcgaacc gcttgcagcg gctattgtcg gagcccttaa 2580

caacatcctg gttgcacata tcgaaggcgg agaaattagc ggaacaatcg atgattcttt 2640

aagacatgcg atttcaaaac ttgctcatat ccatctggtg aacgatgaat ttgcaaaaag 2700

acgccttatg caattaggcg aagatgaaaa atcaatcttt atcatcggat ctccggatct 2760

ggaactgctt aacgataaca aaatctcact tagcgaagcc aaaaaatact acgatattaa 2820

ctacgaaaac tatgcattac tgatgtttca tccggtcaca acagaaatca catctatcaa 2880

aaaccaagcc gataacctgg tgaaagcact tatccagtca aacaaaaact acatcgtcat 2940

ctatccgaat aacgatctgg gctttgaact gatccttcag agctatgaag aatttaaaaa 3000

caacccgaga tttaaactgt ttccgtctct gcgctttgaa tactttatca cacttctgaa 3060

aaacgccgat tttattatcg gaaactcttc atgcatcctg aaagaagcgt tatacctgaa 3120

aacagctggc attctggttg gatcaagaca aaatggccgc ttaggaaatg aaaacacact 3180

gaaagtgaat gcgaacagcg atgaaatcct taaagctatc aacacaatcc ataaaaaaca 3240

ggatttattt tctgctaaac tggaaattct tgatagctct aaactgtttt tcgaatatct 3300

tcaatcaggc gatttcttta aacttagcac acagaaagtt tttaaagata ttaaataaaa 3360

aggaggaact actatgtcac ttgcaattat cccggcgaga ggcggaagca aaggcatcaa 3420

aaacaaaaac ctggttctgc ttaacaacaa accgcttatc tactacacaa tcaaagcagc 3480

gctgaatgct aaatctattt caaaagttgt ggtctcaagc gatagcgatg aaatccttaa 3540

ctacgccaaa tctcaaaacg tggatattct gaaaagaccg atctctcttg cacaggatga 3600

tacaacatca gataaagtct tactgcatgc gctgaaattt tacaaagatt acgaagatgt 3660

tgtgttttta caaccgacat ctccgctgcg cacaaacatt catatcaacg aagcatttaa 3720

cctgtacaaa aattcaaacg ctaatgccct gattagcgtc tctgaatgcg ataacaaaat 3780

ccttaaagca tttgtttgca acgattgtgg cgatttagcc ggaatttgta atgatgaata 3840

tccgtttatg ccgcgccaga aactgccgaa aacatatatg agcaacggag cgatctacat 3900

ccttaaaatc aaagaatttc tgaacaaccc gtcatttctg caaagcaaaa caaaacattt 3960

tcttatggat gaatcatcat cactggatat tgattgcctg gaagatttaa agaaagttga 4020

acaaatttgg aaaaaataaa gtcaatgtat gaatggatac gggatatgaa tcaataagta 4080

cgtgaaagag aaaagcaacc cagatatgat agggaacttt tctctttctt gttttacatt 4140

gaatctttac aatcctattg atataatcta agctagtgta ttttgcgttt aatagtggag 4200

aaaagctagc gattaactaa taaggaggac aaacatggat aaatttgctg aacatgaaat 4260

tccgaaagct gttatcgtgg ccggcaacgg agaatctctt tcacaaatcg attacagact 4320

gcttccgaaa aactatgatg tctttagatg caaccagttt tactttgaag aacgctattt 4380

tcttggcaac aaaatcaaag cagttttctt tacaccggga gtgtttttag aacaatacta 4440

cacactgtac catcttaaac gcaacaacga atactttgtc gataacgtta tcctgtcatc 4500

atttaaccat ccgacagttg atctggaaaa atcacaaaaa atccaggctc tgtttattga 4560

tgtgatcaac ggctacgaaa aatacctgtc taaactgaca gcctttgatg tttatctgag 4620

atacaaagaa ctttacgaaa accaacgcat tacatcagga gtgtatatgt gcgccgtcgc 4680

aatcgcgatg ggctatacag atatttacct tacaggaatc gatttttatc aggcaagcga 4740

agaaaactac gcgtttgata acaaaaaacc gaacatcatc agattactgc cggattttcg 4800

caaagaaaaa acattatttt catatcatag caaagatatt gatcttgaag cattatcttt 4860

tctgcaacag cattaccatg ttaactttta cagcatttct ccgatgtctc cgctgtcaaa 4920

acattttccg atcccgacag tggaagatga ttgtgaaaca acatttgtcg cgccgctgaa 4980

agaaaactac atcaacgata ttcttttacc gccgcatttt gtctatgaaa aacttggcgt 5040

tgataaactt gcagcggctt tagaacatca tcatcatcat cattaaagtg atagcggtac 5100

cattataggt aagagaggaa tgtacacatg tactatttaa aaaacacaaa cttttggatg 5160

ttcggtttat tctttttctt ttactttttt atcatgggag cctacttccc gtttttcccg 5220

atttggctac atgacatcaa ccatatcagc aaaagtgata cgggtattat ttttgccgct 5280

atttctctgt tctcgctatt attccaaccg ctgtttggtc tgctttctga caaactcggg 5340

ctgcgcaaat acctgctgtg gattattacc ggcatgttag tgatgtttgc gccgttcttt 5400

atttttatct tcgggccact gttacaatac aacattttag taggatcgat tgttggtggt 5460

atttatctag gcttttgttt taacgccggt gcgccagcag tagaggcatt tattgagaaa 5520

gtcagccgtc gcagtaattt cgaatttggt cgcgcgcgga tgtttggctg tgttggctgg 5580

gcgctgtgtg cctcgattgt cggcatcatg ttcaccatca ataatcagtt tgttttctgg 5640

ctgggctctg gctgtgcact catcctcgcc gttttactct ttttcgccaa aacggatgcg 5700

ccctcttctg ccacggttgc caatgcggta ggtgccaacc attcggcatt tagccttaag 5760

ctggcactgg aactgttcag acagccaaaa ctgtggtttt tgtcactgta tgttattggc 5820

gtttcctgca cctacgatgt ttttgaccaa cagtttgcta atttctttac ttcgttcttt 5880

gctaccggtg aacagggtac gcgggtattt ggctacgtaa cgacaatggg cgaattactt 5940

aacgcctcga ttatgttctt tgcgccactg atcattaatc gcatcggtgg gaaaaacgcc 6000

ctgctgctgg ctggcactat tatgtctgta cgtattattg gctcatcgtt cgccacctca 6060

gcgctggaag tggttattct gaaaacgctg catatgtttg aagtaccgtt cctgctggtg 6120

ggctgcttta aatatattac cagccagttt gaagtgcgtt tttcagcgac gatttatctg 6180

gtctgtttct gcttctttaa gcaactggcg atgattttta tgtctgtact ggcgggcaat 6240

atgtatgaaa gcatcggttt ccagggcgct tatctggtgc tgggtctggt ggcgctgggc 6300

ttcaccttaa tttccgtgtt cacgcttagc ggccccggcc cgctttccct gctgcgtcgt 6360

caggtgaatg aagtcgctta aggatccatg tctagagtcg acgtccccgg ggcagcccgc 6420

ctaatgagcg ggcttttttc acgtcccagg catcaaataa aacgaaaggc tcagtcgaaa 6480

gactgggcct ttcgttttat ctgttgtttg tcggtgaacg ctctctacta gagtcacact 6540

ggctcacctt cgggtgggcc tttctgcgtt tatagaattc atattactta gaggatacta 6600

ttgagagagc tattgataag atggttgaga cattacctga gagccaaaaa actttttatg 6660

aatatgaatt aaaaaaaaga accaacaaag gctgagacag actccaaacg agtctgtttt 6720

tttaaaaaaa atattaggag cattgaatat atattagaga attaagaaag acatgggaat 6780

aaaaatattt taaatccagt aaaaatatga taagattatt tcagaatatg aagaactctg 6840

tttgtttttg atgaaaaaac aaacaaaaaa aatccaccta acggaatctc aatttaacta 6900

acagcggcca aactgagaag ttaaatttga gaaggggaaa aggcggattt atacttgtat 6960

ttaactatct ccattttaac attttattaa accccataca agtgaaaatc ctcttttaca 7020

ctgttccttt aggtgatcgc ggagggacat tatgagtgaa gtaaacctaa aaggaaatac 7080

agatgaatta gtgtattatc gacagcaaac cactggaaat aaaatcgcca ggaagagaat 7140

caaaaaaggg aaagaagaag tttattatgt tgctgaaacg gaagagaaga tatggacaga 7200

agagcaaata aaaaactttt ctttagacaa atttggtacg catatacctt acatagaagg 7260

tcattataca atcttaaata attacttctt tgatttttgg ggctattttt taggtgctga 7320

aggaattgcg ctctatgctc acctaactcg ttatgcatac ggcagcaaag acttttgctt 7380

tcctagtcta caaacaatcg ctaaaaaaat ggacaagact cctgttacag ttagaggcta 7440

cttgaaactg cttgaaaggt acggttttat ttggaaggta aacgtccgta ataaaaccaa 7500

ggataacaca gaggaatccc cgatttttaa gattagacgt aaggttcctt tgctttcaga 7560

agaactttta aatggaaacc ctaatattga aattccagat gacgaggaag cacatgtaaa 7620

gaaggcttta aaaaaggaaa aagagggtct tccaaaggtt ttgaaaaaag agcacgatga 7680

atttgttaaa aaaatgatgg atgagtcaga aacaattaat attccagagg ccttacaata 7740

tgacacaatg tatgaagata tactcagtaa aggagaaatt cgaaaagaaa tcaaaaaaca 7800

aatacctaat cctacaacat cttttgagag tatatcaatg acaactgaag aggaaaaagt 7860

cgacagtact ttaaaaagcg aaatgcaaaa tcgtgtctct aagccttctt ttgatacctg 7920

gtttaaaaac actaagatca aaattgaaaa taaaaattgt ttattacttg taccgagtga 7980

atttgcattt gaatggatta agaaaagata tttagaaaca attaaaacag tccttgaaga 8040

agctggatat gttttcgaaa aaatcgaact aagaaaagtg caataaactg ctgaagtatt 8100

tcagcagttt tttttattta gaaatagtga aaaaaatata atcagggagg tatcaatatt 8160

taatgagtac tgatttaaat ttatttagac tggaattaat aattaacacg tagactaatt 8220

aaaatttaat gagggataaa gaggatacaa aaatattaat ttcaatccct attaaatttt 8280

aacaaggggg ggattaaaat ttaattagag gtttatccac aagaaaagac cctaataaaa 8340

tttttactag ggttataaca ctgattaatt tcttaatggg ggagggatta aaatttaatg 8400

acaaagaaaa caatctttta agaaaagctt ttaaaagata ataataaaaa gagctttgcg 8460

attaagcaaa actctttact ttttcattga cattatcaaa ttcatcgatt tcaaattgtt 8520

gttgtatcat aaagttaatt ctgttttgca caaccttttc aggaatataa aacacatctg 8580

aggcttgttt tataaactca gggtcgctaa agtcaatgta acgtagcata tgatatggta 8640

tagcttccac ccaagttagc ctttctgctt cttctgaatg tttttcatat acttccatgg 8700

gtatctctaa atgattttcc tcatgtagca aggtatgagc aaaaagttta tggaattgat 8760

agttcctctc tttttcttca acttttttat ctaaaacaaa cactttaaca tctgagtcaa 8820

tgtaagcata agatgttttt ccagtcataa tttcaatccc aaatctttta gacagaaatt 8880

ctggacgtaa atcttttggt gaaagaattt ttttatgtag caatatatcc gatacagcac 8940

cttctaaaag cgttggtgaa tagggcattt tacctatctc ctctcatttt gtggaataaa 9000

aatagtcata ttcgtccatc tacctatcct attatcgaac agttgaactt tttaatcaag 9060

gatcagtcct ttttttcatt attcttaaac tgtgctctta actttaacaa ctcgatttgt 9120

ttttccagat ctcgagggta actagcctcg ccgatcccgc aagaggcccg gcagtcaggt 9180

ggcacttttc ggggaaatgt gcgcggaacc cctatttgtt tatttttcta aatacattca 9240

aatatgtatc cgctcatgag acaataaccc tgataaatgc ttcaataata ttgaaaaagg 9300

aagagtatga gtattcaaca tttccgtgtc gcccttattc ccttttttgc ggcattttgc 9360

cttcctgttt ttgctcaccc agaaacgctg gtgaaagtaa aagatgctga agatcagttg 9420

ggtgcacgag tgggttacat cgaactggat ctcaacagcg gtaagatcct tgagagtttt 9480

cgccccgaag aacgttttcc aatgatgagc acttttaaag ttctgctatg tggcgcggta 9540

ttatcccgta ttgacgccgg gcaagagcaa ctcggtcgcc gcatacacta ttctcagaat 9600

gacttggttg agtactcacc agtcacagaa aagcatctta cggatggcat gacagtaaga 9660

gaattatgca gtgctgccat aaccatgagt gataacactg cggccaactt acttctgaca 9720

acgatcggag gaccgaagga gctaaccgct tttttgcaca acatggggga tcatgtaact 9780

cgccttgatc gttgggaacc ggagctgaat gaagccatac caaacgacga gcgtgacacc 9840

acgatgcctg tagcaatggc aacaacgttg cgcaaactat taactggcga actacttact 9900

ctagcttccc ggcaacaatt aatagactgg atggaggcgg ataaagttgc aggaccactt 9960

ctgcgctcgg cccttccggc tggctggttt attgctgata aatctggagc cggtgagcgt 10020

gggtctcgcg gtatcattgc agcactgggg ccagatggta agccctcccg tatcgtagtt 10080

atctacacga cggggagtca ggcaactatg gatgaacgaa atagacagat cgctgagata 10140

ggtgcctcac tgattaagca ttggtaactg tcagaccaag tttactcata tatactttag 10200

attgatttaa aacttcattt ttaatttaaa aggatctagg tgaagatcct ttttgataat 10260

ctcatgacca aaatccctta acgtgagttt tcgttccact gagcgtcaga ccccgtagaa 10320

aagatcaaag gatcttcttg agatcctttt tttctgcgcg taatctgctg cttgcaaaca 10380

aaaaaaccac cgctaccagc ggtggtttgt ttgccggatc aagagctacc aactcttttt 10440

ccgaaggtaa ctggcttcag cagagcgcag ataccaaata ctgtccttct agtgtagccg 10500

tagttaggcc accacttcaa gaactctgta gcaccgccta catacctcgc tctgctaatc 10560

ctgttaccag tggctgctgc cagtggcgat aagtcgtgtc ttaccgggtt ggactcaaga 10620

cgatagttac cggataaggc gcagcggtcg ggctgaacgg ggggttcgtg cacacagccc 10680

agcttggagc gaacgaccta caccgaactg agatacctac agcgtgagct atgagaaagc 10740

gccacgcttc ccgaagggag aaaggcggac aggtatccgg taagcggcag ggtcggaaca 10800

ggagagcgca cgagggagct tccaggggga aacgcctggt atctttatag tcctgtcggg 10860

tttcgccacc tctgacttga gcgtcgattt ttgtgatgct cgtcaggggg gcggagccta 10920

tggaaaaacg ccagcaacgc ggccttttta cggttcctgg ccttttgctg gccttttgct 10980

cacatgttct ttcctgcgtt atcccctgat tctgtggata accgtattac cgcctttgag 11040

tgagctgata ccgctcgccg cagccgaacg accgagcgca gcgagtcagt gagcgaggaa 11100

gcggaagagc gcccaatacg catgc 11125

<210> 3
<211> 13805
<212> DNA
<213> Artificial Sequence

<220>
<223> Expression plasmid

<400> 3
ttaagttatt ggtatgactg gttttaagcg caaaaaaagt tgctttttcg tacctattaa 60

tgtatcgttt tagaaaaccg actgtaaaaa gtacagtcgg cattatctca tattataaaa 120

gccagtcatt aggcctatct gacaattcct gaatagagtt cataaacaat cctgcatgat 180

aaccatcaca aacagaatga tgtacctgta aagatagcgg taaatatatt gaattacctt 240

tattaatgaa ttttcctgct gtaataatgg gtagaaggta attactatta ttattgatat 300

ttaagttaaa cccagtaaat gaagtccatg gaataataga aagagaaaaa gcattttcag 360

gtataggtgt tttgggaaac aatttccccg aaccattata tttctctaca tcagaaaggt 420

ataaatcata aaactctttg aagtcattct ttacaggagt ccaaatacca gagaatgttt 480

tagatacacc atcaaaaatt gtataaagtg gctctaactt atcccaataa cctaactctc 540

cgtcgctatt gtaaccagtt ctaaaagctg tatttgagtt tatcaccctt gtcactaaga 600

aaataaatgc agggtaaaat ttatatcctt cttgttttat gtttcggtat aaaacactaa 660

tatcaatttc tgtggttata ctaaaagtcg tttgttggtt caaataatga ttaaatatct 720

cttttctctt ccaattgtct aaatcaattt tattaaagtt catttgatat gcctcctaaa 780

tttttatcta aagtgaattt aggaggctta cttgtctgct ttcttcatta gaatcaatcc 840

ttttttaaaa gtcaatatta ctgtaacata aatatatatt ttaaaaatat cccactttat 900

ccaattttcg tttgttgaac taatgggtgc tttagttgaa gaataaagac cacattaaaa 960

aatgtggtct tttgtgtttt tttaaaggat ttgagcgtag cgaaaaatcc ttttctttct 1020

tatcttgata ataagggtaa ctattgccga tcgtccattc cgacagcatc gccagtcact 1080

atggcgtgct gctagcgcca ttcgccattc aggctgcgca actgttggga agggcgatcg 1140

gtgcgggcct cttcgctatt acgccagctg gcgaaagggg gatgtgctgc aaggcgatta 1200

agttgggtaa cgccagggtt ttcccagtca cgacgttgta aaacgacggc cagtgaattc 1260

gagctcaggc cttaactcac attaattgcg ttgcgctcac tgcccgcttt ccagtcggga 1320

aacctgtcgt gccagctgca ttaatgaatc ggccaacgcg cggggagagg cggtttgcgt 1380

attgggcgcc agggtggttt ttcttttcac cagtgagacg ggcaacagct gattgccctt 1440

caccgcctgg ccctgagaga gttgcagcaa gcggtccacg ctggtttgcc ccagcaggcg 1500

aaaatcctgt ttgatggtgg ttgacggcgg gatataacat gagctgtctt cggtatcgtc 1560

gtatcccact accgagatat ccgcaccaac gcgcagcccg gactcggtaa tggcgcgcat 1620

tgcgcccagc gccatctgat cgttggcaac cagcatcgca gtgggaacga tgccctcatt 1680

cagcatttgc atggtttgtt gaaaaccgga catggcactc cagtcgcctt cccgttccgc 1740

tatcggctga atttgattgc gagtgagata tttatgccag ccagccagac gcagacgcgc 1800

cgagacagaa cttaatgggc ccgctaacag cgcgatttgc tggtgaccca atgcgaccag 1860

atgctccacg cccagtcgcg taccgtcttc atgggagaaa ataatactgt tgatgggtgt 1920

ctggtcagag acatcaagaa ataacgccgg aacattagtg caggcagctt ccacagcaat 1980

ggcatcctgg tcatccagcg gatagttaat gatcagccca ctgacgcgtt gcgcgagaag 2040

attgtgcacc gccgctttac aggcttcgac gccgcttcgt tctaccatcg acaccaccac 2100

gctggcaccc agttgatcgg cgcgagattt aatcgccgcg acaatttgcg acggcgcgtg 2160

cagggccaga ctggaggtgg caacgccaat cagcaacgac tgtttgcccg ccagttgttg 2220

tgccacgcgg ttgggaatgt aattcagctc cgccatcgcc gcttccactt tttcccgcgt 2280

tttcgcagaa acgtggctgg cctggttcac cacgcgggaa acggtctgat aagagacacc 2340

ggcatactct gcgacatcgt ataacgttac tggtttcatc aaaatcgtct ccctccgttt 2400

gaatatttga ttgatcgtaa ccagatgaag cactctttcc actatcccta cagtgttatg 2460

gcttgaacaa tcacgaaaca ataattggta cgtacgatct ttcagccgac tcaaacatca 2520

aatcttacaa atgtagtctt tgaaagtatt acatatgtaa gatttaaatg caaccgtttt 2580

ttcggaagga aatgatgacc tcgtttccac cggaattagc ttggtaccaa aggaggtaag 2640

gatcactaga aaatttttta aaaaatctct tgacattgga agggagatat gttattataa 2700

gaattgcgga attgtgagcg gataacaatt cccatataga ttaactaata aggaggacaa 2760

acatgaaaga aatcaaaatc caaaacatca tcatcagcga agaaaaagcg ccgctggttg 2820

tgccggaaat cggcattaat cataacggat ctttagaact ggctaaaatc atggttgatg 2880

cagcgttttc agctggagcc aaaatcatca aacatcaaac acatatcgtc gaagatgaaa 2940

tgagcaaagc tgcaaagaaa gttatcccgg gcaacgctaa aatctctatc tacgaaatca 3000

tgcagaaatg cgctttagat tacaaagatg aacttgccct gaaagaatac acagaaaaac 3060

tgggacttgt gtatcttagc acaccgtttt caagagcagg cgcgaatcgc ttagaagata 3120

tgggagtctc tgcatttaaa atcggctcag gagaatgtaa taactatccg ctgatcaaac 3180

atattgcagc gtttaaaaaa ccgatgattg tgtctacagg catgaactca atcgaaagca 3240

ttaaaccgac agtgaaaatc ctgcttgata acgaaatccc gtttgtcctg atgcatacaa 3300

caaacctgta tccgacaccg cataatcttg ttagattaaa cgccatgctg gaactgaaga 3360

aagaatttag ctgcatggtg ggactttctg atcatacaac agataatctg gcatgccttg 3420

gcgcggtcgt tcttggagcg tgtgtcttag aaagacattt tacagattca atgcatcgca 3480

gcggaccgga tattgtttgt tctatggata caaaagcctt aaaagaactg atcattcaat 3540

cagaacagat ggcaatcatt cgcggcaata acgaatcaaa gaaagcagcc aaacaagaac 3600

aggttacaat cgattttgct tttgcctctg tggtctcaat caaagatatt aagaaaggcg 3660

aagttctgtc aatggataat atttgggtga aaagaccggg acttggcgga atcagcgcag 3720

cggaatttga aaatattctg ggcaagaaag cactgcgcga tattgaaaac gatgcgcagt 3780

taagctatga agattttgct taagctctta aggaggattt tagaatgaag aaaattctgt 3840

ttatcacagg ctcaagagcg gattactcta aaatcaaatc acttatgtac cgcgtccaaa 3900

attcaagcga atttgaactt tacatctttg ctacaggcat gcatctgagc aaaaactttg 3960

gatacacagt taaagaatta tataaaaatg gctttaaaaa catctacgaa tttatcaact 4020

acgataaata ttatcagaca gataaagccc tggcaacaac aattgatgga ttttcaagat 4080

atgcgaacga attaaaaccg gatctgattg ttgtgcatgg cgatcgcatc gaaccgcttg 4140

cagcggctat tgtcggagcc cttaacaaca tcctggttgc acatatcgaa ggcggagaaa 4200

ttagcggaac aatcgatgat tctttaagac atgcgatttc aaaacttgct catatccatc 4260

tggtgaacga tgaatttgca aaaagacgcc ttatgcaatt aggcgaagat gaaaaatcaa 4320

tctttatcat cggatctccg gatctggaac tgcttaacga taacaaaatc tcacttagcg 4380

aagccaaaaa atactacgat attaactacg aaaactatgc attactgatg tttcatccgg 4440

tcacaacaga aatcacatct atcaaaaacc aagccgataa cctggtgaaa gcacttatcc 4500

agtcaaacaa aaactacatc gtcatctatc cgaataacga tctgggcttt gaactgatcc 4560

ttcagagcta tgaagaattt aaaaacaacc cgagatttaa actgtttccg tctctgcgct 4620

ttgaatactt tatcacactt ctgaaaaacg ccgattttat tatcggaaac tcttcatgca 4680

tcctgaaaga agcgttatac ctgaaaacag ctggcattct ggttggatca agacaaaatg 4740

gccgcttagg aaatgaaaac acactgaaag tgaatgcgaa cagcgatgaa atccttaaag 4800

ctatcaacac aatccataaa aaacaggatt tattttctgc taaactggaa attcttgata 4860

gctctaaact gtttttcgaa tatcttcaat caggcgattt ctttaaactt agcacacaga 4920

aagtttttaa agatattaaa taaaaaggag gaactactat gtcacttgca attatcccgg 4980

cgagaggcgg aagcaaaggc atcaaaaaca aaaacctggt tctgcttaac aacaaaccgc 5040

ttatctacta cacaatcaaa gcagcgctga atgctaaatc tatttcaaaa gttgtggtct 5100

caagcgatag cgatgaaatc cttaactacg ccaaatctca aaacgtggat attctgaaaa 5160

gaccgatctc tcttgcacag gatgatacaa catcagataa agtcttactg catgcgctga 5220

aattttacaa agattacgaa gatgttgtgt ttttacaacc gacatctccg ctgcgcacaa 5280

acattcatat caacgaagca tttaacctgt acaaaaattc aaacgctaat gccctgatta 5340

gcgtctctga atgcgataac aaaatcctta aagcatttgt ttgcaacgat tgtggcgatt 5400

tagccggaat ttgtaatgat gaatatccgt ttatgccgcg ccagaaactg ccgaaaacat 5460

atatgagcaa cggagcgatc tacatcctta aaatcaaaga atttctgaac aacccgtcat 5520

ttctgcaaag caaaacaaaa catttcttta tggatgaatc atcatcactg gatattgatt 5580

gcctggaaga tttaaagaaa gttgaacaaa tttggaaaaa ataaagatta actaataagg 5640

aggacaaaca tgaaaagaat cttttgcctt gtgtctgcaa tcctgctttc agcgtgtaat 5700

gataaccaga atacagtcga tgttgtggtc tcaacagtga acgataacgt catcgaaaac 5760

aacacatacc aggtcaaacc gatcgataca ccgacaacat ttgatagcta ttcttggatt 5820

caaacatgcg gcacaccgat ccttaaagat gatgaaaaat actcactgtc atttgatttt 5880

gttgctccgg aattagatca ggatgaaaaa ttttgttttg aatttacagg cgatgttgat 5940

ggaaaacgct atgtgacaca aacaaatctg acagttgtgg caccgacact tgaagtgtat 6000

gtcgatcatg cgagcctgcc gtctcttcaa cagctgatga aaatcatcca acagaaaaac 6060

gaatactctc agaacgaaag atttatctca tggggccgca tcggacttac agaagataac 6120

gctgaaaaac tgaatgccca tatttatccg ctggctggaa ataacacatc tcaagaatta 6180

gtcgatgcag ttatcgatta cgcggattca aaaaacagac tgaatctgga acttaacaca 6240

aacacagctc atagctttcc gaacttagcc ccgattctgc gcattatctc aagcaaaagc 6300

aacatcctga tctctaacat caacctttac gatgatggct cagccgaata cgtgaacctg 6360

tacaactgga aagatacaga agataaaagc gtcaaactgt ctgattcatt tttagttctg 6420

aaagattact ttaatggaat ttcttcagaa aaaccgagcg gcatctatgg aagatataac 6480

tggcatcagc tgtacaacac atcttactat tttctgagaa aagattatct gacagtcgaa 6540

ccgcaactgc atgatcttcg cgaatatctt ggcggatcat taaaacagat gagctgggat 6600

ggcttttcac aactgagcaa aggagataaa gaactgtttc tgaacattgt tggctttgat 6660

caagaaaaac tgcaacagga atatcaacag agcgaacttc cgaattttgt gtttacagga 6720

acaacaacat gggcaggcgg agaaacaaaa gaatattatg cgcaacagca agttaacgtc 6780

gttaacaacg caatcaacga aacatctccg tactacctgg gcagagaaca tgatcttttc 6840

tttaaaggac atccgcgcgg cggaattatc aatgatatta tcctgggctc atttaataac 6900

atgattgata ttccggcgaa agttagcttt gaagtgctta tgatgacagg aatgttaccg 6960

gatacagtgg gcggaattgc atcatcactg tattttagca tcccggccga aaaagtctct 7020

tttatcgttt ttacatcaag cgatacaatc acagatcgcg aagatgctct gaaatctccg 7080

cttgttcagg tgatgatgac actgggcatt gtgaaagaaa aagatgtcct gttttggtca 7140

gatttaccgg attgctcttc aggagtttgt attgcccaat attaaagtga tagcggtacc 7200

attataggta agagaggaat gtacacatgt actatttaaa aaacacaaac ttttggatgt 7260

tcggtttatt ctttttcttt tactttttta tcatgggagc ctacttcccg tttttcccga 7320

tttggctaca tgacatcaac catatcagca aaagtgatac gggtattatt tttgccgcta 7380

tttctctgtt ctcgctatta ttccaaccgc tgtttggtct gctttctgac aaactcgggc 7440

tgcgcaaata cctgctgtgg attattaccg gcatgttagt gatgtttgcg ccgttcttta 7500

tttttatctt cgggccactg ttacaataca acattttagt aggatcgatt gttggtggta 7560

tttatctagg cttttgtttt aacgccggtg cgccagcagt agaggcattt attgagaaag 7620

tcagccgtcg cagtaatttc gaatttggtc gcgcgcggat gtttggctgt gttggctggg 7680

cgctgtgtgc ctcgattgtc ggcatcatgt tcaccatcaa taatcagttt gttttctggc 7740

tgggctctgg ctgtgcactc atcctcgccg ttttactctt tttcgccaaa acggatgcgc 7800

cctcttctgc cacggttgcc aatgcggtag gtgccaacca ttcggcattt agccttaagc 7860

tggcactgga actgttcaga cagccaaaac tgtggttttt gtcactgtat gttattggcg 7920

tttcctgcac ctacgatgtt tttgaccaac agtttgctaa tttctttact tcgttctttg 7980

ctaccggtga acagggtacg cgggtatttg gctacgtaac gacaatgggc gaattactta 8040

acgcctcgat tatgttcttt gcgccactga tcattaatcg catcggtggg aaaaacgccc 8100

tgctgctggc tggcactatt atgtctgtac gtattattgg ctcatcgttc gccacctcag 8160

cgctggaagt ggttattctg aaaacgctgc atatgtttga agtaccgttc ctgctggtgg 8220

gctgctttaa atatattacc agccagtttg aagtgcgttt ttcagcgacg atttatctgg 8280

tctgtttctg cttctttaag caactggcga tgatttttat gtctgtactg gcgggcaata 8340

tgtatgaaag catcggtttc cagggcgctt atctggtgct gggtctggtg gcgctgggct 8400

tcaccttaat ttccgtgttc acgcttagcg gccccggccc gctttccctg ctgcgtcgtc 8460

aggtgaatga agtcgcttaa ggatccatgt ctagagtcga cgtccccggg gcagcccgcc 8520

taatgagcgg gcttttttca cgtcccaggc atcaaataaa acgaaaggct cagtcgaaag 8580

actgggcctt tcgttttatc tgttgtttgt cggtgaacgc tctctactag agtcacactg 8640

gctcaccttc gggtgggcct ttctgcgttt atacccgggg cagcccgcct aatgagcggg 8700

cttttttcac gtcacgcgtc catggagatc tttgtctgca actgaaaagt ttatacctta 8760

cctggaacaa atggttgaaa catacgaggc taatatcggc ttattaggaa tagtccctgt 8820

actaataaaa tcaggtggat cagttgatca gtatattttg gacgaagctc ggaaagaatt 8880

tggagatgac ttgcttaatt ccacaattaa attaagggaa agaataaagc gatttgatgt 8940

tcaaggaatc acggaagaag atactcatga taaagaagct ctaaaactat tcaataacct 9000

tacaatggaa ttgatcgaaa gggtggaagg ttaatggtac gaaaattagg ggatctacct 9060

agaaagccac aaggcgatag gtcaagctta aagaaccctt acatggatct tacagattct 9120

gaaagtaaag aaacaacaga ggttaaacaa acagaaccaa aaagaaaaaa agcattgttg 9180

aaaacaatga aagttgatgt ttcaatccat aataagatta aatcgctgca cgaaattctg 9240

gcagcatccg aagggaattc atattactta gaggatacta ttgagagagc tattgataag 9300

atggttgaga cattacctga gagccaaaaa actttttatg aatatgaatt aaaaaaaaga 9360

accaacaaag gctgagacag actccaaacg agtctgtttt tttaaaaaaa atattaggag 9420

cattgaatat atattagaga attaagaaag acatgggaat aaaaatattt taaatccagt 9480

aaaaatatga taagattatt tcagaatatg aagaactctg tttgtttttg atgaaaaaac 9540

aaacaaaaaa aatccaccta acggaatctc aatttaacta acagcggcca aactgagaag 9600

ttaaatttga gaaggggaaa aggcggattt atacttgtat ttaactatct ccattttaac 9660

attttattaa accccataca agtgaaaatc ctcttttaca ctgttccttt aggtgatcgc 9720

ggagggacat tatgagtgaa gtaaacctaa aaggaaatac agatgaatta gtgtattatc 9780

gacagcaaac cactggaaat aaaatcgcca ggaagagaat caaaaaaggg aaagaagaag 9840

tttattatgt tgctgaaacg gaagagaaga tatggacaga agagcaaata aaaaactttt 9900

ctttagacaa atttggtacg catatacctt acatagaagg tcattataca atcttaaata 9960

attacttctt tgatttttgg ggctattttt taggtgctga aggaattgcg ctctatgctc 10020

acctaactcg ttatgcatac ggcagcaaag acttttgctt tcctagtcta caaacaatcg 10080

ctaaaaaaat ggacaagact cctgttacag ttagaggcta cttgaaactg cttgaaaggt 10140

acggttttat ttggaaggta aacgtccgta ataaaaccaa ggataacaca gaggaatccc 10200

cgatttttaa gattagacgt aaggttcctt tgctttcaga agaactttta aatggaaacc 10260

ctaatattga aattccagat gacgaggaag cacatgtaaa gaaggcttta aaaaaggaaa 10320

aagagggtct tccaaaggtt ttgaaaaaag agcacgatga atttgttaaa aaaatgatgg 10380

atgagtcaga aacaattaat attccagagg ccttacaata tgacacaatg tatgaagata 10440

tactcagtaa aggagaaatt cgaaaagaaa tcaaaaaaca aatacctaat cctacaacat 10500

cttttgagag tatatcaatg acaactgaag aggaaaaagt cgacagtact ttaaaaagcg 10560

aaatgcaaaa tcgtgtctct aagccttctt ttgatacctg gtttaaaaac actaagatca 10620

aaattgaaaa taaaaattgt ttattacttg taccgagtga atttgcattt gaatggatta 10680

agaaaagata tttagaaaca attaaaacag tccttgaaga agctggatat gttttcgaaa 10740

aaatcgaact aagaaaagtg caataaactg ctgaagtatt tcagcagttt tttttattta 10800

gaaatagtga aaaaaatata atcagggagg tatcaatatt taatgagtac tgatttaaat 10860

ttatttagac tggaattaat aattaacacg tagactaatt aaaatttaat gagggataaa 10920

gaggatacaa aaatattaat ttcaatccct attaaatttt aacaaggggg ggattaaaat 10980

ttaattagag gtttatccac aagaaaagac cctaataaaa tttttactag ggttataaca 11040

ctgattaatt tcttaatggg ggagggatta aaatttaatg acaaagaaaa caatctttta 11100

agaaaagctt ttaaaagata ataataaaaa gagctttgcg attaagcaaa actctttact 11160

ttttcattga cattatcaaa ttcatcgatt tcaaattgtt gttgtatcat aaagttaatt 11220

ctgttttgca caaccttttc aggaatataa aacacatctg aggcttgttt tataaactca 11280

gggtcgctaa agtcaatgta acgtagcata tgatatggta tagcttccac ccaagttagc 11340

ctttctgctt cttctgaatg tttttcatat acttccatgg gtatctctaa atgattttcc 11400

tcatgtagca aggtatgagc aaaaagttta tggaattgat agttcctctc tttttcttca 11460

acttttttat ctaaaacaaa cactttaaca tctgagtcaa tgtaagcata agatgttttt 11520

ccagtcataa tttcaatccc aaatctttta gacagaaatt ctggacgtaa atcttttggt 11580

gaaagaattt ttttatgtag caatatatcc gatacagcac cttctaaaag cgttggtgaa 11640

tagggcattt tacctatctc ctctcatttt gtggaataaa aatagtcata ttcgtccatc 11700

tacctatcct attatcgaac agttgaactt tttaatcaag gatcagtcct ttttttcatt 11760

attcttaaac tgtgctctta actttaacaa ctcgatttgt ttttccagat ctcgagggta 11820

actagcctcg ccgatcccgc aagaggcccg gcagtcaggt ggcacttttc ggggaaatgt 11880

gcgcggaacc cctatttgtt tatttttcta aatacattca aatatgtatc cgctcatgag 11940

acaataaccc tgataaatgc ttcaataata ttgaaaaagg aagagtatga gtattcaaca 12000

tttccgtgtc gcccttattc ccttttttgc ggcattttgc cttcctgttt ttgctcaccc 12060

agaaacgctg gtgaaagtaa aagatgctga agatcagttg ggtgcacgag tgggttacat 12120

cgaactggat ctcaacagcg gtaagatcct tgagagtttt cgccccgaag aacgttttcc 12180

aatgatgagc acttttaaag ttctgctatg tggcgcggta ttatcccgta ttgacgccgg 12240

gcaagagcaa ctcggtcgcc gcatacacta ttctcagaat gacttggttg agtactcacc 12300

agtcacagaa aagcatctta cggatggcat gacagtaaga gaattatgca gtgctgccat 12360

aaccatgagt gataacactg cggccaactt acttctgaca acgatcggag gaccgaagga 12420

gctaaccgct tttttgcaca acatggggga tcatgtaact cgccttgatc gttgggaacc 12480

ggagctgaat gaagccatac caaacgacga gcgtgacacc acgatgcctg tagcaatggc 12540

aacaacgttg cgcaaactat taactggcga actacttact ctagcttccc ggcaacaatt 12600

aatagactgg atggaggcgg ataaagttgc aggaccactt ctgcgctcgg cccttccggc 12660

tggctggttt attgctgata aatctggagc cggtgagcgt gggtctcgcg gtatcattgc 12720

agcactgggg ccagatggta agccctcccg tatcgtagtt atctacacga cggggagtca 12780

ggcaactatg gatgaacgaa atagacagat cgctgagata ggtgcctcac tgattaagca 12840

ttggtaactg tcagaccaag tttactcata tatactttag attgatttaa aacttcattt 12900

ttaatttaaa aggatctagg tgaagatcct ttttgataat ctcatgacca aaatccctta 12960

acgtgagttt tcgttccact gagcgtcaga ccccgtagaa aagatcaaag gatcttcttg 13020

agatcctttt tttctgcgcg taatctgctg cttgcaaaca aaaaaaccac cgctaccagc 13080

ggtggtttgt ttgccggatc aagagctacc aactcttttt ccgaaggtaa ctggcttcag 13140

cagagcgcag ataccaaata ctgtccttct agtgtagccg tagttaggcc accacttcaa 13200

gaactctgta gcaccgccta catacctcgc tctgctaatc ctgttaccag tggctgctgc 13260

cagtggcgat aagtcgtgtc ttaccgggtt ggactcaaga cgatagttac cggataaggc 13320

gcagcggtcg ggctgaacgg ggggttcgtg cacacagccc agcttggagc gaacgaccta 13380

caccgaactg agatacctac agcgtgagct atgagaaagc gccacgcttc ccgaagggag 13440

aaaggcggac aggtatccgg taagcggcag ggtcggaaca ggagagcgca cgagggagct 13500

tccaggggga aacgcctggt atctttatag tcctgtcggg tttcgccacc tctgacttga 13560

gcgtcgattt ttgtgatgct cgtcaggggg gcggagccta tggaaaaacg ccagcaacgc 13620

ggccttttta cggttcctgg ccttttgctg gccttttgct cacatgttct ttcctgcgtt 13680

atcccctgat tctgtggata accgtattac cgcctttgag tgagctgata ccgctcgccg 13740

cagccgaacg accgagcgca gcgagtcagt gagcgaggaa gcggaagagc gcccaatacg 13800

catgc 13805

<210> 4
<211> 10838
<212> DNA
<213> Artificial Sequence

<220>
<223> Suicide plasmid

<400> 4

ttctcatgtt tgacagctta tcatcgataa gctttaatgc ggtagtttat cacagttaaa 60

ttgctaacgc agtcaggcac cgtgtatgaa atctaacaat gcgctcatcg tcatcctcgg 120

caccgtcacc ctggatgctg taggcatagg cttggttatg ccggtactgc cgggcctctt 180

gcgggatatc gtccattccg acagcatcgc cagtcactat ggcgtgctgc tagcgctata 240

tgcgttgatg caatttctat gcgcacccgt tctcggagca ctgtccgacc gctttggccg 300

ccgcccagtc ctgctcgctt cgctacttgg agccactatc gactacgcga tcatggcgac 360

cacacccgtc ctgtggatcc tctacgccgg acgcatcgtg gccggcatca ccggcgccac 420

aggtgcggtt gctggcgcct atatcgccga catcaccgat ggggaagatc gggctcgcca 480

cttcgggctc atgagcgctt gtttcggcgt gggtatggtg gcaggccccg tggccggggg 540

actgttgggc gccatctcct tgcatgcacc attccttgcg gcggcggtgc tcaacggcct 600

caacctacta ctgggctgct tcctaatgca ggagtcgcat aagggagagc gtcgaccgat 660

gcccttgaga gccttcaacc cagtcagctc cttccggtgg gcgcggggca tgactatcgt 720

cgccgcactt atgactgtct tctttatcat gcaactcgta ggacaggtgc cggcagcgct 780

ctgggtcatt ttcggcgagg accgctttcg ctggagcgcg acgatgatcg gcctgtcgct 840

tgcggtattc ggaatcttgc acgccctcgc tcaagccttc gtcactggtc ccgccaccaa 900

acgtttcggc gagaagcagg ccattatcgc cggcatggcg gccgacgcgc tgggctacgt 960

cttgctggcg ttcgcgacgc gaggctggat ggccttcccc attatgattc ttctcgcttc 1020

cggcggcatc gggatgcccg cgttgcaggc catgctgtcc aggcaggtag atgacgacca 1080

tcagggacag cttcaaggat cgctcgcggc tcttaccagc ctaacttcga tcactggacc 1140

gctgatcgtc acggcgattt atgccgcctc ggcgagcaca tggaacgggt tggcatggat 1200

tgtaggcgcc gccctatacc ttgtctgcct ccccgcgttg cgtcgcggtg catggagccg 1260

ggccacctcg acctgaatgg aagccggcgg cacctcgcta acggattcac cactccaaga 1320

attggagcca atcaattctt gcggagaact gtgaatgcgc aaaccaaccc ttggcagaac 1380

atatccatcg cgtccgccat ctccagcagc cgcacgcggc gcatctcggg cagcgttggg 1440

tcctggccac gggtgcgcat gatcgtgctc ctgtcgttga ggacccggct aggctggcgg 1500

ggttgcctta ctggttagca gaatgaatca ccgatacgcg agcgaacgtg aagcgactgc 1560

tgctgcaaaa cgtctgcgac ctgagcaaca acatgaatgg tcttcggttt ccgtgtttcg 1620

taaagtctgg aaacgcggaa gtcagcgccc tgcaccatta tgttccggat ctgcatcgca 1680

ggatgctgct ggctaccctg tggaacacct acatctgtat taacgaagcg ctggcattga 1740

ccctgagtga tttttctctg gtcccgccgc atccataccg ccagttgttt accctcacaa 1800

cgttccagta accgggcatg ttcatcatca gtaacccgta tcgtgagcat cctctctcgt 1860

ttcatcggta tcattacccc catgaacaga aatccccctt acacggaggc atcagtgacc 1920

aaacaggaaa aaaccgccct taacatggcc cgctttatca gaagccagac attaacgctt 1980

ctggagaaac tcaacgagct ggacgcggat gaacaggcag acatctgtga atcgcttcac 2040

gaccacgctg atgagcttta ccgcagctgc ctcgcgcgtt tcggtgatga cggtgaaaac 2100

ctctgacaca tgcagctccc ggagacggtc acagcttgtc tgtaagcgga tgccgggagc 2160

agacaagccc gtcagggcgc gtcagcgggt gttggcgggt gtcggggcgc agccatgacc 2220

cagtcacgta gcgatagcgg agtgtatact ggcttaacta tgcggcatca gagcagattg 2280

tactgagagt gcaccagcgg gcaaggaaag ccttcaatat gtgcagtggt atgtcaactc 2340

tatgaagatc agcctgttta caatggcagg gtctttgctc tgtgtgacgt ttacggccta 2400

tgcgttttcg cgctttcggt ttaaagggag gaaatacgct ttaacgctct ttttattgct 2460

gcagatgatt cctcagtttt cagctttaat tgccttgttt gtgctggcgc aaatcttggg 2520

aatgatcaat agccactggc tgctaatctt gctttatatc ggcggcctga tcccgatgaa 2580

tacgtatttg atgaaagggt acatggattc cattccgatg gatttagacg aaagcgccaa 2640

gattgacgga gccagcagca ccagaatctt cttccagatc attctgccat tatcaaaacc 2700

gatggcggca gtcgtggcca tgaacggctt taccggtccg ctcggagatt ttgtgctgtc 2760

ctcaaccata ttgagaacgc ctgaatcata tacattgccc gtcggtctat tcaatttagt 2820

gaatgatgtc atgggggcca gctatacgac atttgcggcc ggagccctgc ttatcagcat 2880

accggttgcc gtcatcttta ttatgctgca aaagaatttt gtgtccggat taaccgcagg 2940

cggaacgaag ggctaagaga acaaggagga gaatgtgatg tcaaagcttg aaaaaacgca 3000

cgtaacaaaa gcaaaattta tgctccatgg gggagactac aaccccgatc agtggctgga 3060

tcggcccgat attttagctg acgatatcaa actgatgaag ctttctcata cgaatacgtt 3120

ttctgtcggc aattttacat ttttagaaat gggcgtgaaa aaaagcgcgc gattatgtaa 3180

aatataaaga ttaactaata aggaggacaa acatgacaga acagtacatc aaaaacgttg 3240

aagtgtatct tgattatgca acaatcccga cactgaacta cttttaccat tttacagaaa 3300

acaaagatga tattgcgaca attagactgt ttggccttgg acgctttaac atctctaaat 3360

caatcatcga atcatatccg gaaggcatta tcagatattg cccgattatc tttgaagatc 3420

agacagcttt tcaacagctg tttatcacac tgcttacaga agattctttt tgtcaatacc 3480

gctttaactt tcatattaac ctgtttcatt catggaaaat gcttatcccg ctgctgcata 3540

ttatctggca atttaaacat aaagtcctgg atattaaact taacttttat gatgatggct 3600

ctgaaggact ggttacactg tcaaaaatcg aacaaaacta ttcaagcgaa attcttcaga 3660

aaatcatcga tattgatagc caatcttttt acgctgataa acttagcttt ttagatgaag 3720

atattgccag atatttatgg aatagcctgt ttgaatcaca ttactatctt ttaaacgatt 3780

ttctgcttaa aaacgaaaaa ctgagcttac tgaaaaactc tatcaaatac tgccatatca 3840

tggatcttga acgctattta caatttacac aggaagaaaa agatttcttt aacgaacttc 3900

tgggcattaa catccagagc cttgaagata aaatcaaaat ctttcaacaa aagaaaacat 3960

ttatttttac aggaacaaca atcttttctc tgccgaaaga agaagaagaa acactttaca 4020

gactgcatct gaacgctatc cttaactaca tccatccgaa cggcaaatac tttatcggcg 4080

atggatttac actggtgatc aaaggacatc cgcatcaaaa agaaatgaat agcagactgg 4140

aaaaatcatt tgaaaaagcc gtcatgctgc cggataacat cccgtttgaa attctttacc 4200

tgatcggctg caaaccggat aaaattggcg gatttgtttc aacaagctac ttttcatgtg 4260

ataagaaaaa tattgcggat ctgctgttta tcagcgcgag acaggaagaa gtgcgcaaaa 4320

acgattatct ttttaacatc caataccagc tgagagatat gatgatcaaa acaggattta 4380

tccaagaaga aaaaacacat ttttacagcg atattccgat ctttatttct taaaaaggag 4440

gaactactat gtcacttgca attatcccgg cgagaggcgg aagcaaaggc atcaaaaaca 4500

aaaacctggt tctgcttaac aacaaaccgc ttatctacta cacaatcaaa gcagcgctga 4560

atgctaaatc tatttcaaaa gttgtggtct caagcgatag cgatgaaatc cttaactacg 4620

ccaaatctca aaacgtggat attctgaaaa gaccgatctc tcttgcacag gatgatacaa 4680

catcagataa agtcttactg catgcgctga aattttacaa agattacgaa gatgttgtgt 4740

ttttacaacc gacatctccg ctgcgcacaa acattcatat caacgaagca tttaacctgt 4800

acaaaaattc aaacgctaat gccctgatta gcgtctctga atgcgataac aaaatcctta 4860

aagcatttgt ttgcaacgat tgtggcgatt tagccggaat ttgtaatgat gaatatccgt 4920

ttatgccgcg ccagaaactg ccgaaaacat atatgagcaa cggagcgatc tacatcctta 4980

aaatcaaaga atttctgaac aacccgtcat ttctgcaaag caaaacaaaa catttcttta 5040

tggatgaatc atcatcactg gatattgatt gcctggaaga tttaaagaaa gttgaacaaa 5100

tttggaaaaa ataagctctt aaggaggatt ttagaatgag cacaacaaca caaaatatcc 5160

cgtggtatcg ccatcttaac agagcccagt ggcgcgcatt ttctgcagcg tggttaggct 5220

atctgcttga tggatttgat tttgttctga ttgcactggt tcttacagaa gtgcaaggcg 5280

aatttggact tacaacagtg caggctgcct ctttaatttc agcagcgttt atcagcagat 5340

ggtttggcgg attaatgctg ggcgcaatgg gagatcgcta tggaagacgc ctggcgatgg 5400

tcacatctat tgttctgttt tcagcgggca cattagcttg cggctttgcc ccgggatata 5460

ttacaatgtt tatcgcgaga cttgtgattg gcatgggaat ggcaggcgaa tatggatcaa 5520

gcgcgacgta tgttatcgaa tcttggccga aacatctgcg caataaagca tcaggatttc 5580

ttattagcgg cttttctgtg ggagcggttg tggctgccca agtctattct cttgtcgttc 5640

cggtttgggg atggagagca ctgtttttca ttggcatcct tccgattatc tttgctcttt 5700

ggttacgcaa aaatatcccg gaagccgaag attggaaaga aaaacatgcc ggaaaagcac 5760

ctgtgagaac aatggtcgat attctgtata gaggcgaaca tcgcattgct aacatcgtta 5820

tgacattagc agcggctaca gctctgtggt tttgctttgc cggaaattta caaaacgccg 5880

caatcgttgc tgtgctgggc ttactgtgtg cggctatttt tatcagcttt atggttcagt 5940

ctgccggaaa acgctggccg acaggcgtga tgttaatggt ggtcgttctg tttgcttttc 6000

tttattcatg gccgattcaa gcccttttac cgacatacct gaaaacagat ttagcttaca 6060

atccgcatac agtggccaac gtcctgtttt tctcaggctt tggagccgca gttggctgct 6120

gtgtgggcgg atttcttggc gattggttag gaacaagaaa agcgtatgtt tgttcactgc 6180

ttgcgagcca gttactgatt atcccggttt ttgcaatcgg cggagcgaat gtctgggttc 6240

ttggactttt actgtttttc caacagatgt taggccaagg aattgctggc atcttaccga 6300

aactgattgg cggatatttt gatacagatc agagagcggc tggccttgga tttacatata 6360

atgttggagc actgggcgga gcccttgcac cgattatcgg agcgttaatt gctcaacgcc 6420

tggatcttgg cacagcgctt gcttctttat catttagcct gacatttgtg gtcattcttt 6480

taatcggcct tgatatgccg tcaagagttc agcgctggtt aagaccggaa gcactgagaa 6540

cacatgatgc gattgatggc aaaccgtttt caggagctgt gccgtttggc agcgccaaaa 6600

acgatctggt caaaacaaaa tcataaccag gcatcaaata aaacgaaagg ctcagtcgaa 6660

agactgggcc tttcgtttta tctgttgttt gtcggtgaac gctctctact agagtcacac 6720

tggctcacct tcgggtgggc ctttctgcgt ttatatacgc gttaacccgg gcccgcggat 6780

ggatatgatc agatccttta actctggcaa ccctcaaaat tgaatgagac atgctacacc 6840

tccggataat aaatatatat aaacgtatat agatttcata aagtctaaca cactagactt 6900

atttacttcg taattaagtc gttaaaccgt gtgctctacg accaaaacta taaaaccttt 6960

aagaactttc tttttttaca agaaaaaaga aattagataa atctctcata tcttttattc 7020

aataatcgca tccgattgca gtataaattt aacgatcact catcatgttc atatttatca 7080

gagctcgtgc tataattata ctaattttat aaggaggaaa aaatatgggc atttttagta 7140

tttttgtaat cagcacagtt cattatcaac caaacaaaaa ataagtggtt ataatgaatc 7200

gttaataagc aaaattcata taaccaaatt aaagagggtt ataatgaacg agaaaaatat 7260

aaaacacagt caaaacttta ttacttcaaa acataatata gataaaataa tgacaaatat 7320

aagattaaat gaacatgata atatctttga aatcggctca ggaaaaggcc attttaccct 7380

tgaattagta aagaggtgta atttcgtaac tgccattgaa atagaccata aattatgcaa 7440

aactacagaa aataaacttg ttgatcacga taatttccaa gttttaaaca aggatatatt 7500

gcagtttaaa tttcctaaaa accaatccta taaaatatat ggtaatatac cttataacat 7560

aagtacggat ataatacgca aaattgtttt tgatagtata gctaatgaga tttatttaat 7620

cgtggaatac gggtttgcta aaagattatt aaatacaaaa cgctcattgg cattactttt 7680

aatggcagaa gttgatattt ctatattaag tatggttcca agagaatatt ttcatcctaa 7740

acctaaagtg aatagctcac ttatcagatt aagtagaaaa aaatcaagaa tatcacacaa 7800

agataaacaa aagtataatt atttcgttat gaaatgggtt aacaaagaat acaagaaaat 7860

atttacaaaa aatcaattta acaattcctt aaaacatgca ggaattgacg atttaaacaa 7920

tattagcttt gaacaattct tatctctttt caatagctat aaattattta ataagtaagc 7980

gaggcaggat caggacaatg attatatttt tgtcatgaat ttcacggaag aaaaacagct 8040

ggtcacgttt gatcagagtg tgaaggacat aatgacagga gacatattgt caggcgacct 8100

gacgatggaa aagtatgaag tgagaattgt cgtaaacaca cattaggctg atgctccgct 8160

cgatatgggc ggattctttt ttctatagaa tgaaaacgct tgctaagtct tggggggatg 8220

aaatcatgaa aagcaaagtg aaaatgttct ttgcggctgc catcgtgtgg agtgcatgta 8280

gttcaacagg atatgccgct gccattgaga aggagaagca cgtgtcagag cttcgggcag 8340

aggatctttt tgttaaaaaa gtagagggga tgaacaagga ttttatcaaa ggggcagatg 8400

tatccagcgt tattgctttg gaaaacagcg gtgtcacctt ttacaataca aacggaaaac 8460

gccaggatat ctttacaact ttaaaacagg ctggggtcaa ctatgttcgc gtccgcatct 8520

ggaatcaccc gtatgattca aatggcaacg ggtatggcgg gggaaacaat gatgttcaaa 8580

aagccatcga aatcggaaaa agagcgacag cgaacggaat gaaggtgctg gccgactttc 8640

actactctga tttctgggcc gatccagcga aacaaaaggt gcccaaagcc tgggcgaatc 8700

tcagctttga agcaaaaaaa gcaaagctct atgagtatac gaaacaaagc ctgcaaaaga 8760

tgatcaagga aggcgtgcgg tgtgaaatac cgcacagatg cgtaaggaga aaataccgca 8820

tcaggcgctc ttccgcttcc tcgctcactg actcgctgcg ctcggtcgtt cggctgcggc 8880

gagcggtatc agctcactca aaggcggtaa tacggttatc cacagaatca ggggataacg 8940

caggaaagaa catgtgagca aaaggccagc aaaaggccag gaaccgtaaa aaggccgcgt 9000

tgctggcgtt tttccatagg ctccgccccc ctgacgagca tcacaaaaat cgacgctcaa 9060

gtcagaggtg gcgaaacccg acaggactat aaagatacca ggcgtttccc cctggaagct 9120

ccctcgtgcg ctctcctgtt ccgaccctgc cgcttaccgg atacctgtcc gcctttctcc 9180

cttcgggaag cgtggcgctt tctcatagct cacgctgtag gtatctcagt tcggtgtagg 9240

tcgttcgctc caagctgggc tgtgtgcacg aaccccccgt tcagcccgac cgctgcgcct 9300

tatccggtaa ctatcgtctt gagtccaacc cggtaagaca cgacttatcg ccactggcag 9360

cagccactgg taacaggatt agcagagcga ggtatgtagg cggtgctaca gagttcttga 9420

agtggtggcc taactacggc tacactagaa ggacagtatt tggtatctgc gctctgctga 9480

agccagttac cttcggaaaa agagttggta gctcttgatc cggcaaacaa accaccgctg 9540

gtagcggtgg tttttttgtt tgcaagcagc agattacgcg cagaaaaaaa ggatctcaag 9600

aagatccttt gatcttttct acggggtctg acgctcagtg gaacgaaaac tcacgttaag 9660

ggattttggt catgagatta tcaaaaagga tcttcaccta gatcctttta aattaaaaat 9720

gaagttttaa atcaatctaa agtatatatg agtaaacttg gtctgacagt taccaatgct 9780

taatcagtga ggcacctatc tcagcgatct gtctatttcg ttcatccata gttgcctgac 9840

tccccgtcgt gtagataact acgatacggg agggcttacc atctggcccc agtgctgcaa 9900

tgataccgcg agacccacgc tcaccggctc cagatttatc agcaataaac cagccagccg 9960

gaagggccga gcgcagaagt ggtcctgcaa ctttatccgc ctccatccag tctattaatt 10020

gttgccggga agctagagta agtagttcgc cagttaatag tttgcgcaac gttgttgcca 10080

ttgctgcagg catcgtggtg tcacgctcgt cgtttggtat ggcttcattc agctccggtt 10140

cccaacgatc aaggcgagtt acatgatccc ccatgttgtg caaaaaagcg gttagctcct 10200

tcggtcctcc gatcgttgtc agaagtaagt tggccgcagt gttatcactc atggttatgg 10260

cagcactgca taattctctt actgtcatgc catccgtaag atgcttttct gtgactggtg 10320

agtactcaac caagtcattc tgagaatagt gtatgcggcg accgagttgc tcttgcccgg 10380

cgtcaacacg ggataatacc gcgccacata gcagaacttt aaaagtgctc atcattggaa 10440

aacgttcttc ggggcgaaaa ctctcaagga tcttaccgct gttgagatcc agttcgatgt 10500

aacccactcg tgcacccaac tgatcttcag catcttttac tttcaccagc gtttctgggt 10560

gagcaaaaac aggaaggcaa aatgccgcaa aaaagggaat aagggcgaca cggaaatgtt 10620

gaatactcat actcttcctt tttcaatatt attgaagcat ttatcagggt tattgtctca 10680

tgagcggata catatttgaa tgtatttaga aaaataaaca aataggggtt ccgcgcacat 10740

ttccccgaaa agtgccacct gacgtctaag aaaccattat tatcatgaca ttaacctata 10800

aaaataggcg tatcacgagg ccctttcgtc ttcaagaa 10838

<210> 5
<211> 4254
<212> DNA
<213> Artificial Sequence

<220>
<223> Integration cassette

<400> 5
gcaggctgtt attgtaacat gtaagccata agccattcgt aaaagtgcgg gaggaaggtc 60

atgaataatc tgcgtaatag actttcaggc gtgaatggga aaaataagag agtaaaagaa 120

aaagaacaaa aaatctggtc ggagattggg atgatagcgg gagcatttgc gctgcttgat 180

gtgatcatcc gcggcattat gtttgaattt ccgtttaaag aatgggctgc aagccttgtg 240

tttttgttca tcattatctt atattactgc atcagggctg cggcatccgg aatgctcatg 300

ccgagaatag acaccaaaga agaactgcaa aaacgggtga agcagcagcg aatagaatca 360

attgcggtcg cctttgcggt agtggtgctt acgatgtacg acagggggat tccccataca 420

ttcttcgctt ggctgaaaat gattcttctt tttatcgtct gcggcggcgt tctgtttctg 480

cttcggtatg tgattgtgaa gctggcttac agaagagcgg taaaagaaga aataaaaaag 540

aaatcatctt ttttgtttgg aaagcgaggg aagcgttcac agtttcgggc agcttttttt 600

ataggaacat tgatttgtat tcactctgcc aagttgtttt gatagagtga ttgtgataat 660

tttaaatgta agcgttaaca aaattctcca gtcttcacat cggtttgaaa ggaggaagcg 720

gaagaatgaa gtaagaggga tttttgactc cgaagtaagt cttcaaaaaa tcaaataagg 780

agtgtcaaga atgtttgcaa aacgattcaa aacctcttta ctgccgttat tcgctggatt 840

tttattgctg tttcatttgg ttctggcagg accggcggct gcgagtgctg aaacggcgaa 900

caaatcgaat gagcttacag caccgtcgat caaaagcgga accattcttc atgcatggcc 960

agtgaattcg agctcggtac ctaccgttcg tataatgtat gctatacgaa gttatgataa 1020

aaaatttaga agccaatgaa atctataaat aaactaaatt aagtttattt aattaacaac 1080

tatggatata aaataggtac taatcaaaat agtgaggagg atatatttga atacatacga 1140

acaaattaat aaagtgaaaa aaatacttcg gaaacattta aaaaataacc ttattggtac 1200

ttacatgttt ggatcaggag ttgagagtgg actaaaacca aatagtgatc ttgacttttt 1260

agtcgtcgta tctgaaccat tgacagatca aagtaaagaa atacttatac aaaaaattag 1320

acctatttca aaaaaaatag gagataaaag caacttacga tatattgaat taacaattat 1380

tattcagcaa gaaatggtac cgtggaatca tcctcccaaa caagaattta tttatggaga 1440

atggttacaa gagctttatg aacaaggata cattcctcag aaggaattaa attcagattt 1500

aaccataatg ctttaccaag caaaacgaaa aaataaaaga atatacggaa attatgactt 1560

agaggaatta ctacctgata ttccattttc tgatgtgaga agagccatta tggattcgtc 1620

agaggaatta atagataatt atcaggatga tgaaaccaac tctatattaa ctttatgccg 1680

tatgatttta actatggaca cgggtaaaat cataccaaaa gatattgcgg gaaatgcagt 1740

ggctgaatct tctccattag aacataggga gagaattttg ttagcagttc gtagttatct 1800

tggagagaat attgaatgga ctaatgaaaa tgtaaattta actataaact atttaaataa 1860

cagattaaaa aaattataaa aaaattgaaa aaatggtgga aacacttttt tcaatttttt 1920

tgttttatta tttaatattt gggaaatatt cattctaata taacttcgta taatgtatgc 1980

tatacgaacg gtaggatcct ctagagtcga cctgcaggca ttttacattt ttagaaatgg 2040

gcgtgaaaaa aagcgcgcga ttatgtaaaa tataaagtga tagcggtacc attataggta 2100

agagaggaat gtacacatgt actatttaaa aaacacaaac ttttggatgt tcggtttatt 2160

ctttttcttt tactttttta tcatgggagc ctacttcccg tttttcccga tttggctaca 2220

tgacatcaac catatcagca aaagtgatac gggtattatt tttgccgcta tttctctgtt 2280

ctcgctatta ttccaaccgc tgtttggtct gctttctgac aaactcgggc tgcgcaaata 2340

cctgctgtgg attattaccg gcatgttagt gatgtttgcg ccgttcttta tttttatctt 2400

cgggccactg ttacaataca acattttagt aggatcgatt gttggtggta tttatctagg 2460

cttttgtttt aacgccggtg cgccagcagt agaggcattt attgagaaag tcagccgtcg 2520

cagtaatttc gaatttggtc gcgcgcggat gtttggctgt gttggctggg cgctgtgtgc 2580

ctcgattgtc ggcatcatgt tcaccatcaa taatcagttt gttttctggc tgggctctgg 2640

ctgtgcactc atcctcgccg ttttactctt tttcgccaaa acggatgcgc cctcttctgc 2700

cacggttgcc aatgcggtag gtgccaacca ttcggcattt agccttaagc tggcactgga 2760

actgttcaga cagccaaaac tgtggttttt gtcactgtat gttattggcg tttcctgcac 2820

ctacgatgtt tttgaccaac agtttgctaa tttctttact tcgttctttg ctaccggtga 2880

acagggtacg cgggtatttg gctacgtaac gacaatgggc gaattactta acgcctcgat 2940

tatgttcttt gcgccactga tcattaatcg catcggtggg aaaaacgccc tgctgctggc 3000

tggcactatt atgtctgtac gtattattgg ctcatcgttc gccacctcag cgctggaagt 3060

ggttattctg aaaacgctgc atatgtttga agtaccgttc ctgctggtgg gctgctttaa 3120

atatattacc agccagtttg aagtgcgttt ttcagcgacg atttatctgg tctgtttctg 3180

cttctttaag caactggcga tgatttttat gtctgtactg gcgggcaata tgtatgaaag 3240

catcggtttc cagggcgctt atctggtgct gggtctggtg gcgctgggct tcaccttaat 3300

ttccgtgttc acgcttagcg gccccggccc gctttccctg ctgcgtcgtc aggtgaatga 3360

agtcgcttaa gcaatcaatg tcggatgcca gcctggcttt gattacgtgc taaatggttt 3420

atataatgac tcgggcttaa gcggttctct tccccattga gggcaaggct agacgggact 3480

taccgaaaga aaccatcaat gatggtttct tttttgttca taaatcagac aaaacttttc 3540

tcttgcaaaa gtttgtgaag tgttgcacaa tataaatgtg aaatacttca caaacaaaaa 3600

gacatcaaag agaaacatac cctggaagga tgattaatga tgaacaaaca tgtaaataaa 3660

gtagctttaa tcggagcggg ttttgttgga agcagttatg catttgcgtt aattaaccaa 3720

ggaatcacag atgagcttgt ggtcattgat gtaaataaag aaaaagcaat gggcgatgtg 3780

atggatttaa accacggaaa ggcgtttgcg ccacaaccgg tcaaaacatc ttacggaaca 3840

tatgaagact gcaaggatgc tgatattgtc tgcatttgcg ccggagcaaa ccaaaaacct 3900

ggtgagacac gccttgaatt agtagaaaag aacttgaaga ttttcaaagg catcgttagt 3960

gaagtcatgg cgagcggatt tgacggcatt ttcttagtcg cgacaaatcc ggttgatatc 4020

ctgacttacg caacatggaa attcagcggc ctgccaaaag agcgggtgat tggaagcggc 4080

acaacacttg attctgcgag attccgtttc atgctgagcg aatactttgg cgcagcgcct 4140

caaaacgtac acgcgcatat tatcggagag cacggcgaca cagagcttcc tgtttggagc 4200

cacgcgaatg tcggcggtgt gccggtcagt gaactcgttg agaaaaacga tgcg 4254